# EUROPEAN PATENT APPLICATION

(11) **EP 2 228 435 A1**
(43) Date of publication of application: **15.09.2010**
(21) Application number: 08858273.9
(22) Date of filing: 05.12.2008
(51) Int. Cl.: C12M 3/00, A61K 35/12, A61P 7/00, A61P 7/06, A61P 9/00, A61P 9/10, A61P 35/00, A61P 35/02, A61P 37/04, A61P 37/06, C12N 5/06

(54) **METHOD FOR AMPLIFICATION OF HEMATOPOIETIC STEM CELL USING HETEROCYCLIC COMPOUND**

(30) Priority: 05.12.2007 JP 2007315165
(71) Applicant: Nissan Chemical Industries, Ltd., Chiyoda-ku Tokyo 101-0054 (JP)
(72) Inventor: NAKAMURA, Takanori, Minamisaitama-gun Saitama 349-0294 (JP); MIYAMURA, Atsushi, Minamisaitama-gun Saitama 349-0294 (JP); NISHINO, Taito, Tokyo 101-0054 (JP); ISHIWATA, Norihisa, Minamisaitama-gun Saitama 349-0294 (JP); MIYAJI, Katsuaki, Funabashi-shi Chiba 274-8507 (JP)
(74) Representative: Blodig, Wolfgang
(86) International application number: PCT/JP2008/072188
(87) International publication number: WO 2009/072624

(57) **Abstract**

An object of the present invention is to expand CD34⁺ cells ex vivo efficiently in a short term using a biologically safe and inexpensively obtainable low molecular weight compound. A still another object of the present invention is to provide an expansion agent for CD34⁺ cells useful for treatment of various hematopoietic disorders caused by dysfunctional hematopoietic stem cells and/or hematopoietic progenitor cells.

A method for expanding CD34⁺ cells, which comprises culturing CD34⁺ cells ex vivo in the presence of a compound represented by the formula (I) (wherein A, B, L¹, L², L³, L⁴, R¹, R², R³, X and Y are defined in the description), a tautomer or pharmaceutically acceptable salt of the compound or a solvate thereof.

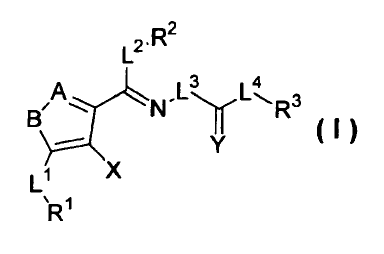

## Description

### TECHNICAL FIELD

The present invention relates to a method for expanding CD34⁺ cells using a low molecular weight compound having a blood cell expanding effect, in particular, to a method for expanding CD34⁺ cells in a culture medium containing various cytokines and/or growth factors in the presence of the compound and a material for cell therapy using the CD34⁺ cells obtained by the expansion method.

### BACKGROUND ART

Blood contains various lineages of blood cells having biological functions, such as the erythrocytic lineage associated with oxygen delivery, the megakaryocytic lineage generating thrombocytes, the granulocytic lineage associated with prevention of infections, the myeloid lineage such as monocytes and/or macrophages and the lymphocytic lineage responsible for immunity such as T cells and B cells. All these blood cells differentiate and mature from the common origin, hematopoietic stem cells, and are maintained and generated in an individual throughout its life. Hematopoietic stem cells are defined as cells having both pluripotency which allows them to differentiate into functional cells such as lymphocytes, erythrocytes and leukocytes and the ability to regenerate themselves while maintaining the pluripotency (self-renewal).

Previous studies have revealed that hematopoietic stem cells first diverge two ways into the myeloid lineage and the lymphoid lineage, then differentiate into myeloid stem cells (mixed colony forming cells, CFU-GEMM) and into lymphoid stem cells, respectively. Further, myeloid stem cells differentiate into erythrocytes via erythroid burst forming cells (BFU-E) and erythroid colony forming cells (CFU-E), into thrombocytes via megakaryocyte colony forming cells (CFU-MEG), into monocytes, neutrophils and basophils via granulocyte-macrophage colony forming cells (CFU-GM), and into eosinophils via eosinophil colony forming cells (CFU-EO), while lymphoid stem cells differentiate into T cells via T lymphoid progenitor cells and into B cells via B lymphoid progenitor cells. These myeloid stem cells and various hematopoietic progenitor cells derived from them are identified by the properties of colonies they form on soft agar, semisolid methylcellulose media or the like in the presence of various cytokines (Non-Patent Document 1).

In recent years, as a curative therapy for a number of intractable diseases such as various blood diseases attributed to hematopoietic dysfunction and immune dysfunction, cancer, immunodeficiency, autoimmune diseases and inborn error of metabolism, autologous or allogeneic transplantation of hematopoietic stem cells have been carried out. Quite recently, the effectiveness of hematopoietic stem cell transplantation in treating cerebral infarction, myocardial infarction and obstructive arteriosclerosis was reported (Non-Patent Documents 2, 3 and 4). Among them, bone marrow transplantation has been used in many cases of treatment and most established as a standard hematopoietic cell transplantation therapy. However, because for bone marrow transplantation, the human leukocyte antigens (HLA) of the bone marrow donor and the transplant recipient have to match closely, there is a problem that bone marrow from donors are in short supply. Besides, the need for at least 4 days of hospitalization and pain, fever and bleeding caused by collection of a large amount of bone marrow are a heavy burden to donors.

In addition to bone marrow, peripheral blood is also used as an alternative source of hematopoietic stem cells nowadays. Hematopoietic stem cells mobilized from the bone marrow to peripheral blood by administration of granulocyte colony stimulating factor (G-CSF) to a human are used for transplantation after enrichment using a blood cell separator. However, donors for peripheral blood hematopoietic stem cell transplantation have to bear a heavy burden of the need for administration of G-CSF for 4 to 6 consecutive days which may cause side effects (such as blood coagulation and spleen hypertrophy). Besides, because the efficiency of the mobilization of hematopoietic stem cells from the bone marrow to peripheral blood by G-CSF varies from donor to donor, hematopoietic stem cells are not obtained sufficiently in some cases.

Just recently, it was found that cord blood contains as many hematopoietic stem cells as bone marrow and is useful for hematopoietic stem cell transplantation (Non-Patent Document 5). Because cord blood transplantation does not require complete HLA matching and is less likely to cause severe acute graft-versus-host disease (GVHD) than bone marrow and peripheral blood transplantation, cord blood is established as useful and has been used more frequently. However, because cord blood is obtained in a small amount from one donor and does not contain many hematopoietic stem cells, its use is mainly limited to children.

To solve the above-mentioned problems with therapeutic hematopoietic stem cell transplantation, a technique for expanding hematopoietic stem cells and hematopoietic progenitor cells ex vivo is demanded, and various culture methods have been attempted so far.

Here, hematopoietic stem cells and hematopoietic progenitor cells, which are to be cultured, are explained. It was revealed that in human, hematopoietic stem cells and various hematopoietic progenitor cells derived from them are found in populations of CD34⁺ cells expressing the CD34 molecule as a cell surface antigen, and hence hematopoietic stem cells can be enriched as a CD34⁺ cell population (Non-Patent Document 6). Specifically speaking, they are often enriched by mixing a cell population to be separated with a CD34 antibody labeled with magnetic beads and magnetically collecting CD34⁺ cells (Non-Patent Documents 7 and 8).

Conventional techniques for expanding hematopoietic stem cells and/or hematopoietic progenitor cells will also be explained. As mentioned above, since hematopoietic stem cells are more enriched in CD34⁺ cells, CD34⁺ cells are mainly used as the starting cells for expansion. Expansion of CD34⁺ cells from CD34⁺ cells in culture in the presence of a cytokine or a growth factor such as stem cell factor (SCF), interleukin 3 (IL-3), interleukin 6 (IL-6), interleukin 6 (IL-6)/soluble IL-6 receptor complex, interleukin 11 (IL-11), granulocyte colony stimulating factor (G-CSF), granulocyte-macrophage colony stimulating factor (GM-CSF), flk2/flt3 ligand (FL), thrombopoietin (TPO) and erythropoietin or Notch ligand (such as Delta 1) has been reported (Non-Patent Document 9). Among them, TPO is especially excellent in hematopoietic stem cell expansion effect and used for in most of cases of expansion (Non-Patent Document 10). Hematopoietic stem cells and hematopoietic progenitor cells expand in culture in the presence of such various cytokines and growth factors, but these cytokines and growth factors are all produced as recombinant proteins, it may be difficult to obtain them for expansion stably in a large amount at low cost quickly.

For ex vivo expansion of hematopoietic stem cells, coculture systems using a different type of cells as feeder cells in the presence of various cytokines were reported. For example, expansion of CD34⁺ cells in coculture with human bone marrow stromal cells was attempted (Non-Patent Document 11 and Patent Document 1). An attempt to expand CD34⁺ cells in the presence of TPO, FL and SCF using mouse bone marrow cell line HESS-5 was also reported (Non-Patent Document 12). However, these coculture systems use foreign cells, there is a risk that cells infected with an unknown pathogen whose existence has not been confirmed may also be transplanted to patients. Furthermore, when stromal cells from a different kind of animal are used, the stromal cells have to be separated completely from CD34⁺ cells because otherwise there is a risk of causing immune response in the recipient after transplantation.

In addition, ex vivo expansion of hematopoietic stem cells in culture in the presence of various cytokines combined with low molecular weight compounds, not just various cytokines only, has been reported. Examples of such low molecular weight compounds include copper chelators, the combination of a histone deacetylase inhibitor and a DNA methylase inhibitor, all-trans retinoic acid, aldehyde dehydrogenase inhibitors (Non-Patent Documents 13, 14 and 15 and Patent Document 2). However, addition of any of them is not effective enough since hematopoietic stem cells have to be cultured for about 3 weeks.

Patent Document 1: JP-A-2006-61106

Patent Document 2: JP-A-2002-502617

Non-Patent Document 1: Lu, L. et al.; Exp. Hematol., 11, 721-9,1983

Non-Patent Document 2: Taguchi, A et al.; J Clin Invest., 114, 330-8. 2004

Non-Patent Document 3: Orlic, D et al.; Nature, 410, 701-5. 2001

Non-Patent Document 4: Tateishi-Yuyama, E et al.; Lancet, 360, 427-35. 2002

Non-Patent Document 5: Kurtzbert, J. et al.; New Eng. J. Med., 335, 157-66, 1996

Non-Patent Document 6: Ema, H. et al.; Blood, 75, 1941-6, 1990

Non-Patent Document 7: Ishizawa, L. et al.; J Hematother., 2, 333-8, 1993

Non-Patent Document 8: Cassel, A. et al.; Exp. Hematol., 21, 585-91, 1993

Non-Patent Document 9: Lam AC et al. Transfusion. 2001 Dec; 41 (12): 1567-76

Non-Patent Document 10: Kaushansky, K et al.; Ann NY Acad Sci., 1044,139-141,2005

Non-Patent Document 11: Rosler, E, et al.; Exp Hematol., 28, 841-52, 2000

Non-Patent Document 12: Shimakura, Y et al.; Stem Cells, 18, 183-9, 2000

Non-Patent Document 13: Chute, JP et al.; Proc Natl Acad Sci USA., 103, 11707-12, 2006

Non-Patent Document 14: Milhem, M et al.; Blood., 103, 4102-10, 2004

Non-Patent Document 15: Leung, AY et al.; Exp Hematol., 33, 422-7, 2005

### DISCLOSURE OF THE INVENTION

### OBJECT TO BE ACCOMPLISHED BY THE INVENTION

An object of the present invention is to expand CD34⁺ cells ex vivo efficiently in a short term using a biologically safe and inexpensively obtainable low molecular weight compound. A still another object of the present invention is to provide an expansion agent for CD34⁺ cells useful for treatment of various hematopoietic disorders caused by dysfunctional hematopoietic stem cells and/or hematopoietic progenitor cells.

### MEANS TO ACCOMPLISH THE OBJECT

The present inventors conducted extensive search for compounds having expansion activity to find a method for expanding CD34⁺ cells ex vivo. As a result, they found that the compounds represented by the following formula show excellent expansion activity on CD34⁺ cells, even in the absence of TPO and are highly useful as an expansion agent for cell populations rich in human hematopoietic stem cells and/or hematopoietic progenitor cells and accomplished the present invention.

Namely, the present invention relates:
(1) A method for expanding CD34⁺ cells, which comprises culturing CD34⁺ cells ex vivo in the presence of a compound represented by the formula (I), a tautomer or pharmaceutically acceptable salt of the compound or a solvate thereof: 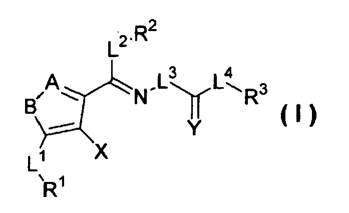 wherein A is a nitrogen atom or CR⁴ (wherein R⁴ is a hydrogen atom, a hydroxyl group (the hydroxyl group may be substituted with a C₂₋₆ alkenyl group or a C₂₋₆ alkynyl group), a thiol group (the thiol group may be substituted with a C₁₋₁₀ alkyl group, a C₂₋₆ alkenyl group, a C₂₋₆ alkynyl group or a C₁₋₁₀ alkylcarbonyl group), an amino group (the amino group may be substituted with one or two C₂₋₆ alkenyl groups or one or two C₂₋₆ alkynyl groups), a formyl group, a halogen atom, a nitro group, a cyano group, a C₁₋₁₀ alkyl group, a C₂₋₆ alkenyl group, a C₂₋₆ alkynyl group, a C₁₋₁₀ alkylcarbonylamino group, a mono- or di-C₁₋₁₀ alkylamino group, a C₁₋₁₀ alkoxy group (the C₁₋₁₀ alkyl group, the C₂₋₆ alkenyl group, the C₂₋₆ alkynyl group, the C₁₋₁₀ alkylcarbonylamino group, the mono- or di-C₁₋₁₀ alkylamino group and the C₁₋₁₀ alkoxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), a C₂₋₁₄ aryloxy group (the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), SO₂R⁵, SOR⁵ or COR⁵ (wherein R⁵ is a hydroxyl group, a C₁₋₁₀ alkyl group, a C₂₋₆ alkenyl group, a C₂₋₆ alkynyl group, a C₁₋₁₀ alkoxy group (the C₁₋₁₀ alkyl group, the C₂₋₆ alkenyl group, the C₂₋₆ alkynyl group and the C₁₋₁₀ alkoxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl group may be substituted with one or more halogen atoms) or one or more halogen atoms)), a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), a C₂₋₁₄ aryloxy group (the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) or NR⁶R⁷ (wherein each of R⁶ and R⁷ is independently a hydrogen atom, a hydroxyl group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), or R⁶ and R⁷ mean, together with each other, -(CH₂)ₘ₁-E-(CH₂)ₘ₂- (wherein E is an oxygen atom, a sulfur atom, CR²⁶R²⁷ (wherein each of R²⁶ and R²⁷ is independently a hydrogen atom, a C₁₋₁₀ alkyl group, a C₂₋₁₄ aryl group, a C₁₋₁₀ alkoxy group, a C₂₋₁₄ aryloxy group, a hydroxyl group or a protected hydroxyl group) or NR⁸ (wherein R⁸ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), and each of m1 and m2 is independently an integer of from 0 to 5 provided that m1+m2 is 3, 4 or 5)))),
   B is an oxygen atom, a sulfur atom or NR⁹ (wherein R⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) or a C₂₋₁₄ aryloxy group (the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)) (provided that when A is a nitrogen atom, B is not NH),
   R¹ is a C₂₋₁₄ aryl group (the C₂₋₁₄ aryl group may be substituted with one or more substituents selected from the group consisting of: halogen atoms, carboxyl groups, nitro groups, formyl groups, cyano groups, hydroxyl groups, protected hydroxyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyl groups, C₁₋₁₀ alkylcarbonyloxy groups, C₁₋₁₀ alkoxycarbonyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyl groups, the C₁₋₁₀ alkylcarbonyloxy groups and the C₁₋₁₀ alkoxycarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups (the C₂₋₁₄ aryl groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), thiol groups and amino groups (the thiol groups and the amino groups may be optionally substituted with one or two substituents selected from the group consisting of: formyl groups, C₁₋₁₀ alkyl groups, C₂₋₆ alkenyl groups, C₂₋₆ alkynyl groups and C₁₋₁₀ alkylcarbonyl groups (the C₁₋₁₀ alkyl groups, the C₂₋₆ alkenyl groups, the C₂₋₆ alkynyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)))),
   L¹ is a bond, CR¹⁰R¹¹ (wherein each of R¹⁰ and R¹¹ is independently a hydrogen atom or a C₁₋₆ alkyl group (the C₁₋₆ alkyl group may be substituted with one or more halogen atoms)), an oxygen atom, a sulfur atom or NR¹² (wherein R¹² is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
   X is OR¹³, SR¹³ or NR¹⁴R¹⁵ (wherein R¹³ is a hydrogen atom, a C₁₋₁₀ alkyl group, a C₂₋₆ alkenyl group, a C₂₋₆ alkynyl group or a C₁₋₁₀ alkylcarbonyl group (the C₁₋₁₀ alkyl group, the C₂₋₆ alkenyl group, the C₂₋₆ alkynyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), and each of R¹⁴ and R¹⁵ is independently a hydrogen atom, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
   R² is a hydrogen atom, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups),
   L² is a bond, CR³⁴R³⁵ (wherein each of R³⁴ and R³⁵ is independently a hydrogen atom or a C₁₋₆ alkyl group (the C₁₋₆ alkyl group may be substituted with one or more halogen atoms)), an oxygen atom, a sulfur atom or NR¹⁶ (wherein R¹⁶ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
   L³ is a bond, CR¹⁷R¹⁸ (wherein each of R¹⁷ and R¹⁸ is independently a hydrogen atom, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), an oxygen atom, a sulfur atom or NR¹⁹ (wherein R¹⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
   L⁴ is a bond, CR²⁰R²¹ (wherein each of R²⁰ and R²¹ is independently a hydrogen atom, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), an oxygen atom, a sulfur atom or NR²² (wherein R²² is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
   Y is an oxygen atom, a sulfur atom or NR²³ (wherein R²³ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), and
   R³ is a C₂₋₁₄ aryl group (the C₂₋₁₄ aryl group is optionally substituted with one or more substituents independently represented by -V¹ (wherein V¹ is a hydrogen atom, a hydroxyl group, a protected hydroxyl group, an amino group, a thiol group, a nitro group, a cyano group, a halogen atom, a carboxyl group, a sulfo group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono- or di-C₁₋₁₀ alkylamino group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono- or di-C₁₋₁₀ alkylamino group, the C₁₋₁₀ alkylaminosulfonyl group, the C₁₋₁₀ alkylaminocarbonyl group, the C₁₋₁₀ alkylsulfonylamino group and the C₁₋₁₀ thioalkyl group may be optionally substituted with one or more substituents selected from the group consisting of: carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group or a C₂₋₁₄ aryloxy group (the C₂₋₁₄ aryl group and the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)) and with one or more substituents independently represented by -W¹(CW²W³)ₘW⁴ (wherein W¹ is (CR²⁴R²⁵)ₙ (wherein each of R²⁴ and R²⁵ is independently a hydrogen atom, a hydroxyl group, an amino group, a C₁₋₆ alkyl group or a C₁₋₆ alkoxy group (the C₁₋₆ alkyl group and the C₁₋₆ alkoxy group may be substituted with one or more halogen atoms), and n is 0, 1, 2 or 3), an oxygen atom, a sulfur atom or NR³⁶ (wherein R³⁶ is a hydrogen atom, a C₁₋₆ alkyl group, a formyl group or a C₁₋₆ alkylcarbonyl group), each of W² and W³ is independently a hydrogen atom or a C₁₋₃ alkyl group (the C₁₋₃ alkyl group may be substituted with one or more halogen atoms), m is 0, 1, 2 or 3, and W⁴ is SO₂R²⁸, SOR²⁸ or COR²⁸ (wherein R²⁸ is NR²⁹R³⁰ (wherein R²⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group or a C₂₋₁₄ aryloxy group (the C₂₋₁₄ aryl group and the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), and R³⁰ is an amino group, a mono- or di-C₁₋₁₀ alkylamino group, a C₁₋₁₀ alkylcarbonylamino group, a C₁₋₁₀ alkylaminocarbonyl group, a C₂₋₉ heterocyclyl group (the amino group, the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group are optionally substituted with one or more substituents selected from the group consisting of: hydrogen atoms, carboxyl groups, carbamoyl groups, sulfamoyl groups, sulfo groups, nitro groups, cyano groups, halogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) and with one or more substituents selected from the group consisting of: carboxyl groups, carbamoyl groups, sulfo groups, sulfamoyl groups and C₂₋₉ heterocyclyl groups (the C₂₋₉ heterocyclyl groups may be optionally substituted with one or more substituents selected from the group consisting of: hydrogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups, C₂₋₉ heterocyclyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups, the C₁₋₁₀ alkylcarbonyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁-₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))), or R²⁹ and R³⁰ mean, together with each other, -(CH₂)ₘ₃-G-(CH₂)ₘ₄- (wherein G is CR³¹R³² (wherein R³¹ is a hydrogen atom, a C₁₋₁₀ alkyl group, a C₂₋₁₄ aryl group, a C₁₋₁₀ alkoxy group, a C₂₋₁₄ aryloxy group (the C₁₋₁₀ alkyl group, the C₂₋₁₄ aryl group, the C₁₋₁₀ alkoxy group and the C₂₋₁₄ aryloxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a hydroxyl group or a protected hydroxyl group, and R³² is an amino group, a mono- or di-C₁₋₁₀ alkylamino group, a C₁₋₁₀ alkylcarbonylamino group, a C₁₋₁₀ alkylaminocarbonyl group or a C₂₋₉ heterocyclyl group (the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, amino groups, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))) or NR³³ (wherein R³³ is an amino group, a mono- or di-C₁₋₁₀ alkylamino group, a C₁₋₁₀ alkylcarbonylamino group, a C₁₋₁₀ alkylaminocarbonyl group or a C₂₋₉ heterocyclyl group (the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, amino groups, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))), and each of m3 and m4 is independently an integer of from 0 to 5, provided that m3+m4 is 3, 4 or 5), or NR²⁹R³⁰, as a whole, means a nitrogen-containing C₂₋₉ cyclyl group (the nitrogen-containing C₂₋₉ cyclyl group may be optionally substituted with one or more hydrogen atoms and is substituted with two or three substituents independently selected from the group consisting of: hydroxyl groups, amino groups, thiol groups, nitro groups, cyano groups, halogen atoms, carboxyl groups, carbamoyl groups, sulfamoyl groups, formyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyloxy groups, C₁₋₁₀ alkoxycarbonyl groups, C₁₋₁₀ alkylcarbonyl groups, C₁₋₁₀ alkylcarbonylamino groups, mono- or di-C₁₋₁₀ alkylamino groups, C₁₋₁₀ alkylsulfonylamino groups, C₁₋₁₀ thioalkyl groups, C₂₋₉ heterocyclyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyloxy groups, the C₁₋₁₀ alkoxycarbonyl groups, the C₁₋₁₀ alkylcarbonyl groups, the C₁₋₁₀ alkylcarbonylamino groups, the mono- or di-C₁₋₁₀ alkylamino groups, the C₁₋₁₀ alkylsulfonylamino groups, the C₁₋₁₀ thioalkyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))) or a substituted C₂₋₉ heterocyclyl group (the substituted C₂₋₉ heterocyclyl group may be optionally substituted with one or more substituents selected from the group consisting of: hydrogen atoms, hydroxyl groups, amino groups, thiol groups, nitro groups, cyano groups, halogen atoms, carboxyl groups, carbamoyl groups, sulfamoyl groups, formyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyloxy groups, C₁₋₁₀ alkoxycarbonyl groups, C₁₋₁₀ alkylcarbonyl groups, C₁₋₁₀ alkylcarbonylamino groups, mono- or di-C₁₋₁₀ alkylamino groups, C₁₋₁₀ alkylsulfonylamino groups, C₁₋₁₀ thioalkyl groups, C₂₋₉ heterocyclyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyloxy groups, the C₁₋₁₀ alkoxycarbonyl groups, the C₁₋₁₀ alkylcarbonyl groups, the C₁₋₁₀ alkylcarbonylamino groups, the mono- or di-C₁₋₁₀ alkylamino groups, the C₁₋₁₀ alkylsulfonylamino groups, the C₁₋₁₀ thioalkyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))))), a C₂₋₁₄ aryl group (the C₂₋₁₄ aryl group is optionally substituted with one or more substituents independently represented by -V¹ (wherein V¹ is the same as defined above) and with one or more substituents selected from the group consisting of: C₁₋₆ alkyl groups, C₂₋₆ alkenyl groups and C₂₋₆ alkynyl groups (the C₁₋₆ alkyl groups, the C₂₋₆ alkenyl groups and the C₂₋₆ alkynyl groups are optionally substituted with one or more substituents selected from the group consisting of: hydroxyl groups, amino groups, mono- or di-C₁₋₁₀ alkylamino groups and C₁₋₆ alkoxy groups (the mono- or di-C₁₋₁₀ alkylamino groups and the C₁₋₆ alkoxy groups may be substituted with one or more halogen atoms), with one or more substituents independently represented by -V² (wherein V² is the same as V¹, and V¹ is the same as defined above) and with -W⁵ (wherein W⁵ is SO₂R⁴², SOR⁴², COR⁴² (wherein R⁴² is a hydrogen atom, a hydroxyl group or NR⁴³R⁴⁴, R⁴³ is the same as R⁶, R⁴⁴ is the same as R⁷, and R⁶ and R⁷ are the same as defined above) or W⁴, and W⁴ is the same as defined above))), a C₂₋₁₄ aryl group (the C₂₋₁₄ aryl group is optionally substituted with one or more substituents independently represented by -V¹ (wherein V¹ is the same as defined above) and with one or more substituents selected from the group consisting of: C₁₋₆ alkyl groups, C₂₋₆ alkenyl groups, C₂₋₆ alkynyl groups, C₁₋₁₀ alkylcarbonyl groups, C₁₋₁₀ alkylthiocarbonyl groups and C₁₋₁₀ alkylsulfonyl groups (the C₁₋₆ alkyl groups, the C₂₋₆ alkenyl groups, the C₂₋₆ alkynyl groups, the C₁₋₁₀ alkylcarbonyl groups, the C₁₋₁₀ alkylthiocarbonyl groups and the C₁₋₁₀ alkylsulfonyl groups are optionally substituted with one or more substituents independently represented by -V² (wherein V² is the same as V¹, and V¹ is the same as defined above) and with one or more substituents selected from C₂₋₉ heterocyclyl groups (the C₂₋₉ heterocyclyl groups are optionally substituted with one or more substituents independently represented by -V³ (wherein V³ is the same as V¹, and V¹ is the same as defined above)))), a C₂₋₁₄ aryl group (the C₂₋₁₄ aryl group is optionally substituted with one or more substituents independently represented by -V¹ (wherein V¹ is the same as defined above) and with one or more substituents selected from the group consisting of: C₁₋₆ alkyl groups, C₂₋₆ alkenyl groups, C₂₋₆ alkynyl groups, C₁₋₁₀ alkylcarbonyl groups, C₁₋₁₀ alkylthiocarbonyl groups and C₁₋₁₀ alkylsulfonyl groups (the C₁₋₆ alkyl groups, the C₂₋₆ alkenyl groups, the C₂₋₆ alkynyl groups, the C₁₋₁₀ alkylcarbonyl groups, the C₁₋₁₀ alkylthiocarbonyl groups and the C₁₋₁₀ alkylsulfonyl groups are optionally substituted with one or more substituents independently represented by -V² (wherein V² is the same as V¹, and V¹ is the same as defined above) and with one or more substituents selected from the group consisting of: amino groups (the amino groups are substituted with a C₁₋₁₀ alkyl group (the C₁₋₁₀ alkyl group is optionally substituted with one or more substituents independently represented by -V³ (wherein V³ is the same as V¹, and V¹ is the same as defined above)) and with a C₂₋₉ heterocyclyl group or a C₂₋₁₄ aryl group (the C₂₋₉ heterocyclyl group and the C₂₋₁₄ aryl group are optionally substituted with one or more substituents independently represented by -V⁴ (wherein V⁴ is the same as V¹, and V¹ is the same as defined above))), amino groups, mono- or di-C₁₋₁₀ alkylamino groups, C₁₋₁₀ alkoxy groups, C₁₋₁₀ thioalkyl groups, C₁₋₁₀ alkylsulfonyl groups, sulfonyl groups, sulfinyl groups and thiol groups (the amino groups, the mono- or di-C₁₋₁₀ alkylamino groups, the C₁₋₁₀ alkoxy groups, the C₁₋₁₀ thioalkyl groups, the C₁₋₁₀ alkylsulfonyl groups, the sulfonyl groups, the sulfinyl groups and the thiol groups are optionally substituted with one or more C₂₋₉ heterocyclyl groups, one or more C₂₋₁₄ aryl groups or one or more C₂₋₁₄ aryloxide groups (the C₂₋₉ heterocyclyl groups, the C₂₋₁₄ aryl groups and the C₂₋₁₄ aryloxide groups are optionally substituted with one or more substituents independently represented by -V⁵ (wherein V⁵ is the same as V¹, and V¹ is the same as defined above))))) or a C₂₋₁₄ substituted aryl group (the C₂₋₁₄ substituted aryl group may be optionally substituted with one or more substituents independently represented by -V¹ (wherein V¹ is the same as defined above)).
(2) The method for expanding CD34⁺ cells according to (1), wherein A is CR⁴ (wherein R⁴ is the same as defined in (1), B is a sulfur atom, L¹ is a bond, L² is a bond, L³ is NH, L⁴ is a bond or NH, X is a hydroxyl group, and Y is an oxygen atom or a sulfur atom.
(3) The method for expanding CD34⁺ cells according to (1), wherein A is a nitrogen atom, B is NR³⁷ (wherein R³⁷ is a C₁₋₁₀ alkyl group (the C₁₋₁₀ alkyl group may be optionally substituted with one or more substituents selected from the group consisting of carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms))), L¹ is a bond, L² is a bond, L³ is NH, L⁴ is a bond or NH, X is a hydroxyl group, and Y is an oxygen atom or a sulfur atom.
(4) The method for expanding CD34⁺ cells according to (2), wherein A is CH.
(5) The method for expanding CD34⁺ cells according to (3), wherein B is NR³⁸ (wherein R³⁸ is a C₁₋₁₀ alkyl group).
(6) The method for expanding CD34⁺ cells according to any one of (1) to (5), wherein R¹ is a C₂₋₁₄ aryl group (the C₂₋₁₄ aryl group may be optionally substituted with one or more halogen atoms, one or more C₁₋₁₀ alkyl groups or one or more C₁₋₁₀ alkoxy groups (the C₁₋₁₀ alkyl groups and the C₁₋₁₀ alkoxy groups may be optionally substituted with one or more halogen atoms)), and R² is a C₁₋₁₀ alkyl group (the C₁₋₁₀ alkyl group may be optionally substituted with one or more halogen atoms).
(7) The method for expanding CD34⁺ cells according to any one of (1) to (6), which involves addition of at least one blood cell stimulating factor.
(8) The method for expanding CD34⁺ cells according to (7), wherein the blood cell stimulating factor is selected from the group consisting of stem cell factor (SCF), interleukin 3 (IL-3), interleukin 6 (IL-6), interleukin 11 (IL-11), flk2/flt3 ligand (FL), granulocyte colony stimulating factor (G-CSF), granulocyte-macrophage colony stimulating factor (GM-CSF), thrombopoietin (TPO) and erythropoietin (EPO).
(9) The method for expanding CD34⁺ cells according to (8), wherein the blood cell stimulating factor is stem cell factor (SCF).
(10) The method for expanding CD34⁺ cells according to any one of (1) to (9), wherein the CD34⁺ cells are obtained from the bone marrow, the liver, the spleen or peripheral or cord blood.
(11) The method for expanding CD34⁺ cells according to (10), wherein the CD34⁺ cells are obtained from cord blood.
(12) The method for expanding CD34⁺ cells according to (11), wherein CD34⁺ cells obtained from cord blood are cultured in the presence of stem cell factor (SCF).
(13) CD34⁺ cells expanded by the method as defined in any one of (1) to (12).
(14) A material for cell therapy by transplanting CD34⁺ cells expanded by the method as defined in any one of (1) to (12) into a human for treatment of a disease.
(15) The material for cell therapy according to (14), wherein the disease to be treated is leukemia, aplastic anemia, myelodysplastic syndrome, malignant lymphoma, multiple myeloma, myeloproliferative disease, a genetic blood disease, a solid tumor, an autoimmune disease, immunodeficiency, cerebral infarction, myocardial infarction or obstructive arteriosclerosis.

### EFFECTS OF THE INVENTION

According to the method of the present invention, it is possible to expand CD34⁺ cells by culturing them ex vivo. CD34⁺ cells expanded by the method of the present invention are rich in hematopoietic stem cells and hematopoietic progenitor cells can be used as a cell transplant for treatment of diseases. The method of the present invention also makes it possible to provide a cell transplant (graft) soon as required even from a transplant source which can be obtained in a limited amount, by expanding cell populations of hematopoietic stem cells and hematopoietic progenitor cells easily.

The low molecular weight compound to be used in the present invention can be produced by an ordinary process for organic synthesis and is obtained without using any substances from an animal other than human or a microorganism. Therefore, it is possible to prevent contamination with an unknown pathogen or a biomaterial from an animal other than human or a microorganism, as compared with expansion of hematopoietic stem cells using a protein such as cytokines and growth factors obtained by gene recombination technology.

Namely, CD34⁺ cells obtained by the method of the present invention can avoid infection, contamination with foreign genes or immune response to foreign proteins. While being proteins, cytokines and growth factors can be stored or used within very narrow optimal ranges in terms of pH, heat and ion strength, the low molecular weight compound in the present invention can be used and stored under relatively broad ranges of conditions. In addition, because the low molecular weight compound in the present invention can be produced inexpensively and continuously unlike proteins, it is possible to eventually reduce treatment cost.

### BEST MODE FOR CARRYING OUT THE INVENTION

Now, the present invention will be described in further detail.

The terms used herein are defined as follows.

Hematopoietic stem cells are defined as cells having both pluripotency which allows them to differentiate into blood cells of all lineages and the ability to regenerate themselves while maintaining the pluripotency. Multipotential hematopoietic progenitor cells are cells which can differentiate into a plurality of blood cell lineages, though not into all blood cell lineages, but have no self-renewal ability. Unipotential hematopoietic progenitor cells are cells which can differentiate into only one blood cell lineage and have no self-renewal ability. Hematopoietic progenitor cells are a group of cells which covers both multipotential and unipotential hematopoietic progenitor cells. For example, the hematopoietic progenitor cells in the present invention may be granulocyte-macrophage colony forming cells (CFU-GM), eosinophil colony forming cells (EO-CFC), erythroid burst forming cells (BFU-E) as erythroid progenitor cells, megakaryocyte colony forming cells (CFU-MEG) or myeloid stem cells (mixed colony forming cells, CFU-GEMM).

CD34⁺ means expressing CD (cluster of differentiation) 34 antigen on the cell surface. This antigen is a marker for hematopoietic stem cells and/or hematopoietic progenitor cells and disappears as the cell differentiates. Populations of CD34⁺ cells are enriched with hematopoietic stem cells and hematopoietic progenitor cells.

The low molecular weight compound used in the present invention acts on hematopoietic stem cells and/or hematopoietic progenitor cells and shows such an activity that it helps CD34⁺ cells proliferate and survive when they are cultured ex vivo. The low molecular weight compound is capable of proliferate hematopoietic stem cells with minimal differentiation. In some cases of treatment by transplantation of hematopoietic stem cells such as peripheral stem cells and cord blood stem cells, hematopoietic stem cells and/or hematopoietic progenitor cells as the transplant cannot be obtained in sufficient numbers to carry out the transplantation. Use of the low molecular weight compound makes it possible to expand CD34⁺ cells ex vivo and obtain hematopoietic stem cells and hematopoietic progenitor cells in the amount required to carry out the transplantation even in such cases. Specifically speaking, it is possible to expand CD34⁺ cells with minimal differentiation by culturing them in a medium containing the low molecular weight compound and use them for transplantation. It is also possible to expand CD34⁺ cells more efficiently by further adding various cytokines or growth factors, by coculturing them with stromal cells, or by further adding other low molecular weight compounds which act on CD34⁺ cells.

In the method of the present invention, the collected cells to be cultured for transplantation may be an isolated population of either hematopoietic stem cells or hematopoietic progenitor cells or a population containing both of them. The cells may contain either hematopoietic stem cells or hematopoietic progenitor cells and further contain other mature blood cells.

The source of the CD34⁺ cells in the method of the present invention may be any tissue as long as it contains hematopoietic stem cells, and it may be human bone marrow, peripheral blood, peripheral blood containing hematopoietic stem cells mobilized by a cytokine, spleen, liver or cord blood.

The CD34⁺ cells can be cultured in a culture vessel generally used for animal cell culture such as a Petri dish, a flask, a plastic bag, a Teflon (registered trademark) bag, optionally after preliminary coating with an extracellular matrix or a cell adhesion molecule. The material for such a coating may be collagens I to XIX, fibronectin, vitronectin, laminins 1 to 12, nitogen, tenascin, thrombospondin, von Willebrand factor, osteoponin, fibrinogen, various elastins, various proteoglycans, various cadherins, desmocolin, desmoglein, various integrins, E-selectin, P-selectin, L-selectin, immunoglobulin superfamily, Matrigel, poly-D-lysine, poly-L-lysine, chitin, chitosan, Sepharose, alginic acid gel, hydrogel or a fragment thereof. Such a coating material may be a recombinant material having an artificially modified amino acid sequence. The CD34⁺ cells may be cultured by using a bioreactor which can mechanically control the medium composition, pH and the like and obtain high density culture (Schwartz RM, Proc. Natl. Acad. Sci. U.S.A., 88:6760, 1991; Koller MR, Bone Marrow Transplant, 21:653, 1998; Koller, MR, Blood, 82: 378, 1993; Astori G, Bone Marrow Transplant, 35: 1101, 2005).

The nutrient medium to be used in the method of the present invention may be a natural medium, a semi-synthetic medium or a synthetic medium in terms of composition, and may be a solid medium, a semisolid medium or a liquid medium in terms of shape, and any nutrient medium used for animal cell culture, especially for hematopoietic stem cell and/or hematopoietic progenitor cell culture, may be used. As such a nutrient medium, Dulbecco's Modified Eagles's Medium (DMEM), Ham's Nutrient Mixture H12 Mixture F12, McCoy's 5A medium, Eagles's Minimum Essential Medium (EMEM), RPMI1640 medium, Isocove's Modified Dulbecco's Medium (IMDM), StemPro34 (Invitrogen), X-VIVO 10 (Cambrex), X-VIVO 15 (Cambrex), HPGM (Cambrex), StemSpan H3000 (Stemcell Technologies), StemSpan SFEM (Stemcell Technologies), Stemline II (Sigma-Aldrich) or QBSF-60 (Quality Biological) may be mentioned.

Such a medium may contain sodium, potassium, calcium, magnesium, phosphorus, chlorine, amino acids, vitamins, cytokines, hormones, antibiotics, serum, fatty acids, saccharides or the like. In the culture, other chemical components or biological components may be incorporated singly or in combination, as the case requires. Such components to be incorporated in the medium may be fetal calf serum, human serum, horse serum, insulin, transfferin, lactoferrin, cholesterol, ethanolamine, sodium selenite, monothioglycerol, 2-mercaptoethanol, bovine serum albumin, sodium pyruvate, polyethylene glycol, various vitamins, various amino acids, various cytokines, various growth factors or the like. The cytokines to be added to the medium may be interleukin 1 (IL-1), interleukin 2 (IL-2), interleukin 3 (IL-3), interleukin 4 (IL-4), interleukin 5 (IL-5), interleukin 6 (IL-6), interleukin 7 (IL-7), interleukin 8 (IL-8), interleukin 9 (IL-9), interleukin 10 (IL-10), interleukin 11 (IL-11), interleukin 12 (IL-12), interleukin 13 (IL-13), interleukin 14 (IL-14), interleukin 15 (IL-15), interleukin 18 (IL-18), interleukin 21 (IL-21), interferon-α (IFN-α), interferon-β (IFN-β), interferon-γ (IFN-γ), granulocyte colony stimulating factor (G-CSF), monocyte colony stimulating factor (M-CSF), granulocyte-macrophage colony stimulating factor (GM-CSF), stem cell factor (SCF), flk2/flt3 ligand (FL), leukemia inhibitory factor (LIF), oncostatin M (OM), erythropoietin (EPO) and thrombopoietin (TPO), but are not restricted to those mentioned above. The growth factors to be added to the medium may be transforming growth factor-β (TGF-β), macrophage inflammatory protein-1α (MIP-1α), epidermal growth factor (EGF), fibroblast growth factor (FGF), nerve growth factor (NGF), hepatocyte growth factor (HGF), protease nexin I, protease nexin II, platelet-derived growth factor (PDGF), cholinergic differentiation factor (CDF), chemokines, Notch ligand (such as Delta 1) and Wnt protein, but are not restricted to those mentioned above. Besides, recombinant cytokines or growth factors having an artificially modified amino acid sequence such as IL-6/soluble IL-6 receptor complex, and Hyper IL-6 (IL-6/soluble IL-6 receptor fusion protein) may also be added.

Among the above-mentioned cytokines and growth factors, preferred are stem cell factor (SCF), interleukin 3 (IL-3), interleukin 6 (IL-6), interleukin 11 (IL-11), flk2/flt3 ligand (FL), granulocyte colony stimulating factor (G-CSF), granulocyte-macrophage colony stimulating factor (GM-CSF), thrombopoietin (TPO), erythropoietin (EPO), Notch ligand (Delta 1) and the like, and more preferred are stem cell factor (SCF), flk2/flt3 ligand (FL), thrombopoietin (TPO) and the like.

Cytokines and growth factors are usually added to culture at a concentration of 0.1 ng/mL to 1000 ng/mL, preferably from 1 ng/mL to 100 ng/mL.

In addition, at least one chemical substance known to be effective for expansion of hematopoietic stem cells may be added to the medium singly or in combination. Examples of such substances include copper chelators represented by tetraethylenepentamine, histone deacetylase inhibitors represented by trichostain A, DNA methylase inhibitors represented by 5-aza-2'-deoxycytidine, retinoic acid receptor ligands represented by all-trans retinoic acid, aldehyde dehydrogenase inhibitors represented by dimethylaminobenzaldehyde, but they are not restricted to those mentioned above.

The chemical components and biological components mentioned above may be used not only by adding them to the medium but also by immobilizing them onto the surface of the substrate or support used for the culture, specifically speaking, by dissolving a component to be used in an appropriate solvent, coating the substrate or support with the resulting solution and then washing away an excess of the component. Such a component to be used may be added to the substrate or support preliminarily coated with a substance which binds to the component.

When the low molecular weight compound of the present invention is added to such a medium as mentioned above, it is first dissolved in an appropriate solvent and added to the medium so that the concentration of the compound will be from 1 ng/mL to 100 µg/mL, preferably from 2 ng/mL to 50 µg/mL, more preferably from 20 ng/mL to 10 µg/mL, particularly preferably from 300 ng/mL to 3 µg/mL. Examples of the appropriate solvent include dimethyl sulfoxide (DMSO) and various alcohols, but it is not restricted thereto. The low molecular weight compound of the present invention may be immobilized on the surface of the substrate or support used for the culture. The low molecular weight compound of the present invention may be provided or stored in a certain form, for example, in a solid form as a tablet, a pill, a capsule or a granule, in a liquid form as a solution or suspension in an appropriate solvent or resolvent, or in the form bound to the substrate or support. When it is formulated into such a form, additives such as a preservative like p-hydroxybenzoates, an excipient like lactose, glucose, sucrose and mannitol; a lubricant like magnesium stearate and talc; a binder like polyvinyl alcohol, hydroxypropylcellulose and gelatin, a surfactant like fatty acid esters, a plasticizer like glycerin may be added. The additives are not restricted to those mentioned above and a person skilled in the art can use any additives of choice.

The CD34⁺ cells are cultured usually at a temperature of from 25 to 39°C, preferably from 33 to 39°C, in the atmosphere having a CO₂ concentration of from 4 to 10 vol%, preferably from 4 to 6 vol%, usually for a period of from 3 to 35 days, preferably from 5 to 21 days, more preferably from 7 to 14 days.

In the method of the present invention, when the CD34⁺ cells are cocultured with stromal cells, collected bone marrow cells may be grown directly in culture. Alternatively, it is possible to separate collected bone marrow into stromal cells, CD34⁺ cells, and coculture the CD34⁺ cells with stromal cells from an individual other than the bone marrow donor. It is also possible to first grow stromal cells only and add and grow CD34⁺ cells in coculture in such a medium under such conditions as mentioned above.

CD34⁺ cells expanded by the method of the present invention can be used as a cell transplant. Because hematopoietic stem cells can differentiate into blood cells of all lineages, they may be transplanted after differentiated into a certain type of blood cells ex vivo. CD34⁺ cells expanded by the method of the present invention may be transplanted as they are, or after enrichment using a cell surface antigen as an index, for example, by a magnetic bead method or by a cell sorting method. Such a cell surface antigen molecule may be CD2, CD3, CD4, CD8, CD13, CD14, CD15, CD16, CD19, CD24, CD33, CD34, CD38, CD41, CD45, CD56, CD66, CD90, CD133 or glycophorin A, but is not restricted thereto. The expanded CD34⁺ cells may be transplanted to its donor or another individual.

Namely, CD34⁺ cells expanded by the method of the present invention can be used as a transplant for hematopoietic stem cell therapy as a substitute for conventional bone marrow or cord blood transplantation. The transplantation of CD34⁺ cells expanded by using the low molecular compound of the present invention is carried out in the same manner as conventional bone marrow or cord blood transplantation, except for the cells to be used. The transplant may be a composition containing a buffer solution, an antibiotic, a pharmaceutical in addition to CD34⁺ cells expanded by the method of the present invention.

The CD34⁺ cell transplant obtained by the method of the present invention is useful for treatment of not only various types of leukemia but also various diseases. For example, in a case of treatment of a solid cancer patient by chemotherapy or radiotherapy which may cause myelosuppression as a side effect, the patient can recover from hematopoietic damage quickly if the CD34⁺ cells in bone marrow collected from the patient preliminarily to the treatment are expanded ex vivo and returned to the patient after the treatment. Thus, a more intense chemotherapy becomes available with an improved therapeutic effect. It is also possible to alleviate a deficiency in a certain type of blood cells in a patient by differentiating CD34⁺ cells obtained by the method of the present invention into such a type of blood cells and returning them into the patient. The method of the present invention is effective against diseases accompanying decrease in hematopoietic cells and/or hematopoietic insufficiency, diseases accompanying increase in hematopoietic cells, diseases accompanying hematopoietic dysfunction, decrease in immunocytes, increase in immunocytes, diseases accompanying autoimmunity, immune dysfunction and ischemic diseases.

As specific examples, chronic granulomatosis, severe combined immunodeficiency syndrome, adenylate deaminase (ADA) deficiency, agammaglobulinemia, Wiskott-Aldrich syndrome, Chediak-Higashi syndrome, immunodeficiency syndrome such as acquired immunodeficiency syndrome (AIDS), C3 deficiency, congenital anemia such as thalassemia, hemolytic anemia due to enzyme deficiency and sicklemia, lysosomal storage disease such as Gaucher's disease and mucopolysaccharidosis, adrenoleukodystrophy, various kinds of cancers and tumors, especially blood cancers such as acute or chronic leukemia, Fanconi syndrome, aplastic anemia, malignant lymphoma, Hodgkin's disease, multiple myeloma, chronic hepatopathy, renal failure, massive blood transfusion of bank blood or during operation, hepatitis B, hepatitis C, severe infections, systemic lupus erythematodes, articular rheumatism, xerodermosteosis, systemic sclerosis, polymyositis, dermatomyositis, mixed connective tissue disease, polyarteritis nodosa, Hashimoto's disease, Basedow's disease, myasthenia gravis, insulin dependent diabetes mellitus, autoimmune hemolytic anemia, snake bite, hemolytic uremic syndrome, hypersplenism, bleeding, Bernard-Soulier syndrome, Glanzmann's thrombasthenia, uremia, myelodysplastic syndrome, polycythemia rubra vera, erythremia, essential thrombocythemia, myeloproliferative disease, cerebral infarction, myocardial infarction, obstructive arteriosclerosis and the like may be mentioned.

Preferred embodiments of expansion of CD34⁺ cells and transplantation of the expanded CD34⁺ cells according to the present invention will be described below.

First, for expansion of CD34⁺ cells, cord blood, bone marrow, peripheral blood or the like is collected, and a cell population enriched with CD34⁺ cells is separated from it. CD34⁺ cells can be separated by density centrifugation combined with magnetic cell sorting (MACS) or flow cytometry. For example, CPD (citrate-phosphate-dextran)-treated blood is fractioned by density centrifugation to separate and collect a monocyte enriched fraction (hereinafter referred to as nucleated cell fraction). As density centrifugation, dextran or Ficoll density centrifugation, Ficoll-paque density gradient centrifugation, Percoll discontinuous density gradient centrifugation or Lymphoprep density gradient centrifugation may be mentioned. Then, magnetic beads coated with an anti-human CD34 monoclonal antibody (Miltenyi Biotec; hereinafter referred to CD34 antibody magnetic beads) and the collected nucleated cell fraction are mixed and incubated at from 2 to 8°C (for about 30 minutes) to bind CD34⁺ cells to the antibody magnetic beads. The antibody magnetic bead/CD34⁺ cell complexes are separated and collected by a specialized magnetic cell separator such as autoMACS system (Miltenyi Biotec). The CD34⁺ cells thus obtained are cultured using the low molecular weight compound of the present invention. The conditions, incubator and medium for culturing CD34⁺ cells, the species and amount of the low molecular weight compound, the kinds and amounts of additives and the incubation time and temperature may be selected appropriately from those disclosed herein by the person in charge, but are not restricted thereto.

After culturing, the total cell count is measured by trypan blue assay, Flow-Count^{™} fluorosphere assay or the like, while the cell culture is stained with an anti CD34 antibody labeled with a fluorescent dye such as FITC (fluorescein isothiocyanate), PE (phycoerythrin) or APC (allophycocyanin), and the proportion of CD34⁺ cells is analyzed by flow cytometry. Thus, it is possible to determine how much CD34⁺ cells are expanded in the cell culture. Expanded CD34⁺ cells may be infused by drip, for example, in the case of treatment of leukemia, into patients pretreated with an anticancer drug, total body irradiation or an immunosuppressive drug for eradication of cancer cells or for facilitation of donor cell engraftment. The disease to be treated, the pretreatment and the cell transplantation method are selected appropriately by the person in charge. The engraftment of transplanted hematopoietic stem cells and/or hematopoietic progenitor cells in the recipient, the recovery of hematopoiesis, the presence of side effects of the transplantation and the therapeutic effect of the transplantation can be judged by an ordinary assay used in transplantation therapy.

As described above, the present invention makes it possible to expand hematopoietic stem cells and/or hematopoietic progenitor cells and to carryout transplantation therapy and gene therapy safely and easily in a short term by using the expanded cells.

Now, the compound to be used in the present invention will be described in terms of the definitions of terms used for it and its best mode.

In the present invention, "n" denotes normal, "i" denotes iso, "s" denotes secondary, "t" denotes tertiary, "c" denotes cyclo, "o" denotes ortho, "m" denotes meta, "p" denotes para, "Ph" denotes phenyl, "Py" denotes pyridyl, "Naphthyl" denotes naphthyl, "Me" denotes methyl, "Et" denotes ethyl, "Pr" denotes propyl, "Bu" denotes butyl, and ."Ac" denotes acetyl.

First, the terms in the respective substituents R¹ to R⁴⁴ will be explained.

As a halogen atom, a fluorine atom, a chlorine atom, a bromine atom or an iodine atom may be mentioned.

A C₁₋₃ alkyl group may be linear, branched or a C₃ cycloalkyl group, and methyl, ethyl, n-propyl, i-propyl and c-propyl and the like may be mentioned.

A C₁₋₆ alkyl group may be linear, branched or a C₃₋₆ cycloalkyl group, and for example, in addition to those mentioned above, n-butyl, i-butyl, s-butyl, t-butyl, c-butyl, 1-methyl-c-propyl, 2-methyl-c-propyl, n-pentyl, 1-methyl-n-butyl, 2-methyl-n-butyl, 3-methyl-n-butyl, 1,1-dimethyl-n-propyl, 1,2-dimethyl-n-propyl, 2,2-dimethyl-n-propyl, 1-ethyl-n-propyl, c-pentyl, 1-methyl-c-butyl, 2-methyl-c-butyl, 3-methyl-c-butyl, 1,2-dimethyl-c-propyl, 2,3-dimethyl-c-propyl, 1-ethyl-c-propyl, 2-ethyl-c-propyl, n-hexyl, 1-methyl-n-pentyl, 2-methyl-n-pentyl, 3-methyl-n-pentyl, 4-methyl-n-pentyl, 1,1-dimethyl-n-butyl, 1,2-dimethyl-n-butyl, 1,3-dimethyl-n-butyl, 2,2-dimethyl-n-butyl, 2,3-dimethyl-n-butyl, 3,3-dimethyl-n-butyl, 1-ethyl-n-butyl, 2-ethyl-n-butyl, 1,1,2-trimethyl-n-propyl, 1,2,2-trimethyl-n-propyl, 1-ethyl-1-methyl-n-propyl, 1-ethyl-2-methyl-n-propyl, c-hexyl, 1-methyl-c-pentyl, 2-methyl-c-pentyl, 3-methyl-c-pentyl, 1-ethyl-c-butyl, 2-ethyl-c-butyl, 3-ethyl-c-butyl, 1,2-dimethyl-c-butyl, 1,3-dimethyl-c-butyl, 2,2-dimethyl-c-butyl, 2,3-dimethyl-c-butyl, 2,4-dimethyl-c-butyl, 3,3-dimethyl-c-butyl, 1-n-propyl-c-propyl, 2-n-propyl-c-propyl, 1-i-propyl-c-propyl, 2-i-propyl-c-propyl, 1,2,2-trimethyl-c-propyl, 1,2,3-trimethyl-c-propyl, 2,2,3-trimethyl-c-propyl, 1-ethyl-2-methyl-c-propyl, 2-ethyl-1-methyl-c-propyl, 2-ethyl-2-methyl-c-propyl, 2-ethyl-3-methyl-c-propyl and the like may be mentioned.

A C₁₋₁₀ alkyl group may be linear, branched or a C₃₋₁₀ cycloalkyl group, and for example, in addition to those mentioned above, 1-methyl-1-ethyl-n-pentyl, 1-heptyl, 2-heptyl, 1-ethyl-1,2-dimethyl-n-propyl, 1-ethyl-2,2-dimethyl-n-propyl, 1-octyl, 3-octyl, 4-methyl-3-n-heptyl, 6-methyl-2-n-heptyl, 2-propyl-1-n-heptyl, 2,4,4-trimethyl-1-n-pentyl, 1-nonyl, 2-nonyl, 2,6-dimethyl-4-n-heptyl, 3-ethyl-2,2-dimethyl-3-n-pentyl, 3,5,5-trimethyl-1-n-hexyl, 1-decyl, 2-decyl, 4-decyl, 3,7-dimethyl-1-n-octyl, 3,7-dimethyl-3-n-octyl and the like may be mentioned.

As a C₂₋₆ alkynyl group, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 1-n-propyl-2-propynyl, 2-ethyl-3-butynyl, 1-methyl-1-ethyl-2-propynyl, 1-i-propyl-2-propynyl and the like may be mentioned.

A C₂₋₆ alkenyl group may be linear, branched or a C₃₋₆ cycloalkenyl group, and for example, ethenyl, 1-propenyl, 2-propenyl, 1-methyl-1-ethenyl, 1-butenyl, 2-butenyl, 3-butenyl, 2-methyl-1-propenyl, 2-methyl-2-propenyl, 1-ethylethenyl, 1-methyl-1-propenyl, 1-methyl-2-propenyl, 1-pentenyl, 2-pentenyl, 3-pentenyl, 4-pentenyl, 1-n-propylethenyl, 1-methyl-1-butenyl, 1-methyl-2-butenyl, 1-methyl-3-butenyl, 2-ethyl-2-propenyl, 2-methyl-1-butenyl, 2-methyl-2-butenyl, 2-methyl-3-butenyl, 3-methyl-1-butenyl, 3-methyl-2-butenyl, 3-methyl-3-butenyl, 1,1-dimethyl-2-propenyl, 1-i-propylethenyl, 1,2-dimethyl-1-propenyl, 1,2-dimethyl-2-propenyl, 1-c-pentenyl, 2-c-pentenyl, 3-c-pentenyl, 1-hexenyl, 2-hexenyl, 3-hexenyl, 4-hexenyl, 5-hexenyl, 1-methyl-1-pentenyl, 1-methyl-2-pentenyl, 1-methyl-3-pentenyl, 1-methyl-4-pentenyl, 1-n-butylethenyl, 2-methyl-1-pentenyl, 2-methyl-2-pentenyl, 2-methyl-3-pentenyl, 2-methyl-4-pentenyl, 2-n-propyl-2-propenyl, 3-methyl-1-pentenyl, 3-methyl-2-pentenyl, 3-methyl-3-pentenyl, 3-methyl-4-pentenyl, 3-ethyl-3-butenyl, 4-methyl-1-pentenyl, 4-methyl-2-pentenyl, 4-methyl-3-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1-methyl-2-ethyl-2-propenyl, 1-s-butylethenyl, 1,3-dimethyl-1-butenyl, 1,3-dimethyl-2-butenyl, 1,3-dimethyl-3-butenyl, 1-i-butylethenyl, 2,2-dimethyl-3-butenyl, 2,3-dimethyl-1-butenyl, 2,3-dimethyl-2-butenyl, 2,3-dimethyl-3-butenyl, 2-i-propyl-2-propenyl, 3,3-dimethyl-1-butenyl, 1-ethyl-1-butenyl, 1-ethyl-2-butenyl, 1-ethyl-3-butenyl, 1-n-propyl-1-propenyl, 1-n-propyl-2-propenyl, 2-ethyl-1-butenyl, 2-ethyl-2-butenyl, 2-ethyl-3-butenyl, 1,1,2-trimethyl-2-propenyl, 1-t-butylethenyl, 1-methyl-1-ethyl-2-propenyl, 1-ethyl-2-methyl-1-propenyl, 1-ethyl-2-methyl-2-propenyl, 1-i-propyl-1-propenyl, 1-i-propyl-2-propenyl, 1-methyl-2-c-pentenyl, 1-methyl-3-c-pentenyl, 2-methyl-1-c-pentenyl, 2-methyl-2-c-pentenyl, 2-methyl-3-c-pentenyl, 2-methyl-4-c-pentenyl, 2-methyl-5-c-pentenyl, 2-methylene-c-pentyl, 3-methyl-1-c-pentenyl, 3-methyl-2-c-pentenyl, 3-methyl-3-c-pentenyl, 3-methyl-4-c-pentenyl, 3-methyl-5-c-pentenyl, 3-methylene-c-pentyl, 1-c-hexenyl, 2-c-hexenyl, 3-c-hexenyl and the like may be mentioned.

A C₂₋₉ heterocyclyl group may be a monocyclic or fused bicyclic heterocyclic group containing at least one atom optionally selected from nitrogen atoms, oxygen atoms and sulfur atoms and from 2 to 9 carbon atoms, and specifically mentioned are:

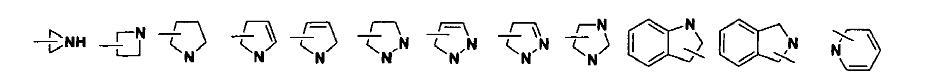

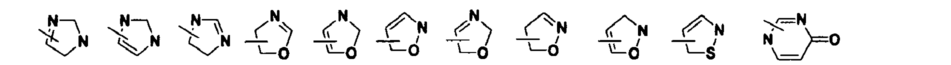

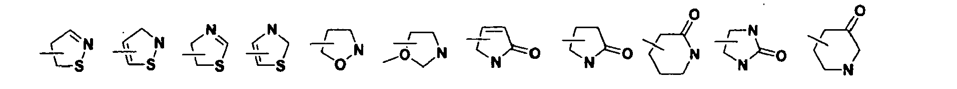

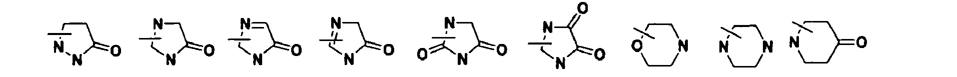

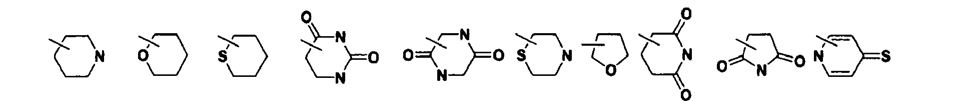

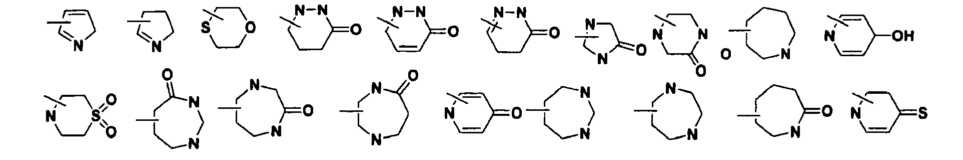

A nitrogen-containing C₂₋₉ cyclyl group may be, among those give above, a heteromonocyclic or fused heterobicyclic group which contains least one nitrogen atom, may contain at least one atom optionally selected from oxygen atoms and sulfur atoms and contains from 2 to 9 carbon atoms, and specifically mentioned are:

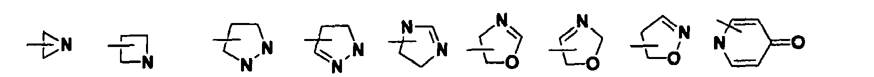

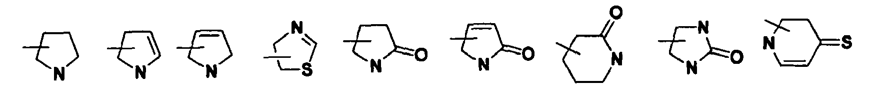

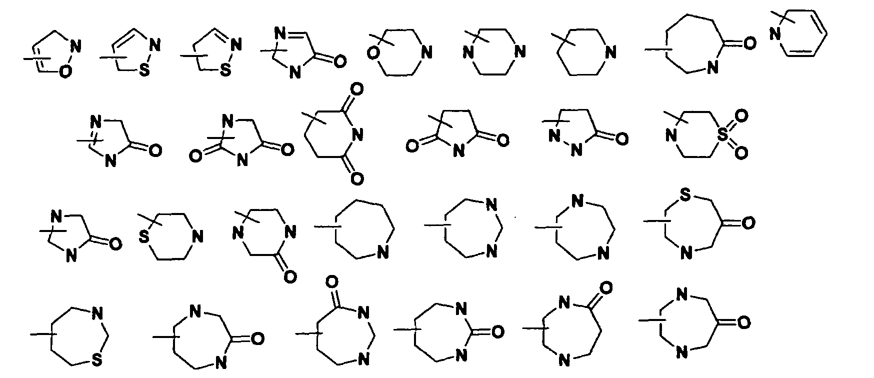

A substituted C₂₋₉ heterocyclyl group may be, among the C₂₋₉ heterocyclyl groups and the nitrogen-containing C₂₋₉ heterocyclyl groups, a heteromonocyclic or fused heterobicyclic group which has no nitrogen atoms and one or three carbonyl groups or thiocarbonyl groups as ring constituting carbon atoms, and specifically mentioned are:

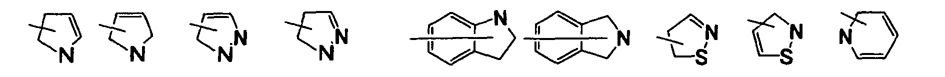

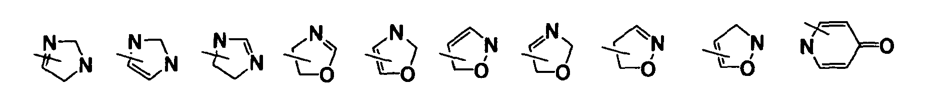

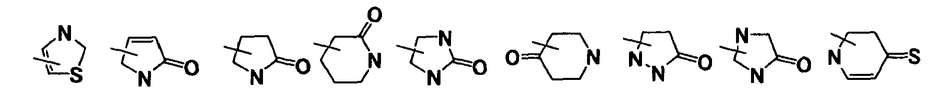

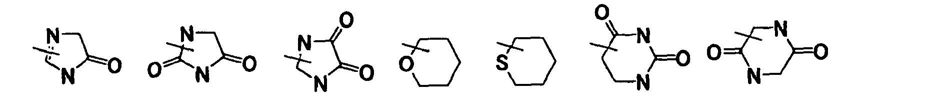

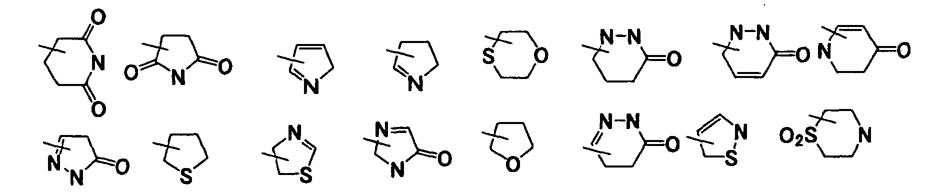

A C₂₋₁₄ aryl group may be a C₆₋₁₄ aryl group containing no hetero atoms as ring constituting atoms or a C₂₋₉ aromatic heterocyclic group, and a C₂₋₉ aromatic heterocyclic group may be a 5 to 7-membered C₂₋₆ heteromonocyclic group or 8 to 10-membered C₅₋₉ fused heterobicyclic group containing from 1 to 3 oxygen atoms, nitrogen atoms or sulfur atoms singly or in combination.

As a C₆₋₁₄ aryl group containing no hetero atoms, a phenyl group, a 1-indenyl group, a 2-indenyl group, a 3-indenyl group, a 4-indenyl group, a 5-indenyl group, a 6-indenyl group, a 7-indenyl group, an α-naphthyl group, a β-naphthyl group, a 1-tetrahydronaphthyl group, a 2-tetrahydronaphthyl group, a 5-tetrahydronaphthyl group, a 6-tetrahydronaphthyl group, an o-biphenylyl group, a m-biphenylyl group, a p-biphenylyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group or the like may be mentioned.

A 5 to 7-membered C₂₋₆ heteromonocyclic group may be a 2-thienyl group, a 3-thienyl group, a 2-furyl group, a 3-furyl group, a 2-pyranyl group, a 3-pyranyl group, a 4-pyranyl group, a 1-pyrrolyl group, a 2-pyrrolyl group, a 3-pyrrolyl group, a 1-imidazolyl group, a 2-imidazolyl group, a 4-imidazolyl group, a 1-pyrazolyl group, a 3-pyrazolyl group, a 4-pyrazolyl group, a 2-thiazolyl group, a 4-thiazolyl group, a 5-thiazolyl group, a 3-isothiazolyl group, a 4-isothiazolyl group, a 5-isothiazolyl group, a 2-oxazolyl group, a 4-oxazolyl group, a 5-oxazolyl group, a 3-isoxazolyl group, a 4-isoxazolyl group, a 5-isoxazolyl group, a 2-pyridyl group, a 3-pyridyl group, a 4-pyridyl group, a 2-pyrazinyl group, a 2-pyrimidinyl group, a 4-pyrimidinyl group, a 5-pyrimidinyl group, a 3-pyridazinyl group, a 4-pyridazinyl group, a 2-1,3,4-oxadiazolyl group, a 2-1,3,4-thiadiazolyl group, a 3-1,2,4-oxadiazolyl group, a 5-1,2,4-oxadiazolyl group, a 3-1,2,4-thiadiazolyl group, a 5-1,2,4-thiadiazolyl group, a 3-1,2,5-oxadiazolyl group, a 3-1,2,5-thiadiazolyl group or the like.

A 8 to 10-membered C₅₋₉ fused heterocyclic group may be a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 4-isobenzofuranyl group, a 5-isobenzofuranyl group, a 2-benzothienyl group, a 3-benzothienyl group, a 4-benzothienyl group, a 5-benzothienyl group, a 6-benzothienyl group, a 7-benzothienyl group, a 1-isobenzothienyl group, a 4-isobenzothienyl group, a 5-isobenzothienyl group, a 2-chromenyl group, a 3-chromenyl group, a 4-chromenyl group, a 5-chromenyl group, a 6-chromenyl group, a 7-chromenyl group, a 8-chromenyl group, a 1-indolizinyl group, a 2-indolizinyl group, a 3-indolizinyl group, a 5-indolizinyl group, a 6-indolizinyl group, a 7-indolizinyl group, a 8-indolizinyl group, a 1-isoindolyl group, a 2-isoindolyl group, a 4-isoindolyl group, a 5-isoindolyl group, a 1-indolyl group, a 2-indolyl group, a 3-indolyl group, a 4-indolyl group, a 5-indolyl group, a 6-indolyl group, a 7-indolyl group, a 1-indazolyl group, a 2-indazolyl group, a 3-indazolyl group, a 4-indazolyl group, a 5-indazolyl group, a 6-indazolyl group, a 7-indazolyl group, a 1-purinyl group, a 2-purinyl group, a 3-purinyl group, a 6-purinyl group, a 7-purinyl group, a 8-purinyl group, a 2-quinolyl group, a 3-quinolyl group, a 4-quinolyl group, a 5-quinolyl group, a 6-quinolyl group, a 7-quinolyl group, a 8-quinolyl group, a 1-isoquinolyl group, a 3-isoquinolyl group, a 4-isoquinolyl group, a 5-isoquinolyl group, a 6-isoquinolyl group, a 7-isoquinolyl group, a 8-isoquinolyl group, a 1-phthalazinyl group, a 5-phthalazinyl group, a 6-phthalazinyl group, a 1-2,7-naphthyridinyl group, a 3-2,7-naphthyridinyl group, a 4-2,7-naphthyridinyl group, a 1-2,6-naphthyridinyl group, a 3-2,6-naphthyridinyl group, a 4-2,6-naphthyridinyl group, a 2-1,8-naphthyridinyl group, a 3-1,8-naphthyridinyl group, a 4-1,8-naphthyridinyl group, a 2-1,7-naphthyridinyl group, a 3-1,7-naphthyridinyl group, a 4-1,7-naphthyridinyl group, a 5-1,7-naphthyridinyl group, a 6-1,7-naphthyridinyl group, a 8-1,7-naphthyridinyl group, 2-1,6-naphthyridinyl group, a 3-1,6-naphthyridinyl group, a 4-1,6-naphthyridinyl group, a 5-1,6-naphthyridinyl group, a 7-1,6-naphthyridinyl group, a 8-1,6-naphthyridinyl group, a 2-1,5-naphthyridinyl group, a 3-1,5-naphthyridinyl group, a 4-1,5-naphthyridinyl group, a 6-1,5-naphthyridinyl group, a 7-1,5-naphthyridinyl group, a 8-1,5-naphthyridinyl group, a 2-quinoxalinyl group, a 5-quinoxalinyl group, a 6-quinoxalinyl group, a 2-quinazolinyl group, a 4-quinazolinyl group, a 5-quinazolinyl group, a 6-quinazolinyl group, a 7-quinazolinyl group, a 8-quinazolinyl group, a 3-cinnolinyl group, a 4-cinnolinyl group, a 5-cinnolinyl group, a 6-cinnolinyl group, a 7-cinnolinyl group, a 8-cinnolinyl group, a 2-pteridinyl group, a 4-pteridinyl group, a 6-pteridinyl group, a 7-pteridinyl group or the like.

A C₂₋₁₄ substituted aryl group may be a fused bycyclic or tricyclic group resulting from fusion of the above-mentioned C₂₋₁₄ aryl group with a C₂₋₉ heterocyclyl group, a nitrogen-containing C₂₋₉ heterocyclyl group or a substituted C₂₋₉ heterocyclyl group, and specifically mentioned are:

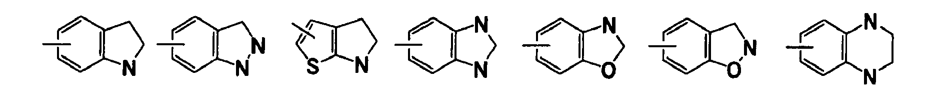

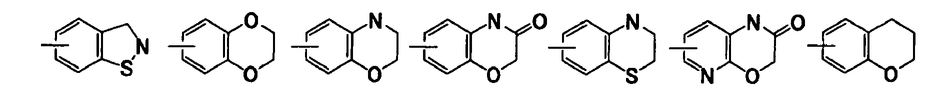

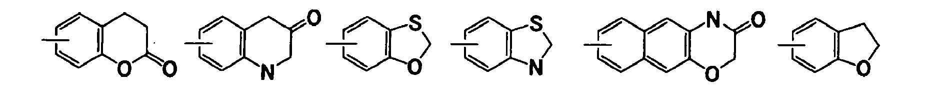

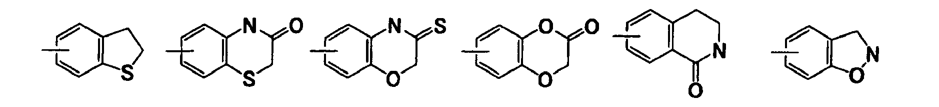

A C₂₋₁₄ aryloxide group is a group obtained by oxidizing a nitrogen atom or a sulfur atom in the C₂₋₁₄ aryl group with oxygen, specifically, a 2-thiophene-S-oxide group, a 3-thiophene-S-oxide group, a 2-thiophene-S-dioxide group, a 3-thiophene-S-dioxide group, a 1-pyrrole-N-oxide group, a 2-pyrrole-N-oxide group, 3-pyrrole-N-oxide, a 1-imidazole-N-oxide group, a 2-imidazole-N-oxide group, a 4-imidazole-N-oxide group, a 1-pyrazole-N-oxide group, a 3-pyrazole-N-oxide group, a 4-pyrazole-N-oxide group, a 2-thiazole-N-oxide group, a 4-thiazole-N-oxide group, a 5-thiazole-N-oxide group, a 3-isothiazole-N-oxide group, a 4-isothiazole-N-oxide group, a 5-isothiazole-N-oxide group, a 2-thiazole-S-oxide group, a 4-thiazole-S-oxide group, a 5-thiazole-S-oxide group, a 3-isothiazole-S-oxide group, a 4-isothiazole-S-oxide group, a 5-isothiazole-S-oxide group, a 2-thiazole-S-dioxide group, a 4-thiazole-S-dioxide group, a 5-thiazole-S-dioxide group, a 3-isothiazole-S-dioxide group, a 4-isothiazole-S-dioxide group, a 5-isothiazole-S-dioxide group, a 2-oxazole-N-oxide group, a 4-oxazole-N-oxide group, a 5-oxazole-N-oxide group, a 3-isooxazole-N-oxide group, a 4-isooxazole-N-oxide group, a 5-isooxazole-N-oxide group, a 2-pyridine-N-oxide group, a 3-pyridine-N-oxide group, a 4-pyridine-N-oxide group or the like may be mentioned.

A C₂₋₁₄ aryloxy group may be a C₆₋₁₄ aryloxy group containing no hetero atoms as ring constituting atoms or a C₂₋₉ aromatic heterocyclyloxy group, and a C₂₋₉ aromatic heterocyclyloxy group may be a 5 to 7-membered C₂₋₆ monocyclic heterocyclyloxy group or 8 to 10-membered C₅₋₉ fused bicyclic heterocyclyloxy group containing from 1 to 3 oxygen atoms, nitrogen atoms or sulfur atoms singly or in combination.

As a C₆₋₁₄ aryloxy group containing no hetero atoms, a phenyloxy group, a 1-indenyloxy group, a 2-indenyloxy group, a 3-indenyloxy group, a 4-indenyloxy group, a 5-indenyloxy group, a 6-indenyloxy group, a 7-indenyloxy group, an α-naphthyloxy group, a β-naphthyloxy group, a 1-tetrahydronaphthyloxy group, a 2-tetrahydronaphthyloxy group, a 5-tetrahydronaphthyloxy group, a 6-tetrahydronaphthyloxy group, an o-biphenylyloxy group, a m-biphenylyloxy group, a p-biphenylyloxy group, a 1-anthryloxy group, a 2-anthryloxy group, a 9-anthryloxy group, a 1-phenanthryloxy group, a 2-phenanthryloxy group, a 3-phenanthryloxy group, a 4-phenanthryloxy group, a 9-phenanthryloxy group or the like may be mentioned.

A 5 to 7-membered C₂₋₆ monocyclic heterocyclyloxy group may be a 2-thienyloxy group, a 3-thienyloxy group, a 2-furyloxy group, a 3-furyloxy group, a 2-pyranyloxy group, a 3-pyranyloxy group, a 4-pyranyloxy group, a 1-pyrrolyloxy group, a 2-pyrrolyloxy group, a 3-pyrrolyloxy group, a 1-imidazolyloxy group, a 2-imidazolyloxy group, a 4-imidazolyloxy group, a 1-pyrazolyloxy group, a 3-pyrazolyloxy group, a 4-pyrazolyloxy group, a 2-thiazolyloxy group, a 4-thiazolyloxy group, a 5-thiazolyloxy group, a 3-isothiazolyloxy group, a 4-isothiazolyloxy group, a 5-isothiazolyloxy group, a 2-oxazolyloxy group, a 4-oxazolyloxy group, a 5-oxazolyloxy group, a 3-isoxazolyloxy group, a 4-isoxazolyloxy group, a 5-isoxazolyloxy group, a 2-pyridyloxy group, a 3-pyridyloxy group, a 4-pyridyloxy group, a 2-pyrazinyloxy group, a 2-pyrimidinyloxy group, a 4-pyrimidinyloxy group, a 5-pyrimidinyloxy group, a 3-pyridazinyloxy group, a 4-pyridazinyloxy group, a 2-1,3,4-oxadiazolyloxy group, a 2-1,3,4-thiadiazolyloxy group, a 3-1,2,4-oxadiazolyloxy group, a 5-1,2,4-oxadiazotyloxy group, a 3-1,2,4-thiadiazolyloxy group, a 5-1,2,4-thiadiazolyloxy group, a 3-1,2,5-oxadiazolyloxy group, a 3-1,2,5-thiadiazolyloxy group or the like.

A 8 to 10-membered C₅₋₉ fused bicyclic heterocyclyloxy group may be a 2-benzofuranyloxy group, a 3-benzofuranyloxy group, a 4-benzofuranyloxy group, a 5-benzofuranyloxy group, a 6-benzofuranyloxy group, a 7-benzofuranyloxy group, a 1-isobenzofuranyloxy group, a 4-isobenzofuranyloxy group, a 5-isobenzofuranyloxy group, a 2-benzothienyloxy group, a 3-benzothienyloxy group, a 4-benzothienyloxy group, a 5-benzothienyloxy group, a 6-benzothienyloxy group, a 7-benzothienyloxy group, a 1-isobenzothienyloxy group, a 4-isobenzothienyloxy group, a 5-isobenzothienyloxy group, a 2-chromenyloxy group, a 3-chromenyloxy group, a 4-chromenyloxy group, a 5-chromenyloxy group, a 6-chromenyloxy group, a 7-chromenyloxy group, a 8-chromenyloxy group, a 1-indolizinyloxy group, a 2-indolizinyloxy group, a 3-indolizinyloxy group, a 5-indolizinyloxy group, a 6-indolizinyloxy group, a 7-indolizinyloxy group, a 8-indolizinyloxy group, a 1-isoindolyloxy group, a 2-isoindolyloxy group, a 4-isoindolyloxy group, a 5-isoindolyloxy group, a 1-indolyloxy group, a 2-indolyloxy group, a 3-indolyloxy group, a 4-indolyloxy group, a 5-indolyloxy group, a 6-indolyloxy group, a 7-indolyloxy group, 1-indazolyloxy group, a 2-indazolyloxy group, a 3-indazolyloxy group, a 4-indazolyloxy group, a 5-indazolyloxy group, a 6-indazolyloxy group, a 7-indazolyloxy group, a 1-purinyloxy group, a 2-purinyloxy group, a 3-purinyloxy group, a 6-purinyloxy group, a 7-purinyloxy group, a 8-purinyloxy group, a 2-quinolyloxy group, a 3-quinolyloxy group, a 4-quinolyloxy group, a 5-quinolyloxy group, a 6-quinolyloxy group, a 7-quinolyloxy group, a 8-quinolyloxy group, a 1-isoquinolyloxy group, a 3-isoquinolyloxy group, a 4-isoquinolyloxy group, a 5-isoquinolyloxy group, a 6-isoquinolyloxy group, a 7-isoquinolyloxy group, a 8-isoquinolyloxy group, a 1-phthalazinyloxy group, a 5-phthalazinyloxy group, a 6-phthalazinyloxy group, a 1-2,7-naphthyridinyloxy group, a 3-2,7-naphthyridinyloxy group, a 4-2,7-naphthyridinyloxy group, a 1-2,6-naphthyridinyloxy group, a 3-2,6-naphthyridinyloxy group, a 4-2,6-naphthyridinyloxy group, a 2-1,8-naphthyridinyloxy group, a 3-1,8-naphthyridinyloxy group, a 4-1,8-naphthyridinyloxy group, a 2-1,7-naphthyridinyloxy group, a 3-1,7-naphthyridinyloxy group, a 4-1,7-naphthyridinyloxy group, a 5-1,7-naphthyridinyloxy group, a 6-1,7-naphthyridinyloxy group, a 8-1,7-naphthyridinyloxy group, 2-1,6-naphthyridinyloxy group, a 3-1,6-naphthyridinyloxy group, a 4-1,6-naphthyridinyloxy group, a 5-1,6-naphthyridinyloxy group, a 7-1,6-naphthyridinyloxy group, a 8-1,6-naphthyridinyloxy group, a 2-1,5-naphthyridinyloxy group, a 3-1,5-naphthyridinyloxy group, a 4-1,5-naphthyridinyloxy group, a 6-1,5-naphthyridinyloxy group, a 7-1,5-naphthyridinyloxy group, a 8-1,5-naphthyridinyloxy group, a 2-quinoxalinyloxy group, a 5-quinoxalinyloxy group, a 6-quinoxalinyloxy group, a 2-quinazolinyloxy group, a 4-quinazolinyloxy group, a 5-quinazolinyloxy group, a 6-quinazolinyloxy group, a 7-quinazolinyloxy group, a 8-quinazolinyloxy group, a 3-cinnolinyloxy group, a 4-cinnolinyloxy group, a 5-cinnolinyloxy group, a 6-cinnolinyloxy group, a 7-cinnolinyloxy group, a 8-cinnolinyloxy group, a 2-pteridinyloxy group, a 4-pteridinyloxy group, a 6-pteridinyloxy group, a 7-pteridinyloxy group or the like.

A C₁₋₆ alkylcarbonyl group may linear, branched or a C₃₋₆ cycloalkylcarbonyl group, and as specific examples, methylcarbonyl, ethylcarbonyl, n-propylcarbonyl, i-propylcarbonyl, c-propylcarbonyl, n-butylcarbonyl, i-butylcarbonyl, s-butylcarbonyl, t-butylcarbonyl, c-butylcarbonyl, 1-methyl-c-propylcarbonyl, 2-methyl-c-propylcarbonyl, n-pentylcarbonyl, 1-methyl-n-butylcarbonyl, 2-methyl-n-butylcarbonyl, 3-methyl-n-butylcarbonyl, 1,1-dimethyl-n-propylcarbonyl, 1,2-dimethyl-n-propylcarbonyl, 2,2-dimethyl-n-propylcarbonyl, 1-ethyl-n-propylcarbonyl, c-pentylcarbonyl, 1-methyl-c-butylcarbonyl, 2-methyl-c-butylcarbonyl, 3-methyl-c-butylcarbonyl, 1,2-dimethyl-c-propylcarbonyl, 2,3-dimethyl-c-propylcarbonyl, 1-ethyl-c-propylcarbonyl, 2-ethyl-c-propylcarbonyl, n-hexylcarbonyl, 1-methyl-n-pentylcarbonyl, 2-methyl-n-pentylcarbonyl, 3-methyl-n-pentylcarbonyl, 4-methyl-n-pentylcarbonyl, 1,1-dimethyl-n-butylcarbonyl, 1,2-dimethyl-n-butylcarbonyl, 1,3-dimethyl-n-butylcarbonyl, 2,2-dimethyl-n-butylcarbonyl, 2,3-dimethyl-n-butylcarbonyl, 3,3-dimethyl-n-butylcarbonyl, 1-ethyl-n-butylcarbonyl, 2-ethyl-n-butylcarbonyl, 1,1,2-trimethyl-n-propylcarbonyl, 1,2,2-trimethyl-n-propylcarbonyl, 1-ethyl-1-methyl-n-propylcarbonyl, 1-ethyl-2-methyl-n-propylcarbonyl, c-hexylcarbonyl, 1-methyl-c-pentylcarbonyl, 2-methyl-c-pentylcarbonyl, 3-methyl-c-pentylcarbonyl, 1-ethyl-c-butylcarbonyl, 2-ethyl-c-butylcarbonyl, 3-ethyl-c-butylcarbonyl, 1,2-dimethyl-c-butylcarbonyl, 1,3-dimethyl-c-butylcarbonyl, 2,2-dimethyl-c-butylcarbonyl, 2,3-dimethyl-c-butylcarbonyl, 2,4-dimethyl-c-butylcarbonyl, 3,3-dimethyl-c-butylcarbonyl, 1-n-propyl-c-propylcarbonyl, 2-n-propyl-c-propylcarbonyl, 1-i-propyl-c-propylcarbonyl, 2-i-propyl-c-propylcarbonyl, 1,2,2-trimethyl-c-propylcarbonyl, 1,2,3-trimethyl-c-propylcarbonyl, 2,2,3-trimethyl-c-propylcarbonyl, 1-ethyl-2-methyl-c-propylcarbonyl, 2-ethyl-1-methyl-c-propylcarbonyl, 2-ethyl-2-methyl-c-propylcarbonyl, 2-ethyl-3-methyl-c-propylcarbonyl or the like may be mentioned.

A C₁₋₁₀ alkylcarbonyl group may linear, branched or a C₃₋₁₀ cycloalkylcarbonyl group, and as specific examples, in addition to those mentioned above, 1-methyl-1-ethyl-n-pentylcarbonyl, 1-heptylcarbonyl, 2-heptylcarbonyl, 1-ethyl-1,2-dimethyl-n-propylcarbonyl, 1-ethyl-2,2-dimethyl-n-propylcarbonyl, 1-octylcarbonyl, 3-octylcarbonyl, 4-methyl-3-n-heptylcarbonyl, 6-methyl-2-n-heptylcarbonyl, 2-propyl-1-n-heptylcarbonyl, 2,4,4-trimethyl-1-n-pentylcarbonyl, 1-nonylcarbonyl, 2-nonylcarbonyl, 2,6-dimethyl-4-n-heptylcarbonyl, 3-ethyl-2,2-dimethyl-3-n-pentylcarbonyl, 3,5,5-trimethyl-1-n-hexylcarbonyl, 1-decylcarbonyl, 2-decylcarbonyl, 4-decylcarbonyl, 3,7-dimethyl-1-n-octylcarbonyl, 3,7-dimethyl-3-n-octylcarbonyl or the like may be mentioned.

A C₁₋₁₀ thioalkyl group may linear, branched or a C₃₋₁₀ cyclothioalkyl group, and as specific examples, methylthio, ethylthio, n-propylthio, i-propylthio, c-propylthio, n-butylthio, i-butylthio, s-butylthio, t-butylthio, c-butylthio, 1-methyl-c-propylthio, 2-methyl-c-propylthio, n-pentylthio, 1-methyl-n-butylthio, 2-methyl-n-butylthio, 3-methyl-n-butylthio, 1,1-dimethyl-n-propylthio, 1,2-dimethyl-n-propylthio, 2,2-dimethyl-n-propylthio, 1-ethyl-n-propylthio, c-pentylthio, 1-methyl-c-butylthio, 2-methyl-c-butylthio, 3-methyl-c-butylthio, 1,2-dimethyl-c-propylthio, 2,3-dimethyl-c-propylthio, 1-ethyl-c-propylthio, 2-ethyl-c-propylthio, n-hexylthio, 1-methyl-n-pentylthio, 2-methyl-n-pentylthio, 3-methyl-n-pentylthio, 4-methyl-n-pentylthio, 1,1-dimethyl-n-butylthio, 1,2-dimethyl-n-butylthio, 1,3-dimethyl-n-butylthio, 2,2-dimethyl-n-butylthio, 2,3-dimethyl-n-butylthio, 3,3-dimethyl-n-butylthio, 1-ethyl-n-butylthio, 2-ethyl-n-butylthio, 1,1,2-trimethyl-n-propylthio, 1,2,2-trimethyl-n-propylthio, 1-ethyl-1-methyl-n-propylthio, 1-ethyl-2-methyl-n-propylthio, c-hexylthio, 1-methyl-c-pentylthio, 2-methyl-c-pentylthio, 3-methyl-c-pentylthio, 1-ethyl-c-butylthio, 2-ethyl-c-butylthio, 3-ethyl-c-butylthio, 1,2-dimethyl-c-butylthio, 1,3-dimethyl-c-butylthio, 2,2-dimethyl-c-butylthio, 2,3-dimethyl-c-butylthio, 2,4-dimethyl-c-butylthio, 3,3-dimethyl-c-butylthio, 1-n-propyl-c-propylthio, 2-n-propyl-c-propylthio, 1-i-propyl-c-propylthio, 2-i-propyl-c-propylthio, 1,2,2-trimethyl-c-propylthio, 1,2,3-trimethyl-c-propylthio, 2,2,3-trimethyl-c-propylthio, 1-ethyl-2-methyl-c-propylthio, 2-ethyl-1-methyl-c-propylthio, 2-ethyl-2-methyl-c-propylthio, 2-ethyl-3-methyl-c-propylthio, 1-methyl-1-ethyl-n-pentylthio, 1-heptylthio, 2-heptylthio, 1-ethyl-1,2-dimethyl-n-propylthio, 1-ethyl-2,2-dimethyl-n-propylthio, 1-octylthio, 3-octylthio, 4-methyl-3-n-heptylthio, 6-methyl-2-n-heptylthio, 2-propyl-1-n-heptylthio, 2,4,4-trimethyl-1-n-pentylthio, 1-nonylthio, 2-nonylthio, 2,6-dimethyl-4-n-heptylthio, 3-ethyl-2,2-dimethyl-3-n-pentylthio, 3,5,5-trimethyl-1-n-hexylthio, 1-decylthio, 2-decylthio, 4-decylthio, 3,7-dimethyl-1-n-octylthio, 3,7-dimethyl-3-n-octylthio or the like may be mentioned.

A C₁₋₆ alkylsulfonylamino group may be linear, branched or a C₃₋₆ cycloalkylsulfonylamino group, and as specific examples, methylsulfonylamino, ethylsulfonylamino, n-propylsulfonylamino, i-propylsulfonylamino, c-propylsulfonylamino, n-butylsulfonylamino, i-butylsulfonylamino, s-butylsulfonylamino, t-butylsulfonylamino, c-butylsulfonylamino, 1-methyl-c-propylsulfonylamino, 2-methyl-c-propylsulfonylamino, n-pentylsulfonylamino, 1-methyl-n-butylsulfonylamino, 2-methyl-n-butylsulfonylamino, 3-methyl-n-butylsulfonylamino, 1,1-dimethyl-n-propylsulfonylamino, 1,2-dimethyl-n-propylsulfonylamino, 2,2-dimethyl-n-propylsulfonylamino, 1-ethyl-n-propylsulfonylamino, c-pentylsulfonylamino, 1-methyl-c-butylsulfonylamino, 2-methyl-c-butylsulfonylamino, 3-methyl-c-butylsulfonylamino, 1,2-dimethyl-c-propylsulfonylamino, 2,3-dimethyl-c-propylsulfonylamino, 1-ethyl-c-propylsulfonylamino, 2-ethyl-c-propylsulfonylamino, n-hexylsulfonylamino, 1-methyl-n-pentylsulfonylamino, 2-methyl-n-pentylsulfonylamino, 3-methyl-n-pentylsulfonylamino, 4-methyl-n-pentylsulfonylamino, 1,1-dimethyl-n-butylsulfonylamino, 1,2-dimethyl-n-butylsulfonylamino, 1,3-dimethyl-n-butylsulfonylamino, 2,2-dimethyl-n-butylsulfonylamino, 2,3-dimethyl-n-butylsulfonylamino, 3,3-dimethyl-n-butylsulfonylamino, 1-ethyl-n-butylsulfonylamino, 2-ethyl-n-butylsulfonylamino, 1,1,2-trimethyl-n-propylsulfonylamino, 1,2,2-trimethyl-n-propylsulfonylamino, 1-ethyl-1-methyl-n-propylsulfonylamino, 1-ethyl-2-methyl-n-propylsulfonylamino, c-hexylsulfonylamino, 1-methyl-c-pentylsulfonylamino, 2-methyl-c-pentylsulfonylamino, 3-methyl-c-pentylsulfonylamino, 1-ethyl-c-butylsulfonylamino, 2-ethyl-c-butylsulfonylamino, 3-ethyl-c-butylsulfonylamino, 1,2-dimethyl-c-butylsulfonylamino, 1,3-dimethyl-c-butylsulfonylamino, 2,2-dimethyl-c-butylsulfonylamino, 2,3-dimethyl-c-butylsulfonylamino, 2,4-dimethyl-c-butylsulfonylamino, 3,3-dimethyl-c-butylsulfonylamino, 1-n-propyl-c-propylsulfonylamino, 2-n-propyl-c-propylsulfonylamino, 1-i-propyl-c-propylsulfonylamino, 2-i-propyl-c-propylsulfonylamino, 1,2,2-trimethyl-c-propylsulfonylamino, 1,2,3-trimethyl-c-propylsulfonylamino, 2,2,3-trimethyl-c-propylsulfonylamino, 1-ethyl-2-methyl-c-propylsulfonylamino, 2-ethyl-1-methyl-c-propylsulfonylamino, 2-ethyl-2-methyl-c-propylsulfonylamino, 2-ethyl-3-methyl-c-propylsulfonylamino or the like may be mentioned.

A C₁₋₁₀ alkylsulfonylamino group may be linear, branched or a C₃₋₁₀ cycloalkylsulfonylamino group, and as specific examples, in addition to those mentioned above, 1-methyl-1-ethyl-n-pentylsulfonylamino, 1-heptylsulfonylamino, 2-heptylsulfonylamino, 1-ethyl-1,2-dimethyl-n-propylsulfonylamino, 1-ethyl-2,2-dimethyl-n-propylsulfonylamino, 1-octylsulfonylamino, 3-octylsulfonylamino, 4-methyl-3-n-heptylsulfonylamino, 6-methyl-2-n-heptylsulfonylamino, 2-propyl-1-n-n-heptylsulfonylamino, 2,4,4-trimethyl-1-n-pentylsulfonylamino, 1-nonylsulfonylamino, 2-nonylsulfonylamino, 2,6-dimethyl-4-n-heptylsulfonylamino, 3-ethyl-2,2-dimethyl-3-n-pentylsulfonylamino, 3,5,5-trimethyl-1-n-hexylsulfonylamino, 1-decylsulfonylamino, 2-decylsulfonylamino, 4-decylsulfonylamino, 3,7-dimethyl-1-n-octylsulfonylamino, 3,7-dimethyl-3-n-octylsulfonylamino, c-heptylsulfonylamino, c-octylsulfonylamino, 1-methyl-c-hexylsulfonylamino, 2-methyl-c-hexylsulfonylamino, 3-methyl-c-hexylsulfonylamino, 1,2-dimethyl-c-hexylsulfonylamino, 1-ethyl-c-hexylsulfonylamino, 1-methyl-c-pentylsulfonylamino, 2-methyl-c-pentylsulfonylamino, 3-methyl-c-pentylsulfonylamino or the like may be mentioned.

A C₁₋₆ alkoxy group may be linear, branched or a C₃₋₆ cycloalkoxy group, and as specific examples, methoxy, ethoxy, n-propoxy, i-propoxy, c-propoxy, n-butoxy, i-butoxy, s-butoxy, t-butoxy, c-butoxy, 1-methyl-c-propoxy, 2-methyl-c-propoxy, n-pentyloxy, 1-methyl-n-butoxy, 2-methyl-n-butoxy, 3-methyl-n-butoxy, 1,1-dimethyl-n-propoxy, 1,2-dimethyl-n-propoxy, 2,2-dimethyl-n-propoxy, 1-ethyl-n-propoxy, c-pentyloxy, 1-methyl-c-butoxy, 2-methyl-c-butoxy, 3-methyl-c-butoxy, 1,2-dimethyl-c-propoxy, 2,3-dimethyl-c-propoxy, 1-ethyl-c-propoxy, 2-ethyl-c-propoxy, n-hexyloxy, 1-methyl-n-pentyloxy, 2-methyl-n-pentyloxy, 3-methyl-n-pentyloxy, 4-methyl-n-pentyloxy, 1,1-dimethyl-n-butoxy, 1,2-dimethyl-n-butoxy, 1,3-dimethyl-n-butoxy, 2,2-dimethyl-n-butoxy, 2,3-dimethyl-n-butoxy, 3,3-dimethyl-n-butoxy, 1-ethyl-n-butoxy, 2-ethyl-n-butoxy, 1,1,2-trimethyl-n-propoxy, 1,2,2-trimethyl-n-propoxy, 1-ethyl-1-methyl-n-propoxy, 1-ethyl-2-methyl-n-propoxy, c-hexyloxy, 1-methyl-c-pentyloxy, 2-methyl-c-pentyloxy, 3-methyl-c-pentyloxy, 1-ethyl-c-butoxy, 2-ethyl-c-butoxy, 3-ethyl-c-butoxy, 1,2-dimethyl-c-butoxy, 1,3-dimethyl-c-butoxy, 2,2-dimethyl-c-butoxy, 2,3-dimethyl-c-butoxy, 2,4-dimethyl-c-butoxy, 3,3-dimethyl-c-butoxy, 1-n-propyl-c-propoxy, 2-n-propyl-c-propoxy, 1-i-propyl-c-propoxy, 2-i-propyl-c-propoxy, 1,2,2-trimethyl-c-propoxy, 1,2,3-trimethyl-c-propoxy, 2,2,3-trimethyl-c-propoxy, 1-ethyl-2-methyl-c-propoxy, 2-ethyl-1-methyl-c-propoxy, 2-ethyl-2-methyl-c-propoxy, 2-ethyl-3-methyl-c-propoxy or the like may be mentioned.

A C₁₋₁₀ alkoxy group may be linear, branched or a C₃₋₁₀ cycloalkoxy group, and as specific examples, methoxy, ethoxy, n-propoxy, i-propoxy, c-propoxy, n-butoxy, i-butoxy, s-butoxy, t-butoxy, c-butoxy, 1-methyl-c-propoxy, 2-methyl-c-propoxy, n-pentyloxy, 1-methyl-n-butoxy, 2-methyl-n-butoxy, 3-methyl-n-butoxy, 1,1-dimethyl-n-propoxy, 1,2-dimethyl-n-propoxy, 2,2-dimethyl-n-propoxy, 1-ethyl-n-propoxy, c-pentyloxy, 1-methyl-c-butoxy, 2-methyl-c-butoxy, 3-methyl-c-butoxy, 1,2-dimethyl-c-propoxy, 2,3-dimethyl-c-propoxy, 1-ethyl-c-propoxy, 2-ethyl-c-propoxy, n-hexyloxy, 1-methyl-n-pentyloxy, 2-methyl-n-pentyloxy, 3-methyl-n-pentyloxy, 4-methyl-n-pentyloxy, 1,1-dimethyl-n-butoxy, 1,2-dimethyl-n-butoxy, 1,3-dimethyl-n-butoxy, 2,2-dimethyl-n-butoxy, 2,3-dimethyl-n-butoxy, 3,3-dimethyl-n-butoxy, 1-ethyl-n-butoxy, 2-ethyl-n-butoxy, 1,1,2-trimethyl-n-propoxy, 1,2,2-trimethyl-n-propoxy, 1-ethyl-1-methyl-n-propoxy, 1-ethyl-2-methyl-n-propoxy, c-hexyloxy, 1-methyl-c-pentyloxy, 2-methyl-c-pentyloxy, 3-methyl-c-pentyloxy, 1-ethyl-c-butoxy, 2-ethyl-c-butoxy, 3-ethyl-c-butoxy, 1,2-dimethyl-c-butoxy, 1,3-dimethyl-c-butoxy, 2,2-dimethyl-c-butoxy, 2,3-dimethyl-c-butoxy, 2,4-dimethyl-c-butoxy, 3,3-dimethyl-c-butoxy, 1-n-propyl-c-propoxy, 2-n-propyl-c-propoxy, 1-i-propyl-c-propoxy, 2-i-propyl-c-propoxy, 1,2,2-trimethyl-c-propoxy, 1,2,3-trimethyl-c-propoxy, 2,2,3-trimethyl-c-propoxy, 1-ethyl-2-methyl-c-propoxy, 2-ethyl-1-methyl-c-propoxy, 2-ethyl-2-methyl-c-propoxy, 2-ethyl-3-methyl-c-propoxy, 1-methyl-1-ethyl-n-pentyloxy, 1-heptyloxy, 2-heptyloxy, 1-ethyl-1,2-dimethyl-n-propyloxy, 1-ethyl-2,2-dimethyl-n-propyloxy, 1-octyloxy, 3-octyloxy, 4-methyl-3-n-heptyloxy, 6-methyl-2-n-heptyloxy, 2-propyl-1-n-heptyloxy, 2,4,4-trimethyl-1-n-pentyloxy, 1-nonyloxy, 2-nonyloxy, 2,6-dimethyl-4-n-heptyloxy, 3-ethyl-2,2-dimethyl-3-n-pentyloxy, 3,5,5-trimethyl-1-n-hexyloxy, 1-decyloxy, 2-decyloxy, 4-decyloxy, 3,7-dimethyl-1-n-octyloxy, 3,7-dimethyl-3-n-octyloxy or the like may be mentioned.

A C₁₋₆ alkylthiocarbonyl group is a C₁₋₆ alkylcarbonyl group having a sulfur atom instead of an oxygen atom and may be linear, branched or a C₃₋₆ cycloalkylthiocarbonyl group, and as specific examples, methyl(thiocarbonyl), ethyl(thiocarbonyl), n-propyl(thiocarbonyl), i-propyl(thiocarbonyl), c-propyl(thiocarbonyl), n-butyl(thiocarbonyl), i-butyl(thiocarbonyl), s-butyl(thiocarbonyl), t-butyl(thiocarbonyl), c-butyl(thiocarbonyl), 1-methyl-c-propyl(thiocarbonyl), 2-methyl-c-propyl(thiocarbonyl), n-pentyl(thiocarbonyl), 1-methyl-n-butyl(thiocarbonyl), 2-methyl-n-butyl(thiocarbonyl), 3-methyl-n-butyt(thiocarbonyl), 1,1-dimethyl-n-propyl(thiocarbonyl), 1,2-dimethyl-n-propyl(thiocarbonyl), 2,2-dimethyl-n-propyl(thiocarbonyl), 1-ethyl-n-propyl(thiocarbonyl), c-pentyl(thiocarbonyl), 1-methyl-c-butyl(thiocarbonyl), 2-methyl-c-butyl(thiocarbonyl), 3-methyl-c-butyl(thiocarbonyl), 1,2-dimethyl-c-propyl(thiocarbonyl), 2,3-dimethyl-c-propyl(thiocarbonyl), 1-ethyl-c-propyl(thiocarbonyl), 2-ethyl-c-propyl(thiocarbonyl), n-hexyl(thiocarbonyl), 1-methyl-n-pentyl(thiocarbonyl), 2-methyl-n-pentyl(thiocarbonyl), 3-methyl-n-pentyl(thiocarbonyl), 4-methyl-n-pentyl(thiocarbonyl), 1,1-dimethyl-n-butyl(thiocarbonyl), 1,2-dimethyl-n-butyl(thiocarbonyl), 1,3-dimethyl-n-butyl(thiocarbonyl), 2,2-dimethyl-n-butyl(thiocarbonyl), 2,3-dimethyl-n-butyl(thiocarbonyl), 3,3-dimethyl-n-butyl(thiocarbonyl), 1-ethyl-n-butyl(thiocarbonyl), 2-ethyl-n-butyl(thiocarbonyl), 1,1,2-trimethyl-n-propyl(thiocarbonyl), 1,2,2-trimethyl-n-propyl(thiocarbonyl), 1-ethyl-1-methyl-n-propyl(thiocarbonyl), 1-ethyl-2-methyl-n-propyl(thiocarbonyl), c-hexyl(thiocarbonyl), 1-methyl-c-pentyl(thiocarbonyl), 2-methyl-c-pentyl(thiocarbonyl), 3-methyl-c-pentyl(thiocarbonyl), 1-ethyl-c-butyl(thiocarbonyl), 2-ethyl-c-butyl(thiocarbonyl), 3-ethyl-c-butyl(thiocarbonyl), 1,2-dimethyl-c-butyl(thiocarbonyl), 1,3-dimethyl-c-butyl(thiocarbonyl), 2,2-dimethyl-c-butyl(thiocarbonyl), 2,3-dimethyl-c-butyl(thiocarbonyl), 2,4-dimethyl-c-butyl(thiocarbonyl), 3,3-dimethyl-c-butyl(thiocarbonyl), 1-n-propyl-c-propyl(thiocarbonyl), 2-n-propyl-c-propyl(thiocarbonyl), 1-i-propyl-c-propyl(thiocarbonyl), 2-i-propyl-c-propyl(thiocarbonyl), 1,2,2-trimethyl-c-propyl(thiocarbonyl), 1,2,3-trimethyl-c-propyl(thiocarbonyl), 2,2,3-trimethyl-c-propyl(thiocarbonyl), 1-ethyl-2-methyl-c-propyl(thiocarbonyl), 2-ethyl-1-methyl-c-propyl(thiocarbonyl), 2-ethyl-2-methyl-c-propyl(thiocarbonyl), 2-ethyl-3-methyl-c-propyl(thiocarbonyl) or the like may be mentioned.

A C₁₋₁₀ alkylthiocarbonyl group may be linear, branched or a C₃₋₁₀ cycloalkylthiocarbonyl group, and as specific examples, in addition to those mentioned above, 1-methyl-1-ethyl-n-pentyl(thiocarbonyl), 1-heptyl(thiocarbonyl), 2-heptyl(thiocarbonyl), 1-ethyl-1,2-dimethyl-n-propyl(thiocarbonyl), 1-ethyl-2,2-dimethyl-n-propyl(thiocarbonyl), 1-octyl(thiocarbonyl), 3-octyl(thiocarbonyl), 4-methyl-3-n-heptyl(thiocarbonyl), 6-methyl-2-n-heptyl(thiocarbonyl), 2-propyl-1-n-heptyl(thiocarbonyl), 2,4,4-trimethyl-1-n-pentyl(thiocarbonyl), 1-nonyl(thiocarbonyl), 2-nonyl(thiocarbonyl), 2,6-dimethyl-4-n-heptyl(thiocarbonyl), 3-ethyl-2,2-dimethyl-3-n-pentyl(thiocarbonyl), 3,5,5-trimethyl-1-n-hexyl(thiocarbonyl), 1-decyl(thiocarbonyl), 2-decyl(thiocarbonyl), 4-decyl(thiocarbonyl), 3,7-dimethyl-1-n-octyl(thiocarbonyl), 3,7-dimethyl-3-n-octyl(thiocarbonyl) or the like may be mentioned.

A C₁₋₆ alkylsulfonyl group may be linear, branched or a C₃₋₆ cycloalkylsulfonyl group, and as specific examples, methylsulfonyl, ethylsulfonyl, n-propylsulfonyl, i-propylsulfonyl, c-propylsulfonyl, n-butylsulfonyl, i-butylsulfonyl, s-butylsulfonyl, t-butylsulfonyl, c-butylsulfonyl, 1-methyl-c-propylsulfonyl, 2-methyl-c-propylsulfonyl, n-pentylsulfonyl, 1-methyl-n-butylsulfonyl, 2-methyl-n-butylsulfonyl, 3-methyl-n-butylsulfonyl, 1,1-dimethyl-n-propylsulfonyl, 1,2-dimethyl-n-propylsulfonyl, 2,2-dimethyl-n-propylsulfonyl, 1-ethyl-n-propylsulfonyl, c-pentylsulfonyl, 1-methyl-c-butylsulfonyl, 2-methyl-c-butylsulfonyl, 3-methyl-c-butylsulfonyl, 1,2-dimethyl-c-propylsulfonyl, 2,3-dimethyl-c-propylsulfonyl, 1-ethyl-c-propylsulfonyl, 2-ethyl-c-propylsulfonyl,n-hexylsulfonyl, 1-methyl-n-pentylsulfonyl, 2-methyl-n-pentylsulfonyl, 3-methyl-n-pentylsulfonyl, 4-methyl-n-pentylsulfonyl, 1,1-dimethyl-n-butylsulfonyl, 1,2-dimethyl-n-butylsulfonyl, 1,3-dimethyl-n-butylsulfonyl, 2,2-dimethyl-n-butylsulfonyl, 2,3-dimethyl-n-butylsulfonyl, 3,3-dimethyl-n-butylsulfonyl, 1-ethyl-n-butylsulfonyl, 2-ethyl-n-butylsulfonyl, 1,1,2-trimethyl-n-propylsulfonyl, 1,2,2-trimethyl-n-propylsulfonyl, 1-ethyl-1-methyl-n-propylsulfonyl, 1-ethyl-2-methyl-n-propylsulfonyl, c-hexylsulfonyl, 1-methyl-c-pentylsulfonyl, 2-methyl-c-pentylsulfonyl, 3-methyl-c-pentylsulfonyl, 1-ethyl-c-butylsulfonyl, 2-ethyl-c-butylsulfonyl, 3-ethyl-c-butylsulfonyl, 1,2-dimethyl-c-butylsulfonyl, 1,3-dimethyl-c-butylsulfonyl, 2,2-dimethyl-c-butylsulfonyl, 2,3-dimethyl-c-butylsulfonyl, 2,4-dimethyl-c-butylsulfonyl, 3,3-dimethyl-c-butylsulfonyl, 1-n-propyl-c-propylsulfonyl, 2-n-propyl-c-propylsulfonyl, 1-i-propyl-c-propylsulfonyl, 2-i-propyl-c-propylsulfonyl, 1,2,2-trimethyl-c-propylsulfonyl, 1,2,3-trimethyl-c-propylsulfonyl, 2,2,3-trimethyl-c-propylsulfonyl, 1-ethyl-2-methyl-c-propylsulfonyl, 2-ethyl-1-methyl-c-propylsulfonyl, 2-ethyl-2-methyl-c-propylsulfonyl, 2-ethyl-3-methyl-c-propylsulfonyl or the like may be mentioned.

A C₁₋₁₀ alkylsulfonyl group may be linear, branched or a C₃₋₁₀ cycloalkylsulfonyl group, and as specific examples, in addition to those mentioned above,1-methyl-1-ethyl-n-pentylsulfonyl, 1-heptylsulfonyl, 2-heptylsulfonyl, 1-ethyl-1,2-dimethyl-n-propylsulfonyl, 1-ethyl-2,2-dimethyl-n-propylsulfonyl, 1-octylsulfonyl, 3-octylsulfonyl, 4-methyl-3-n-heptylsulfonyl, 6-methyl-2-n-heptylsulfonyl, 2-propyl-1-n-n-heptylsulfonyl, 2,4,4-trimethyl-1-n-pentylsulfonyl, 1-nonylsulfonyl, 2-nonylsulfonyl, 2,6-dimetyl-4-n-heptylsulfonyl, 3-ethyl-2,2-dimethyl-3-n-pentylsulfonyl, 3,5,5-trimethyl-1-n-hexylsulfonyl, 1-decylsulfonyl, 2-decylsulfonyl, 4-decylsulfonyl, 3,7-dimethyl-1-n-octylsulfonyl, 3,7-dimethyl-3-n-octylsulfonyl, 3,7-dimethyl-3-n-octylsulfonyl, c-heptylsulfonyl, c-octylsulfonyl, 1-methyl-c-hexylsulfonyl, 2-methyl-c-hexylsulfonyl, 3-methyl-c-hexylsulfonyl, 1,2-dimethyl-c-hexylsulfonyl, 1-ethyl-c-hexylsuffonyl, 1-methyl-c-pentylsulfonyl, 2-methyl-c-pentylsulfonyl, 3-methyl-c-pentylsulfonyl or the like may be mentioned.

A C₁₋₆ alkoxycarbonyl group may be linear, branched or a C₃₋₆ cycloalkoxycarbonyl group, and as specific examples, methoxycarbonyl, ethoxycarbonyl, n-propoxycarbonyl, i-propoxycarbonyl, c-propoxycarbonyl, n-butoxycarbonyl, i-butoxycarbonyl, s-butoxycarbonyl, t-butoxycarbonyl, c-butoxycarbonyl, 1-methyl-c-propoxycarbonyl, 2-methyl-c-propoxycarbonyl, n-pentyloxycarbonyl, 1-methyl-n-butoxycarbonyl, 2-methyl-n-butoxycarbonyl, 3-methyl-n-butoxycarbonyl, 1,1-dimethyl-n-propoxycarbonyl, 1,2-dimethyl-n-propoxycarbonyl, 2,2-dimethyl-n-propoxycarbonyl, 1-ethyl-n-propoxycarbonyl, c-pentyloxycarbonyl, 1-methyl-c-butoxycarbonyl, 2-methyl-c-butoxycarbonyl, 3-methyl-c-butoxycarbonyl, 1,2-dimethyl-c-propoxycarbonyl, 2,3-dimethyl-c-propoxycarbonyl, 1-ethyl-c-propoxycarbonyl, 2-ethyl-c-propoxycarbonyl, n-hexyloxycarbonyl, 1-methyl-n-pentyloxycarbonyl, 2-methyl-n-pentyloxycarbonyl, 3-methyl-n-pentyloxycarbonyl, 4-methyl-n-pentyloxycarbonyl, 1,1-dimethyl-n-butoxycarbonyl, 1,2-dimethyl-n-butoxycarbonyl, 1 ,3-dimethyl-n-butoxycarbonyl, 2,2-dimethyl-n-butoxycarbonyl, 2,3-dimethyl-n-butoxycarbonyl, 3,3-dimethyl-n-butoxycarbonyl, 1-ethyl-n-butoxycarbonyl, 2-ethyl-n-butoxycarbonyl, 1,1,2-trimethyl-n-propoxycarbonyl, 1,2,2-trimethyl-n-propoxycarbonyl, 1-ethyl-1-methyl-n-propoxycarbonyl, 1-ethyl-2-methyl-n-propoxycarbonyl, c-hexyloxycarbonyl, 1-methyl-c-pentyloxycarbonyl, 2-methyl-c-pentyloxycarbonyl, 3-methyl-c-pentyloxycarbonyl, 1-ethyl-c-butoxycarbonyl, 2-ethyl-c-butoxycarbonyl, 3-ethyl-c-butoxycarbonyl, 1,2-dimethyl-c-butoxycarbonyl, 1,3-dimethyl-c-butoxycarbonyl, 2,2-dimethyl-c-butoxycarbonyl, 2,3-dimethyl-c-butoxycarbonyl, 2,4-dimethyl-c-butoxycarbonyl, 3,3-dimethyl-c-butoxycarbonyl, 1-n-propyl-c-propoxycarbonyl, 2-n-propyl-c-propoxycarbonyl, 1-i-propyl-c-propoxycarbonyl, 2-i-propyl-c-propoxycarbonyl, 1,2,2-trimethyl-c-propoxycarbonyl, 1,2,3-trimethyl-c-propoxycarbonyl, 2,2,3-trimethyl-c-propoxycarbonyl, 1-ethyl-2-methyl-c-propoxycarbonyl, 2-ethyl-1-methyl-c-propoxycarbonyl, 2-ethyl-2-methyl-c-propoxycarbonyl, 2-ethyl-3-methyl-c-propoxycarbonyl or the like may be mentioned.

A C₁₋₁₀ alkoxycarbonyl group may be linear, branched or a C₃₋₁₀ cycloalkoxycarbonyl group, and as specific examples, in addition to those mentioned above, 1-methyl-1-ethyl-n-pentyloxycarbonyl, 1-heptyloxycarbonyl, 2-heptyloxycarbonyt, 1-ethyl-1,2-dimethyl-n-propyloxycarbonyl, 1-ethyl-2,2-dimethyl-n-propyloxycarbonyl, 1-octyloxycarbonyl, 3-octyloxycarbonyl, 4-methyl-3-n-heptyloxycarbonyl, 6-methyl-2-n-heptyloxycarbonyl, 2-propyl-1-n-heptyloxycarbonyl, 2,4,4-trimethyl-1-n-pentyloxycarbonyl, 1-nonyloxycarbonyl, 2-nonyloxycarbonyl, 2,6-dimethyl-4-n-heptyloxycarbonyl, 3-ethyl-2,2-dimethyl-3-n-pentyloxycarbonyl, 3,5,5-trimethyl-1-n-hexyloxycarbonyl, 1-decyloxycarbonyl, 2-decyloxycarbonyl, 4-decyloxycarbonyl, 3,7-dimethyl-1-n-octyloxycarbonyl, 3,7-dimethyl-3-n-octyloxycarbonyl or the like may be mentioned.

A C₁₋₁₀ alkylcarbonyloxy group may be linear, branched or a C₃₋₁₀ cycloalkylcarbonyloxy group, and as specific examples, methylcarbonyloxy, ethylcarbonyloxy, n-propylcarbonyloxy, i-propylcarbonyloxy, c-propylcarbonyloxy, n-butylcarbonyloxy, i-butylcarbonyloxy, s-butylcarbonyloxy, t-butylcarbonyloxy, c-butylcarbonyloxy, 1-methyl-c-propylcarbonyloxy, 2-methyl-c-propylcarbonyloxy, n-pentylcarbonyloxy, 1-methyl-n-butylcarbonyloxy, 2-methyl-n-butylcarbonyloxy, 3-methyl-n-butylcarbonyloxy, 1,1-dimethyl-n-propylcarbonyloxy, 1,2-dimethyl-n-propylcarbonyloxy, 2,2-dimethyl-n-propylcarbonyloxy, 1-ethyl-n-propylcarbonyloxy, c-pentylcarbonyloxy, 1-methyl-c-butylcarbonyloxy, 2-methyl-c-butylcarbonyloxy, 3-methyl-c-butylcarbonyloxy, 1,2-dimethyl-c-propylcarbonyloxy, 2,3-dimethyl-c-propylcarbonyloxy, 1-ethyl-c-propylcarbonyloxy, 2-ethyl-c-propylcarbonyloxy, n-hexylcarbonyloxy, 1-methyl-n-pentylcarbonyloxy, 2-methyl-n-pentylcarbonyloxy, 3-methyl-n-pentylcarbonyloxy, 4-methyl-n-pentylcarbonyloxy, 1,1-dimethyl-n-butylcarbonyloxy, 1,2-dimethyl-n-butylcarbonyloxy, 1,3-dimethyl-n-butylcarbonyloxy, 2,2-dimethyl-n-butylcarbonyloxy, 2,3-dimethyl-n-butylcarbonyloxy, 3,3-dimethyl-n-butylcarbonyloxy, 1-ethyt-n-butylcarbonyloxy, 2-ethyl-n-butylcarbonyloxy, 1,1,2-trimethyl-n-propylcarbonyloxy, 1,2,2-trimethyl-n-propylcarbonyloxy, 1-ethyl-1-methyl-n-propylcarbonyloxy, 1-ethyl-2-methyl-n-propylcarbonyloxy, c-hexylcarbonyloxy, 1-methyl-c-pentylcarbonyloxy, 2-methyl-c-pentylcarbonyloxy, 3-methyl-c-pentylcarbonyloxy, 1-ethyl-c-butylcarbonyloxy, 2-ethyl-c-butylcarbonyloxy, 3-ethyl-c-butylcarbonyloxy, 1,2-dimethyl-c-butylcarbonyloxy, 1,3-dimethyl-c-butylcarbonyloxy, 2,2-dimethyl-c-butylcarbonylxoy, 2,3-dimethyl-c-butylcarbonyloxy, 2,4-dimethyl-c-butylcarbonyloxy, 3,3-dimethyl-c-butylcarbonyloxy, 1-n-propyl-c-propylcarbonyloxy, 2-n-propyl-c-propylcarbonyloxy, 1-i-propyl-c-propycarbonyloxy, 2-i-propyl-c-propylcarbonyloxy, 1,2,2-trimethyl-c-propylcarbonyloxy, 1,2,3-trimethyl-c-propylcarbonyloxy, 2,2,3-trimethyl-c-propylcarbonyloxy, 1-ethyl-2-methyl-c-propylcarbonyloxy, 2-ethyl-1-methyl-c-propylcarbonyloxy, 2-ethyl-2-methyl-c-propylcarbonyloxy, 2-ethyl-3-methyl-c-propylcarbonyloxy, 1-methyl-1-ethyl-n-pentylcarbonyloxy, 1-heptylcarbonyloxy, 2-heptylcarbonyloxy, 1-ethy)-1,2-dimethyt-n-propylcarbonyloxy, 1-ethyl-2,2-dimethyl-n-propylcarbonyloxy, 1-octylcarbonyloxy, 3-octylcarbonyloxy, 4-methyl-3-n-heptylcarbonyloxy, 6-methyl-2-n-heptylcarbonyloxy, 2-propyl-1-n-heptylcarbonyloxy, 2,4,4-trimethyl-1-n-pentylcarbonyloxy, 1-nonylcarbonyloxy, 2-nonylcarbonyloxy, 2,6-dimethyl-4-n-heptylcarbonyloxy, 3-ethyl-2,2-dimethyl-3-n-pentylcarbonyloxy, 3,5,5-trimethyl-1-n-hexylcarbonyloxy, 1-decylcarbonyloxy, 2-decylcarbonyloxy, 4-decylcarbonyloxy, 3,7-dimethyl-1-n-octylcarbonyloxy, 3,7-dimethyl-3-n-octylcarbonyloxy or the like may be mentioned.

A C₁₋₁₀ alkylcarbonylamino group may be linear, branched or a C₃₋₁₀ cycloalkylcarbonylamino group, and as specific examples, methylcarbonylamino, ethylcarbonylamino, n-propylcarbonylamino, i-propylcarbonylamino, c-propylcarbonylamino, n-butylcarbonylamino, i-butylcarbonylamino, s-butylcarbonylamino, t-butylcarbonylamino, c-butylcarbonylamino, 1-methyl-c-propylcarbonylamino, 2-methyl-c-propylcarbonylamino, n-pentylcarbonylamino, 1-methyl-n-butylcarbonylamino, 2-methyl-n-butylcarbonylamino, 3-methyl-n-butylcarbonylamino, 1,1-dimethyl-n-propylcarbonylamino, 1,2-dimethyl-n-propylcarbonylamino, 2,2-dimethyl-n-propylcarbonylamino, 1-ethyl-n-propylcarbonylamino, c-pentylcarbonylamino, 1-methyl-c-butylcarbonylamino, 2-methyl-c-butylcarbonylamino, 3-methyl-c-butylcarbonylamino, 1,2-dimethyl-c-propylcarbonylamino, 2,3-dimethyl-c-propylcarbonylamino, 1-ethyl-c-propylcarbonylamino, 2-ethyl-c-propylcarbonylamino, n-hexylcarbonylamino, 1-methyl-n-pentylcarbonylamino, 2-methyl-n-pentylcarbonylamino, 3-methyl-n-pentylcarbonylamino, 4-methyl-n-pentylcarbonylamino, 1,1-dimethyl-n-butylcarbonylamino, 1,2-dimethyl-n-butylcarbonylamino, 1,3-dimethyl-n-butylcarbonylamino, 2,2-dimethyl-n-butylcarbonylamino, 2,3-dimethyl-n-butylcarbonylamino, 3,3-dimethyl-n-butylcarbonylamino, 1-ethyl-n-butylcarbonylamino, 2-ethyl-n-butylcarbonylamino, 1,1,2-trimethyl-n-propylcarbonylamino, 1,2,2-trimethyl-n-propylcarbonylamino, 1-ethyl-1-methyl-n-propylcarbonylamino, 1-ethyl-2-methyl-n-propylcarbonylamino, c-hexylcarbonylamino, 1-methyl-c-pentylcarbonylamino, 2-methyl-c-pentylcarbonylamino, 3-methyl-c-pentylcarbonylamino, 1-ethyl-c-butylcarbonylamino, 2-ethyl-c-butylcarbonylamino, 3-ethyl-c-butylcarbonylamino, 1,2-dimethyl-c-butylcarbonylamino, 1,3-dimethyl-c-butylcarbonylamino, 2,2-dimethyl-c-butylcarbonylamino, 2,3-dimethyl-c-butylcarbonylamino, 2,4-dimethyl-c-butylcarbonylamino, 3,3-dimethyl-c-butylcarbonylamino, 1-n-propyl-c-propylcarbonylamino, 2-n-propyl-c-propylcarbonylamino, 1-i-propyl-c-propylcarbonylamino, 2-i-propyl-c-propylcarbonylamino, 1,2,2-trimethyl-c-propylcarbonylamino, 1,2,3-trimethyl-c-propylcarbonylamino, 2,2,3-trimethyl-c-propylcarbonylamino, 1-ethyl-2-methyl-c-propylcarbonylamino, 2-ethyl-1-methyl-c-propylcarbonylamino, 2-ethyl-2-methyl-c-propylcarbonylamino, 2-ethyl-3-methyl-c-propylcarbonylamino, 1-methyl-1-ethyl-n-pentylcarbonylamino, 1-heptylcarbonylamino, 2-heptylcarbonylamino, 1-ethyl-1,2-dimethyl-n-propylcarbonylamino, 1-ethyl-2,2-dimethyl-n-propylcarbonylamino, 1-octylcarbonylamino, 3-octylcarbonylamino, 4-methyl-3-n-heptylcarbonylamino, 6-methyl-2-n-heptylcarbonylamino, 2-propyl-1-n-heptylcarbonylamino, 2,4,4-trimethyl-1-n-pentylcarbonylamino, 1-nonylcarbonylamino, 2-nonylcarbonylamino, 2,6-dimethyl-4-n-heptylcarbonylamino, 3-ethyl-2,2-dimethyl-3-n-pentylcarbonylamino, 3,5,5-trimethyl-1-n-hexylcarbonylamino, 1-decylcarbonylamino, 2-decylcarbonylamino, 4-decylcarbonylamino, 3,7-dimethyl-1-n-octylcarbonylamino, 3,7-dimethyl-3-n-octylcarbonylamino or the like may be mentioned.

A C₁₋₁₀ monoalkylamino group may be linear, branched or a C₃₋₁₀ cycloalkylamino group, and specific examples, methylamino, ethylamino, n-propylamino, i-propylamino, c-propylamino, n-butylamino, i-butylamino, s-butylamino, t-butylamino, c-butylamino, 1-methyl-c-propylamino, 2-methyl-c-propylamino, n-pentylamino, 1-methyl-n-butylamino, 2-methyl-n-butylamino, 3-methyl-n-butylamino, 1,1-dimethyl-n-propylamino, 1,2-dimethyl-n-propylamino, 2,2-dimethyl-n-propylamino, 1-ethyl-n-propylamino, c-pentylamino, 1-methyl-c-butylamino, 2-methyl-c-butylamino, 3-methyl-c-butylamino, 1,2-dimethyl-c-propylamino, 2,3-dimethyl-c-propylamino, 1-ethyl-c-propylamino, 2-ethyl-c-propylamino, n-hexylamino, 1-methyl-n-pentylamino, 2-methyl-n-pentylamino, 3-methyl-n-pentylamino, 4-methyl-n-pentylamino, 1,1-dimethyl-n-butylamino, 1,2-dimethyl-n-butylamino, 1,3-dimethyl-n-butylamino, 2,2-dimethyl-n-butylamino, 2,3-dimethyl-n-butylamino, 3,3-dimethyl-n-butylamino, 1-ethyl-n-butylamino, 2-ethyl-n-butylamino, 1,1,2-trimethyl-n-propylamino, 1,2,2-trimethyl-n-propylamino, 1-ethyl-1-methyl-n-propylamino, 1-ethyl-2-methyl-n-propylamino, c-hexylamino, 1-methyl-c-pentylamino, 2-methyl-c-pentylamino, 3-methyl-c-pentylamino, 1-ethyl-c-butylamino, 2-ethyl-c-butylamino, 3-ethyl-c-butylamino, 1,2-dimethyl-c-butylamino, 1,3-dimethyl-c-butylamino, 2,2-dimethyl-c-butylamino, 2,3-dimethyl-c-butylamino, 2,4-dimethyl-c-butylamino, 3,3-dimethyl-c-butylamino, 1-n-propyl-c-propylamino, 2-n-propyl-c-propylamino, 1-i-propyl-c-propylamino, 2-i-propyl-c-propylamino, 1,2,2-trimethyl-c-propylamino, 1,2,3-trimethyl-c-propylamino, 2,2,3-trimethyl-c-propylamino, 1-ethyl-2-methyl-c-propylamino, 2-ethyl-1-methyl-c-propylamino, 2-ethyl-2-methyl-c-propylamino, 2-ethyl-3-methyl-c-propylamino, 1-methyl-1-ethyl-n-pentylamino, 1-heptylamino, 2-heptylamino, 1-ethyl-1,2-dimethyl-n-propylamino, 1-ethyl-2,2-dimethyl-n-propylamino, 1-octylamino, 3-octylamino, 4-methyl-3-n-heptylamino, 6-methyl-2-n-heptylamino, 2-propyl-1-n-heptylamino, 2,4,4-trimethyl-1-n-pentylamino, 1-nonylamino, 2-nonylamino, 2,6-dimethyl-4-n-heptylamino, 3-ethyl-2,2-dimethyl-3-n-pentylamino, 3,5,5-trimethyl-1-n-hexylamino, 1-decylamino, 2-decylamino, 4-decylamino, 3,7-dimethyl-1-n-octylamino, 3,7-dimethyl-3-n-octylamino or the like may be mentioned.

A di-C₁₋₁₀ alkylamino group may be symmetric or asymmetric. A symmetric di-C₁₋₁₀ alkylamino group may be linear, branched or a C₃₋₁₀ cycloalkylamino group, and as specific examples, dimethylamino, diethylamino, di-n-propylamino, di-i-propylamino, di-c-propylamino, di-n-butylamino, di-i-butylamino, di-s-butylamino, di-t-butylamino, di-c-butylamino, di-(1-methyl-c-propyl)amino, di-(2-methyl-c-propyl)amino, di-n-pentylamino, di-(1-methyl-n-butyl)amino, di-(2-methyl-n-butyl)amino, di-(3-methyl-n-butyl)amino, di-(1,1-dimethyl-n-propyl)amino, di-(1,2-dimethyl-n-propyl)amino, di-(2,2-dimethyl-n-propyl)amino, di-(1-ethyl-n-propyl)amino, di-c-pentylamino, di-(1-methyl-c-butyl)amino, di-(2-methyl-c-butyl)amino, di-(3-methyl-c-butyl)amino, di-(1,2-dimethyl-c-propyl)amino, di-(2,3-dimethyl-c-propyl)amino, di-(1-ethyl-c-propyl)amino, di-(2-ethyl-c-propyl)amino, di-n-hexylamino, di-(1-methyl-n-pentyl)amino, di-(2-methyl-n-pentyl)amino, di-(3-methyl-n-pentyl)amino, di-(4-methyl-n-pentyl)amino, di-(1,1-dimethyl-n-butyl)amino, di-(1,2-dimethyl-n-butyl)amino, di-(1,3-dimethyl-n-butyl)amino, di-(2,2-dimethyl-n-butyl)amino, di-(2,3-dimethyl-n-butyl)amino, di-(3,3-dimethyl-n-butyl)amino, di-(1-ethyl-n-butyl)amino, di-(2-ethyl-n-butyl)amino, di-(1,1,2-trimethyl-n-propyl)amino, di-(1,2,2-trimethyl-n-propyl)amino, di-(1-ethyl-1-methyl-n-propyl)amino, di-(1-ethyl-2-methyl-n-propyl)amino, di-c-hexylamino, di-(1-methyl-c-pentyl)amino, di-(2-methyl-c-pentyl)amino, di-(3-methyl-c-pentyl)amino, di-(1-ethyl-c-butyl)amino, di-(2-ethyl-c-butyl)amino, di-(3-ethyl-c-butyl)amino, di-(1,2-dimethyl-c-butyl)amino, di-(1,3-dimethyl-c-butyl)amino, di-(2,2-dimethyl-c-butyl)amino, di-(2,3-dimethyl-c-butyl)amino, di-(2,4-dimethyl-c-butyl)amino, di-(3,3-dimethyl-c-butyl)amino, di-(1-n-propyl-c-propyl)amino, di-(2-n-propyl-c-propyl)amino, di-(1-i-propyl-c-propyl)amino, di-(2-i-propyl-c-propyl)amino, di-(1,2,2-trimethyl-c-propyl)amino, di-(1,2,3-trimethyl-c-propyl)amino, di-(2,2,3-trimethyl-c-propyl)amino, di-(1-ethyl-2-methyl-c-propyl)amino, di-(2-ethyl-1-methyl-c-propyl)amino, di-(2-ethyl-2-methyl-c-propyl)amino, di-(2-ethyl-3-methyl-c-propyl)amino, di-(1-methyl-1-ethyl-n-pentyl)amino, di-(1-heptyl)amino, di-(2-heptyl)amino, di-(1-ethyl-1,2-dimethyl-n-propyl)amino, di-(1-ethyl-2,2-dimethyl-n-propyl)amino, di-(1-octyl)amino, di-(3-octyl)amino, di-(4-methyl-3-n-heptyl)amino, di-(6-methyl-2-n-heptyl)amino, di-(2-propyl-1-n-heptyl)amino, di-(2,4,4-trimethyl-1-n-pentyl)amino, di-(1-nonyl)amino, di-(2-nonyl)amino, di-(2,6-dimethyl-4-n-heptyl)amino, di-(3-ethyl-2,2-dimethyl-3-n-pentyl)amino, di-(3,5,5-trimethyl-1-n-hexyl)amino, di-(1-decyl)amino, di-(2-decyl)amino, di-(4-decyl)amino, di-(3,7-dimethyl-1-n-octyl)amino, di-(3,7-dimethyl-3-n-octyl)amino or the like may be mentioned.

An asymmetric di-C₁₋₁₀ alkylamino group may be linear, branched or a C₃₋₁₀ cycloalkylamino group, and as specific examples, (methyl, ethyl)amino, (methyl, n-propyl)amino, (methyl, i-propyl)amino, (methyl, c-propyl)amino, (methyl, n-butyl)amino, (methyl, i-butyl)amino, (methyl, s-butyl)amino, (methyl, t-butyl)amino, (methyl, n-pentyl)amino, (methyl, c-pentyl)amino, (methyl, n-hexyl)amino, (methyl, c-hexyl)amino, (ethyl, n-propyl)amino, (ethyl, i-propyl)amino, (ethyl, c-propyl)amino, (ethyl, n-butyl)amino, (ethyl, i-butyl)amino, (ethyl, s-butyl)amino, (ethyl, t-butyl)amino, (ethyl, n-pentyl)amino, (ethyl, c-pentyl)amino, (ethyl, n-hexyl)amino, (ethyl, c-hexyl)amino, (n-propyl, i-propyl)amino, (n-propyl, c-propyl)amino, (n-propyl, n-butyl)amino, (n-propyl, i-butyl)amino, (n-propyl, s-butyl)amino, (n-propyl, t-butyl)amino, (n-propyl, n-pentyl)amino, (n-propyl, c-pentyl)amino, (n-propyl, n-hexyl)amino, (n-propyl, c-hexyl)amino, (i-propyl, c-propyl)amino, (i-propyl, n-butyl)amino, (i-propyl, i-butyl)amino, (i-propyl, s-butyl)amino, (i-propyl, t-butyl)amino, (i-propyl, n-pentyl)amino, (i-propyl, c-pentyl)amino, (i-propyl, n-hexyl)amino, (i-propyl, c-hexyl)amino, (c-propyl, n-butyl)amino, (c-propyl, i-butyl)amino, (c-propyl, s-butyl)amino, (c-propyl, t-butyl)amino, (c-propyl, n-pentyl)amino, (c-propyl, c-pentyl)amino, (c-propyl, n-hexyl)amino, (c-propyl, c-hexyl)amino, (n-butyl, i-butyl)amino, (n-butyl, s-butyl)amino, (n-butyl, t-butyl)amino, (n-butyl, n-pentyl)amino, (n-butyl, c-pentyl)amino, (n-butyl, n-hexyl)amino, (n-butyl, c-hexyl)amino, (i-butyl, s-butyl)amino, (i-butyl, t-butyl)amino, (i-butyl, n-pentyl)amino, (i-butyl, c-pentyl)amino, (i-butyl, n-hexyl)amino, (i-butyl, c-hexyl)amino, (s-butyl, t-butyl)amino, (s-butyl, n-pentyl)amino, (s-butyl, c-pentyl)amino, (s-butyl, n-hexyl)amino, (s-butyl, c-hexyl)amino, (t-butyl, n-pentyl)amino, (t-butyl, c-pentyl)amino, (t-butyl, n-hexyl)amino, (t-butyl, c-hexyl)amino, (n-pentyl, c-pentyl)amino, (n-pentyl, n-hexyl)amino, (n-pentyl, c-hexyl)amino, (c-pentyl, n-hexyl)amino, (c-pentyl, c-hexyl)amino, (n-hexyl, c-hexyl)amino, (methyl, n-heptyl)amino, (methyl, n-octyl)amino, (methyl, n-nonanyl)amino, (methyl, n-decyl)amino, (ethyl, n-heptyl)amino, (ethyl, n-octyl)amino, (ethyl, n-nonanyl)amino, (ethyl, n-decyl)amino or the like may be mentioned.

A C₁₋₁₀ alkylaminocarbonyl group may be linear, branched or a C₁₋₁₀ cycloalkylaminocarbonyl group, and as specific examples, methylaminocarbonyl, ethylaminocarbonyl, n-propylaminocarbonyl, i-propylaminocarbonyl, c-propylaminocarbonyl, n-butylaminocarbonyl, i-butylaminocarbonyl, s-butylaminocarbonyl, t-butylaminocarbonyl, c-butylaminocarbonyl, 1-methyl-c-propylaminocarbonyl, 2-methyl-c-propylaminocarbonyl, n-pentylaminocarbonyl, 1-methyl-n-butylaminocarbonyl, 2-methyl-n-butylaminocarbonyl, 3-methyl-n-butylaminocarbonyl, 1,1-dimethyl-n-propylaminocarbonyl, 1,2-dimethyl-n-propylaminocarbonyl, 2,2-dimethyl-n-propylaminocarbonyl, 1-ethyl-n-propylaminocarbonyl, c-pentylaminocarbonyl, 1-methyl-c-butylaminocarbonyl, 2-methyl-c-butylaminocarbonyl, 3-methyl-c-butylaminocarbonyl, 1,2-dimethyl-c-propylaminocarbonyl, 2,3-dimethyl-c-propylaminocarbonyl, 1-ethyl-c-propylaminocarbonyl, 2-ethyl-c-propylaminocarbonyl, n-hexylaminocarbonyl, 1-methyl-n-pentylaminocarbonyl, 2-methyl-n-pentylaminocarbonyl, 3-methyl-n-pentylaminocarbonyl, 4-methyl-n-pentylaminocarbonyl, 1,1-dimethyl-n-butylaminocarbonyl, 1,2-dimethyl-n-butylaminocarbonyl, 1,3-dimethyl-n-butylaminocarbonyl, 2,2-dimethyl-n-butylaminocarbonyl, 2,3-dimethyl-n-butylaminocarbonyl, 3,3-dimethyl-n-butylaminocarbonyl, 1-ethyl-n-butylaminocarbonyl, 2-ethyl-n-butylaminocarbonyl, 1,1,2-trimethyl-n-propylaminocarbonyl, 1,2,2-trimethyl-n-propylaminocarbonyl, 1-ethyl-1-methyl-n-propylaminocarbonyl, 1-ethyl-2-methyl-n-propylaminocarbonyl, c-hexylaminocarbonyl, 1-methyl-c-pentylaminocarbonyl, 2-methyl-c-pentylaminocarbonyl, 3-methyl-c-pentylaminocarbonyl, 1-ethyl-c-butylaminocarbonyl, 2-ethyl-c-butylaminocarbonyl, 3-ethyl-c-butylaminocarbonyl, 1,2-dimethyl-c-butylaminocarbonyl, 1,3-dimethyl-c-butylaminocarbonyl, 2,2-dimethyl-c-butylaminocarbonyl, 2,3-dimethyl-c-butylaminocarbonyl, 2,4-dimethyl-c-butylaminocarbonyl, 3,3-dimethyl-c-butylaminocarbonyl, 1-n-propyl-c-propylaminocarbonyl, 2-n-propyl-c-propylaminocarbonyl, 1-i-propyl-c-propylaminocarbonyl, 2-i-propyl-c-propylaminocarbonyl, 1,2,2-trimethyl-c-propylaminocarbonyl, 1,2,3-trimethyl-c-propylaminocarbonyl, 2,2,3-trimethyl-c-propylaminocarbonyl, 1-ethyl-2-methyl-c-propylaminocarbonyl, 2-ethyl-1-methyl-c-propylaminocarbonyl, 2-ethyl-2-methyl-c-propylaminocarbonyl, 2-ethyl-3-methyl-c-propylaminocarbonyl, 1-methyl-1-ethyl-n-pentylaminocarbonyl, 1-heptylaminocarbonyl, 2-heptylaminocarbonyl, 1-ethyl-1,2-dimethyl-n-propylaminocarbonyl, 1-ethyl-2,2-dimethyl-n-propylaminocarbonyl, 1-octylaminocarbonyl, 3-octylaminocarbonyl, 4-methyl-3-n-heptylaminocarbonyl, 6-methyl-2-n-heptylaminocarbonyl, 2-propyl-1-n-heptylaminocarbonyl, 2,4,4-trimethyl-1-n-pentylaminocarbonyl, 1-nonylaminocarbonyl, 2-nonylaminocarbonyl, 2,6-dimethyl-4-n-heptylaminocarbonyl, 3-ethyl-2,2-dimethyl-3-n-pentylaminocarbonyl, 3,5,5-trimethyl-1-n-hexylaminocarbonyl, 1-decylaminocarbonyl, 2-decylaminocarbonyl, 4-decylaminocarbonyl, 3,7-dimethyl-1-n-octylaminocarbonyl, 3,7-dimethyl-3-n-octylaminocarbonyl or the like may be mentioned.

A di-C₁₋₁₀ alkylaminocarbonyl group may be symmetric or asymmetric. A symmetric di-C₁₋₁₀ alkylaminocarbonyl group may be linear, branched or a C₃₋₁₀ cycloalkylaminocarbonyl group, and as specific examples, dimethylaminocarbonyl, diethylaminocarbonyl, di-n-propylaminocarbonyl, di-i-propylaminocarbonyl, di-c-propylaminocarbonyl, di-n-butylaminocarbonyl, di-i-butylaminocarbonyl, di-s-butylaminocarbonyl, di-t-butylaminocarbonyl, di-c-butylaminocarbonyl, di-(1-methyl-c-propyl)aminocarbonyl, di-(2-methyl-c-propyl)aminocarbonyl, di-n-pentylaminocarbonyl, di-(1-methyl-n-butyl)aminocarbonyl, di-(2-methyl-n-butyl)aminocarbonyl, di-(3-methyl-n-butyl)aminocarbonyl, di-(1,1-dimethyl-n-propyl)aminocarbonyl, di-(1,2-dimethyl-n-propyl)aminocarbonyl, di-(2,2-dimethyl-n-propyl)aminocarbonyl, di-(1-ethyl-n-propyl)aminocarbonyl, di-c-pentylaminocarbonyl, di-(1-methyl-c-butyl)aminocarbonyl, di-(2-methyl-c-butyl)aminocarbonyl, di-(3-methyl-c-butyl)aminocarbonyl, di-(1,2-dimethyl-c-propyl)aminocarbonyl, di-(2,3-dimethyl-c-propyl)aminocarbonyl, di-(1-ethyl-c-propyl)aminocarbonyl, di-(2-ethyl-c-propyl)aminocarbonyl, di-n-hexylaminocarbonyl, di-(1-methyl-n-pentyl)aminocarbonyl, di-(2-methyl-n-pentyl)aminocarbonyl, di-(3-methyl-n-pentyl)aminocarbonyl, di-(4-methyl-n-pentyl)aminocarbonyl, di-(1,1-dimethyl-n-butyl)aminocarbonyl, di-(1,2-dimethyl-n-butyl)aminocarbonyl, di-(1,3-dimethyl-n-butyl)aminocarbonyl, di-(2,2-dimethyl-n-butyl)aminocarbonyl, di-(2,3-dimethyl-n-butyl)aminocarbonyl, di-(3,3-dimethyl-n-butyl)aminocarbonyl, di-(1-ethyl-n-butyl)aminocarbonyl, di-(2-ethyl-n-butyl)aminocarbonyl, di-(1,1,2-trimethyl-n-propyl)aminocarbonyl, di-(1,2,2-trimethyl-n-propyl)aminocarbonyl, di-(1-ethyl-1-methyl-n-propyl)aminocarbonyl, di-(1-ethyl-2-methyl-n-propyl)aminocarbonyl, di-c-hexylaminocarbonyl, di-(1-methyl-c-pentyl)aminocarbonyl, di-(2-methyl-c-pentyl)aminocarbonyl, di-(3-methyl-c-pentyl)aminocarbonyl, di-(1-ethyl-c-butyl)aminocarbonyl, di-(2-ethyl-c-butyl)aminocarbonyl, di-(3-ethyl-c-butyl)aminocarbonyl, di-(1,2-dimethyl-c-butyl)aminocarbonyl, di-(1,3-dimethyl-c-butyl)aminocarbonyl, di-(2,2-dimethyl-c-butyl)aminocarbonyl, di-(2,3-dimethyl-c-butyl)aminocarbonyl, di-(2,4-dimethyl-c-butyl)aminocarbonyl, di-(3,3-dimethyl-c-butyl)aminocarbonyl, di-(1-n-propyl-c-propyl)aminocarbonyl, di-(2-n-propyl-c-propyl)aminocarbonyl, di-(1-i-propyl-c-propyl)aminocarbonyl, di-(2-i-propyl-c-propyl)aminocarbonyl, di-(1,2,2-trimethyl-c-propyl)aminocarbonyl, di-(1,2,3-trimethyl-c-propyl)aminocarbonyl, di-(2,2,3-trimethyl-c-propyl)aminocarbonyl, di-(1-ethyl-2-methyl-c-propyl)aminocarbonyl, di-(2-ethyl-1-methyl-c-propyl)aminocarbonyl, di-(2-ethyl-2-methyl-c-propyl)aminocarbonyl, di-(2-ethyl-3-methyl-c-propyl)aminocarbonyl, di-(1-methyl-1-ethyl-n-pentyl)aminocarbonyl, di-(l-heptyl)aminocarbonyl, di-(2-heptyl)aminocarbonyl, di-(1-ethyl-1,2-dimethyl-n-propyl)aminocarbonyl, di-(1-ethy)-2,2-dimethyl-n-propyl)aminocarbonyl, di-(1-octyl)aminocarbonyl, di-(3-octyl)aminocarbonyl, di-(4-methyl-3-n-heptyl)aminocarbonyl, di-(6-methyl-2-n-heptyl)aminocarbonyl, di-(2-propyl-1-n-heptyl)aminocarbonyl, di-(2,4,4-trimethyl-1-n-pentyl)aminocarbonyl, di-(1-nonyl)aminocarbonyl, di-(2-nonyl)aminocarbonyl, di-(2,6-dimethyl-4-n-heptyl)aminocarbonyl, di-(3-ethyl-2,2-dimethyl-3-n-pentyl)aminocarbonyl, di-(3,5,5-trimethyl-1-n-hexyl)aminocarbonyl, di-(1-decyl)aminocarbonyl, di-(2-decyl)aminocarbonyl, di-(4-decyl)aminocarbonyl, di-(3,7-dimethyl-1-n-octyl)aminocarbonyl, di-(3,7-dimethyl-3-n-octyl)aminocarbonyl or the like may be mentioned.

An asymmetric C₁₋₁₀ dialkylaminocarbonyl group may be linear, branched or a C₃₋₁₀ cycloalkylaminocarbonyl group, and as specific examples, (methyl, ethyl)aminocarbonyl, (methyl, n-propyl)aminocarbonyl, (methyl, i-propyl)aminocarbonyl, (methyl, c-propyl)aminocarbonyl, (methyl, n-butyl)aminocarbonyl, (methyl, i-butyl)aminocarbonyl, (methyl, s-butyl)aminocarbonyl, (methyl, t-butyl)aminocarbonyl, (methyl, n-pentyl)aminocarbonyl, (methyl, c-pentyl)aminocarbonyl, (methyl, n-hexyl)aminocarbonyl, (methyl, c-hexyl)aminocarbonyl, (ethyl, n-propyl)aminocarbonyl, (ethyl, i-propyl)aminocarbonyl, (ethyl, c-propyl)aminocarbonyl, (ethyl, n-butyl)aminocarbonyl, (ethyl, i-butyl)aminocarbonyl, (ethyl, s-butyl)aminocarbonyl, (ethyl, t-butyl)aminocarbonyl, (ethyl, n-pentyl)aminocarbonyl, (ethyl, c-pentyl)aminocarbonyl, (ethyl, n-hexyl)aminocarbonyl, (ethyl, c-hexyl)aminocarbonyl, (n-propyl, i-propyl)aminocarbonyl, (n-propyl, c-propyl)aminocarbonyl, (n-propyl, n-butyl)aminocarbonyl, (n-propyl, i-butyl)aminocarbonyl, (n-propyl, s-butyl)aminocarbonyl, (n-propyl, t-butyl)aminocarbonyl, (n-propyl, n-pentyl)aminocarbonyl, (n-propyl, c-pentyl)aminocarbonyl, (n-propyl, n-hexyl)aminocarbonyl, (n-propyl, c-hexyl)aminocarbonyl, (i-propyl, c-propyl)aminocarbonyl, (i-propyl, n-butyl)aminocarbonyl, (i-propyl, i-butyl)aminocarbonyl, (i-propyl, s-butyl)aminocarbonyl, (i-propyl, t-butyl)aminocarbonyl, (i-propyl, n-pentyl)aminocarbonyl, (i-propyl, c-pentyl)aminocarbonyl, (i-propyl, n-hexyl)aminocarbonyl, (i-propyl, c-hexyl)aminocarbonyl, (c-propyl, n-butyl)aminocarbonyl, (c-propyl, i-butyl)aminocarbonyl, (c-propyl, s-butyl)aminocarbonyl, (c-propyl, t-butyl)aminocarbonyl, (c-propyl, n-pentyl)aminocarbonyl, (c-propyl, c-pentyl)aminocarbonyl, (c-propyl, n-hexyl)aminocarbonyl, (c-propyl, c-hexyl)aminocarbonyl, (n-butyl, i-butyl)aminocarbonyl, (n-butyl, s-butyl)aminocarbonyl, (n-butyl, t-butyl)aminocarbonyl, (n-butyl, n-pentyl)aminocarbonyl, (n-butyl, c-pentyl)aminocarbonyl, (n-butyl, n-hexyl)aminocarbonyl, (n-butyl, c-hexyl)aminocarbonyl, (i-butyl, s-butyl)aminocarbonyl, (i-butyl, t-butyl)aminocarbonyl, (i-butyl, n-pentyl)aminocarbonyl, (i-butyl, c-pentyl)aminocarbonyl, (i-butyl, n-hexyl)aminocarbonyl, (i-butyl, c-hexyl)aminocarbonyl, (s-butyl, t-butyl)aminocarbonyl, (s-butyl, n-pentyl)aminocarbonyl, (s-butyl, c-pentyl)aminocarbonyl, (s-butyl, n-hexyl)aminocarbonyl, (s-butyl, c-hexyl)aminocarbonyl, (t-butyl, n-pentyl)aminocarbonyl, (t-butyl, c-pentyl)aminocarbonyl, (t-butyl, n-hexyl)aminocarbonyl, (t-butyl, c-hexyl)aminocarbonyl, (n-pentyl, c-pentyl)aminocarbonyl, (n-pentyl, n-hexyl)aminocarbonyl, (n-pentyl, c-hexyl)aminocarbonyl, (c-pentyl, n-hexyl)aminocarbonyl, (c-pentyl, c-hexyl)aminocarbonyl, (n-hexyl, c-hexyl)aminocarbonyl, (methyl, n-heptyl)aminocarbonyl, (methyl, n-octyl)aminocarbonyl, (methyl, n-nonanyl)aminocarbonyl, (methyl, n-decyl)aminocarbonyl, (ethyl, n-heptyl)aminocarbonyl, (ethyl, n-octyl)aminocarbonyl, (ethyl, n-nonanyl)aminocarbonyl, (ethyl, n-decyl)aminocarbonyl or the like may be mentioned.

A C₁₋₆ alkylaminosulfonyl group may be linear, branched, a C₃₋₆ cycloalkylsulfonylamino group, and as specific examples, methylaminosulfonyl, ethylaminosulfonyl, n-propylaminosulfonyl, i-propylaminosulfonyl, c-propylaminosulfonyl, n-butylaminosulfonyl, i-butylaminosulfonyl, s-butylaminosulfonyl, t-butylaminosulfonyl, c-butylaminosulfonyl, 1-methyl-c-propylaminosulfonyl, 2-methyl-c-propylaminosulfonyl, n-pentylaminosulfonyl, 1-methyl-n-butylaminosulfonyl, 2-methyl-n-butylaminosulfonyl, 3-methyl-n-butylaminosulfonyl, 1,1-dimethyl-n-propylaminosulfonyl, 1,2-dimethyln-propylaminosulfonyl, 2,2-dimethyln-propylaminosulfonyl, 1-ethyl-n-propylaminosulfonyl, c-pentylaminosulfonyl, 1-methyl-c-butylaminosulfonyl, 2-methyl-c-butylaminosulfonyl, 3-methyl-c-butylaminosulfonyl, 1,2-dimethyl-c-propylaminosulfonyl, 2,3-dimethyl-c-propylaminosulfonyl, 1-ethyl-c-propylaminosulfonyl, 2-ethyl-c-propylaminosulfonyl, n-hexylaminosulfonyl, 1-methyl-n-pentylaminosulfonyl, 2-methyl-n-pentylaminosulfonyl, 3-methyl-n-pentylaminosulfonyl, 4-methyl-n-pentylaminosulfonyl, 1,1-dimethyl-n-butylaminosufonyl, 1,2-dimethyl-n-butylaminosufonyl, 1,3-dimethyl-n-butylaminosufonyl, 2,2-dimethyl-n-butylaminosufonyl, 2,3-dimethyl-n-butylaminosufonyl, 3,3-dimethyl-n-butylaminosufonyl, 1-ethyl-n-butylaminosufonyl, 2-ethyl-n-butylaminosufonyl, 1,1,2-trimethyl-n-propylaminosulfonyl, 1,2,2-trimethyl-n-propylaminosulfonyl, 1-ethyl-1-methyl-n-propylaminosulfonyl, 1-ethyl-2-methyl-n-propylaminosulfonyl, c-hexylaminosulfonyl, 1-methyl-c-pentylaminosulfonyl, 2-methyl-c-pentylaminosulfonyl, 3-methyl-c-pentylaminosulfonyl, 1-ethyl-c-butylaminosulfonyl, 2-ethyl-c-butylaminosulfonyl, 3-ethyl-c-butylaminosulfonyl, 1,2-dimethyl-c-butylaminosulfonyl, 1,3-dimethyl-c-butylaminosulfonyl, 2,2-dimethyl-c-butylaminosulfonyl, 2,3-dimethyl-c-butylaminosulfonyl, 2,4-dimethyl-c-butylaminosulfonyl, 3,3-dimethyl-c-butylaminosulfonyl, 1-n-propyl-c-propylaminosulfonyl, 2-n-propyl-c-propylaminosulfonyl, 1-i-propyl-c-propylaminosulfonyl, 2-i-propyl-c-propylaminosulfonyl, 1,2,2-trimethyl-c-propylaminosulfonyl, 1,2,3-trimethyl-c-propylaminosulfonyl, 2,2,3-trimethyl-c-propylaminosulfonyl, 1-ethyl-2-methyl-c-propylaminosulfonyl, 2-ethyl-1-methyl-c-propylaminosulfonyl, 2-ethyl-2-methyl-c-propylaminosulfonyl, 2-ethyl-3-methyl-c-propylaminosulfonyl or the like may be mentioned.

A C₁₋₁₀ alkylaminosulfonyl group may be linear, branched, a C₃₋₁₀ cycloalkylsulfonylamino group or a di-C₁₋₁₀ alkylaminosulfonyl group, and as specific examples, in addition to those mentioned above, 1-methyl-1-ethyl-n-pentylaminosulfonyl, 1-heptylaminosulfonyl, 2-heptylaminosulfonyl, 1-ethyl-1,2-dimethyl-n-propylaminosulfonyl, 1-ethyl-2,2-dimethyl-n-propylaminosulfonyl, 1-octylaminosulfonyl, 3-octylaminosulfonyl, 4-methyl-3-n-heptylaminosulfonyl, 6-methyl-2-n-heptylaminosulfonyl, 2-propyl-1-n-heptylaminosulfonyl, 2,4,4-trimethyl-1-n-pentylaminosulfonyl, 1-nonylaminosulfonyl, 2-nonylaminosulfonyl, 2,6-dimethyl-4-n-heptylaminosulfonyl, 3-ethyl-2,2-dimethyl-3-n-pentylaminosulfonyl, 3,5,5-trimethl-1-n-hexylaminosulfonyl, 1-decylaminosulfonyl, 2-decylaminosulfonyl, 4-decylaminosulfonyl, 3,7-dimetyl-1-n-octylaminosulfonyl, 3,7-dimetyl-3-n-octylaminosulfonyl, c-heptylaminosulfonyl, c-octylaminosulfonyl, 1-methyl-c-hexylaminosulfonyl, 2-methyl-c-hexylaminosulfonyl, 3-methyl-c-hexylaminosulfonyl, 1,2-dimethyl-c-hexylaminosulfonyl, 1-ethyl-c-hexylaminosulfonyl, 1-methyl-c-pentylaminosulfonyl, 2-methyl-c-pentylaminosulfonyl, 3-methyl-c-pentylaminosulfonyl or the like may be mentioned.

The protecting group in a protected hydroxyl group may be a C₁₋₄ alkoxymethyl group (such as MOM: methoxymethyl, MEM: 2-methoxyethoxymethyl, ethoxymethyl, n-propoxymethyl, i-propoxymethyl, n-butoxymethyl, iBM: isobutyloxymethyl, BUM: t-butoxymethyl, POM: pivaloyloxymethyl, SEM: trimethylsilylethoxymethyl and the like, preferably a C₁₋₂ alkoxymethyl or the like), an aryloxymethyl (such as BOM: benzyloxymethyl, PMBM: p-methoxybenzyloxymethyl, P-AOM: p-anisyloxymethyl and the like, preferably benzyloxymethyl), a C₁₋₄ alkylaminomethyl group (such as dimethylaminomethyl), a substituted acetamidomethyl group (such as Acm: acetamidomethyl, Tacm: trimethylacetamidomethyl and the like), a substituted thiomethyl group (such as MTM: methylthiomethyl, PTM: phenylthiomethyl, Btm: benzylthiomethyl and the like), a carboxyl group, a C₁₋₇ acyl group (such as formyl, acetyl, fluoroacetyl, difluoroacetyl, trifluoroacetyl, chloroacetyl, dichloroacetyl, trichloroacetyl, propionyl, Pv: pivaloyl, tigloyl and the like), an arylcarbonyl group (such as benzoyl, p-bromobenzoyl, p-nitrobenzoyl, 2,4-dinitrobenzoyl, benzoylformyl, benzoylpropionyl, phenylpropionyl and the like), a C₁₋₄ alkoxycarbonyl group (such as methoxycarbonyl, ethoxycarbonyl, n-propoxycarbonyl, i-propoxycarbonyl, n-butoxycarbonyl, i-butoxycarbonyl, BOC: t-butoxycarbonyl, AOC: t-amyloxycarbonyl, VOC: vinyloxycarbonyl, AOC: allyloxycarbonyl, Teoc: 2-(trimethylsilyl)ethoxycarbonyl, Troc: 2,2,2-trichloroethoxycarbonyl and the like, preferably BOC and the like), an aryloxycarbonyl group (such as Z: benzyloxycarbonyl, p-nitrobenzyloxycarbonyl, MOZ: p-methoxybenzyloxycarbonyl and the like), a C₁₋₄ alkylaminocarbonyl group (such as methylcarbamoyl, Ec: ethylcarbamoyl, n-propylcarbamoyl and the like), an arylaminocarbonyl group (such as phenylcarbamoyl and the like), a trialkylsilyl group (such as TMS: trimethylsilyl, TES: triethylsilyl, TIPS: triisopropylsilyl, DEIPS: diethylisopropylsilyl, DMIPS: dimethylisopropylsilyl, DTBMS: di-t-butylmethylsilyl, IPDMS: isopropyldimethylsilyl, TBDMS: t-butyldimethylsilyl, TDS: thexyldimethylsilyl and the like, preferably t-butyldimethylsilyl and the like), a trialkylarylsilyl group (such as DPMS: diphenylmethylsilyl, TBDPS: t-butyldiphenylsilyl, TBMPS: t-butyldimethoxyphenylsilyl, TPS: triphenylsilyl and the like), an alkylsulfonyl group, (such as Ms: methanesulfonyl, ethanesulfonyl and the like) or an arylsulfonyl group (such as benzenesulfonyl, Ts: p-toluenesulfonyl, p-chlorobenzenesulfonyl, MBS: p-methoxybenzenesulfonyl, m-nitrobenzenesulfonyl, o-nitrobenzenesulfonyl, p-nitrobenzenesulfonyl,2,4-nitrobenzenesulfonyl, iMds: 2,6-dimethoxy-4-methylbenzenesulfonyl, Mds: 2,6-dimethyl-4-methoxybenzenesulfonyl, Mtb: 2,4,6-trimethoxybenzenesulfonyl, Mte: 2,3,5,6-tetramethyl-4-methoxybenzenesulfonyl, Mtr: 2,3,6-trimethyl-4-methoxybenzenesulfonyl, Mts: 2,4,6-trimethylbenzenesulfonyl, Pme: pentamethylbenzenesulfonyl and the like).

In addition, a 1-methyl-1-methoxyethyl group, a 1-ethoxyethyl group, a 2,2,2-trichloroethyl group, a 2-trimethylsilylethoxy group, a t-butyl group, an allyl group, a benzyl group, a p-methoxybenzyl group, a 2,4-dinitrophenyl group, a p-chlorophenyl group, a p-methoxyphenyl group, a tetrahydropyranyl group, a tetrahydrofuranyl group or the like may be mentioned.

Preferred examples of the substituents in the compound to be used in the present invention (the formula (I)) are given below.

Preferred examples of A are CR^{a} (wherein R^{a} is a hydrogen atom, a methyl group, an ethyl group, a n-propyl group, an i-propyl group or a n-butyl group) and a nitrogen atom.

Particularly preferred examples are CH and a nitrogen atom.

Preferred examples of B are a sulfur atom, NR^{b} (wherein R^{b} is a C₁₋₁₀ alkyl group (the alkyl group may be substituted with one or more halogen atoms, one or more hydroxyl groups or one or more C₂₋₁₄ aryl groups)).

Particularly preferred examples are a sulfur atom and NR^{c} (wherein R^{c} is a methyl group, an ethyl group, a n-propyl group, an i-propyl group or a n-butyl group).

More preferred examples are a sulfur atom and NMe.

Preferred examples of L¹ are a bond, an oxygen atom, a sulfur atom and NH. A particularly preferred example is a bond.

Preferred examples of L² are a bond, an oxygen atom, a sulfur atom and NH. A particularly preferred example is a bond.

Preferred examples of L³ are a bond, NR^{d} (wherein R^{d} is a hydrogen atom, a methyl group, an ethyl group, a n-propyl group, an i-propyl group or a n-butyl group). A particularly preferred example is NH.

Preferred examples of L⁴ are a bond and NH.

A preferred example of R¹ is an C₂₋₁₄ aryl group which is not substituted or is substituted with one or more of the following substituents.

Substituents: halogen atoms, C₁₋₆ alkyl groups (the C₁₋₆ alkyl groups may be substituted with one or more halogen atoms) and C₁₋₆ alkoxy groups (the C₁₋₆ alkoxy groups may be substituted with one or more halogen atoms).

A more preferred example of R¹ is a phenyl group which is not substituted or is substituted with one or more of the following substituents.

Substituents: halogen atoms, C₁₋₆ alkyl groups (the C₁₋₆ alkyl groups may be substituted with one or more halogen atoms) and C₁₋₆ alkoxy groups (the C₁₋₆ alkoxy groups may be substituted with one or more halogen atoms).

A particularly preferred example is a phenyl group substituted with two chlorine atoms.

Preferred examples of R² are a hydrogen atom, a C₁₋₁₀ alkyl group (the C₁₋₁₀ alkyl group is not substituted or is substituted with one or more halogen atoms). More preferred examples are a hydrogen atom, a methyl group, an ethyl group, a n-propyl group, an i-propyl group, a n-butyl group or a trifluoromethyl group. A particularly preferred example is a methyl group.

Preferred examples of X are OH and SH. A particularly preferred example is OH.

Preferred examples of Y are an oxygen atom and a sulfur atom.

Preferred examples of the substituent R³ are a C₂₋₁₄ aryl group which is optionally substituted with one or more substituents selected from the following substituent set A and may be optionally substituted with one or more substituents independently selected from the following substituent set B and a C₂₋₁₄ aryl group which may be optionally substituted with one or more substituents selected from the following substituent set C.
Substituent set A:
-W¹(CW²W³)ₘW⁴ (wherein W¹ is (CR²⁴R²⁵)ₙ (wherein each of R²⁴ and R²⁵ is independently a hydrogen atom, a hydroxyl group, an amino group, a C₁₋₆alkyl group or a C₁₋₆ alkoxy group (the C₁₋₆ alkyl group and the C₁₋₆ alkoxy group may be substituted with one or more halogen atoms), and n is 0, 1, 2 or 3), an oxygen atom, a sulfur atom or NR³⁶ (wherein R³⁶ is a hydrogen atom, a C₁₋₆ alkyl group, a formyl group or a C₁₋₆ alkylcarbonyl group), each of W² and W³ is independently a hydrogen atom or a C₁₋₃ alkyl group (the C₁₋₃ alkyl group may be substituted with one or more halogen atoms), m is 0, 1, 2 or 3, and W⁴ is SO₂R²⁸, SOR²⁸ or COR²⁸ (wherein R²⁸ is NR²⁹R³⁰ (wherein R²⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups andC₂₋₁₄ aryloxy groups (theC₂₋₁₄ aryl groups and theC₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group or a C₂₋₁₄ aryloxy group (the C₂₋₁₄ aryl group and the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), and R³⁰ is an amino group, a mono- or di-C₁₋₁₀ alkylamino group, a C₁₋₁₀ alkylcarbonylamino group, a C₁₋₁₀ alkylaminocarbonyl group, a C₂₋₉ heterocyclyl group (the amino group, the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group are optionally substituted with one or more substituents selected from the group consisting of: hydrogen atoms, carboxyl groups, carbamoyl groups, sulfamoyl groups, sulfo groups, nitro groups, cyano groups, halogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) and with one or more substituents selected from the group consisting of: carboxyl groups, carbamoyl groups, sulfo groups, sulfamoyl groups and C₂₋₉ heterocyclyl groups (the C₂₋₉ heterocyclyl groups may be optionally substituted with one or more substituents selected from the group consisting of: hydrogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups, C₂₋₉ heterocyclyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups, the C₁₋₁₀ alkylcarbonyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))), or R²⁹ and R³⁰ mean, together with each other, -(CH₂)ₘ₃-G-(CH₂)ₘ₄- (wherein G is CR³¹R³² (wherein R³¹ is a hydrogen atom, a C₁₋₁₀ alkyl group, a C₂₋₁₄ aryl group, a C₁₋₁₀ alkoxy group, a C₂₋₁₄ aryloxy group (the C₁₋₁₀ alkyl group, the C₂₋₁₄ aryl group, the C₁₋₁₀ alkoxy group and the C₂₋₁₄ aryloxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- ordi-C₁₋₁₀ alkylamino groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a hydroxyl group, a protected hydroxyl group, and R³² is an amino group, a mono- or di-C₁₋₁₀ alkylamino group, a C₁₋₁₀ alkylcarbonylamino group, a C₁₋₁₀ alkylaminocarbonyl group, a C₂₋₉ heterocyclyl group (the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, amino groups, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))) or NR³³ (wherein R³³ is an amino group, a mono- or di-C₁₋₁₀ alkylamino group, a C₁₋₁₀ alkylcarbonylamino group, a C₁₋₁₀ alkylaminocarbonyl group, a C₂₋₉ heterocyclyl group (the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, amino groups, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))), and each of m3 and m4 is independently an integer of from 0 to 5, provided that m3+m4 is 3, 4 or 5), or NR²⁹R³⁰, as a whole, means a nitrogen-containing C₂₋₉ cyclyl group (the nitrogen-containing C₂₋₉ cyclyl group may be optionally substituted with one or more hydrogen atoms and is substituted with two or three substituents independently selected from the group consisting of: hydroxyl groups, amino groups, thiol groups, nitro groups, cyano groups, halogen atoms, carboxyl groups, carbamoyl groups, sulfamoyl groups, formyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyloxy groups, C₁₋₁₀ alkoxycarbonyl groups, C₁₋₁₀ alkylcarbonyl groups, C₁₋₁₀ alkylcarbonylamino groups, mono- or di-C₁₋₁₀ alkylamino groups, C₁₋₁₀ alkylsulfonylamino groups, C₁₋₁₀ thioalkyl groups, C₂₋₉ heterocyclyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyloxy groups, the C₁₋₁₀ alkoxycarbonyl groups, the C₁₋₁₀ alkylcarbonyl groups, the C₁₋₁₀ alkylcarbonylamino groups, the mono- or di-C₁₋₁₀ alkylamino groups, the C₁₋₁₀ alkylsulfonylamino groups, the C₁₋₁₀ thioalkyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))) or a substituted C₂₋₉ heterocyclyl group (the substituted C₂₋₉ heterocyclyl group may be optionally substituted with one or more substituents selected from the group consisting of: hydrogen atoms, hydroxyl groups, amino groups, thiol groups, nitro groups, cyano groups, halogen atoms, carboxyl groups, carbamoyl groups, sulfamoyl groups, formyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyloxy groups, C₁₋₁₀ alkoxycarbonyl groups, C₁₋₁₀ alkylcarbonyl groups, C₁₋₁₀ alkylcarbonylamino groups, mono- or di-C₁₋₁₀ alkylamino groups, C₁₋₁₀ alkylsulfonylamino groups, C₁₋₁₀ thioalkyl groups, C₂₋₉ heterocyclyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyloxy groups, the C₁₋₁₀ alkoxycarbonyl groups, the C₁₋₁₀ alkylcarbonyl groups, the C₁₋₁₀ alkylcarbonylamino groups, the mono- or di-C₁₋₁₀ alkylamino groups, the C₁₋₁₀ alkylsulfonylamino groups, the C₁₋₁₀ thioalkyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)))), and C₁₋₆ alkyl groups (the C₁₋₆ alkyl groups are optionally substituted with one or more substituents selected from the group consisting of: hydroxyl groups, amino groups, mono- or di-C₁₋₁₀ alkylamino groups and C₁₋₆ alkoxy groups (the mono- or di-C₁₋₁₀ alkylamino groups and the C₁₋₆ alkoxy groups may be substituted with one or more halogen atoms), with one or more substituents selected from the group consisting of: hydrogen atoms, hydroxyl groups, amino groups, thiol groups, nitro groups, cyano groups, halogen atoms, carboxyl groups, carbamoyl groups, sulfamoyl groups, sulfo groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkoxy groups, C₁₋₁₀ alkylcarbonyl groups, C₁₋₁₀ alkylcarbonylamino groups, mono- or di-C₁₋₁₀ alkylamino groups, C₁₋₁₀ alkylaminosulfo groups, C₁₋₁₀ alkylaminocarbonyl groups, C₁₋₁₀ alkylsulfonylamino groups, C₁₋₁₀ thioalkyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups, the C₁₋₁₀ alkoxy groups, the C₁₋₁₀ alkylcarbonyl groups, the C₁₋₁₀ alkylcarbonylamino groups, the mono- or di-C₁₋₁₀ alkylamino groups, the C₁₋₁₀ alkylaminosulfo groups, the C₁₋₁₀ alkylaminocarbonyl groups, the C₁₋₁₀ alkylsulfonylamino groups and the C₁₋₁₀ thioalkyl groups may be optionally substituted with one or more substituents selected from the group consisting of: carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₁₀ alkyl groups (the C₁₋₁₀ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) and with -W⁵ (wherein W⁵ is SO₂R⁴², SOR⁴², COR⁴² (wherein R⁴² is a hydrogen atom, a hydroxyl group or NR⁴³R⁴⁴, and each of R⁴³ and R⁴⁴ is independently a hydrogen atom, a hydroxyl group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), or R⁴³ and R⁴⁴ mean, together with each other, -(CH₂)ₘ₁-E-(CH₂)ₘ₂- (wherein E is an oxygen atom, a sulfur atom, CR²⁶R²⁷ (wherein each of R²⁶ and R²⁷ is independently a hydrogen atom, a C₁₋₁₀ alkyl group, a C₂₋₁₄ aryl group, a C₁₋₁₀ alkoxy group, a C₂₋₁₄ aryloxy group, a hydroxyl group or a protected hydroxyl group) or NR⁸ (wherein R⁸ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), and each of m1 and m2 is independently an integer of from 0 to 5 provided that m1+m2 is 3, 4 or 5)) or W⁴, and W⁴ is the same as defined above)) may be mentioned.
Substituent set B:
Hydrogen atoms, hydroxyl groups, amino groups, thiol groups, nitro groups, cyano groups, halogen atoms, carboxyl groups, carbamoyl groups, sulfamoyl groups, sulfo groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkoxy groups, C₁₋₁₀ alkylcarbonyl groups, C₁₋₁₀ alkylcarbonylamino groups, mono- or di-C₁₋₁₀ alkylamino groups, C₁₋₁₀ alkylaminosulfonyl groups, C₁₋₁₀ alkylaminocarbonyl groups, C₁₋₁₀ alkylsulfonylamino groups, C₁₋₁₀ thioalkyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups, the C₁₋₁₀ alkoxy groups, the C₁₋₁₀ alkylcarbonyl groups, the C₁₋₁₀ alkylcarbonylamino groups, the mono- or di-C₁₋₁₀ alkylamino groups, the C₁₋₁₀ alkylaminosulfonyl groups, the C₁₋₁₀ alkylaminocarbonyl groups, the C₁₋₁₀ alkylsulfonylamino groups and the C₁₋₁₀ thioalkyl groups may be optionally substituted with one or more substituents selected from the group consisting of: carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) may be mentioned.
Substituent set C:
Hydrogen atoms and C₁₋₁₀ alkylcarbonyl groups (the C₁₋₁₀ alkylcarbonyl groups may be optionally substituted with one or more substituents selected from the group consisting of: amino groups, thiol groups, sulfonyl groups, C₂₋₉ heterocyclyl groups, C₂₋₁₄ aryl groups and C₂₋₁₄ aryloxy groups (the amino groups, the thiol groups, the sulfonyl groups, the C₂₋₉ heterocyclyl groups, the C₂₋₁₄ aryl groups and the C₂₋₁₄ aryloxy groups may be optionally substituted with one or more substituents selected from the groups consisting of: C₁₋₆ alkyl groups, C₂₋₁₄ aryl groups and C₂₋₁₄ aryloxy groups)) may be mentioned.

A particularly preferred example of the substituent R³ is a phenyl group or a 2-thenyl group substituted with COR^{e} (wherein R^{e} is a 1-pyrrolidyl group (the 1-pyrrolidyl group is substituted with one -NHCOMe or with two hydroxyl groups), -NHNMe₂, - NH(CH₂)₂CONH₂, -NHNH-(2-pyridyl), -N(NH₂)CH₂-(4-pyridyl), -CH₂S-(4-pyridyl), - CH₂SO₂-(4-pyridyloxide or -CH₂O-(1,3-dimethyl-(1H)-pyrazol-5-yl)).

Favorable compounds to be used for the present invention are as follows.
(1) Compounds represented by the formula (II), which are compounds represented by the formula (I) wherein A is CH, B is a sulfur atom, L¹ is a bond, L² is a bond, L³ is NH, and L⁴ is a bond, tautomers or pharmaceutically acceptable salts of the compounds or solvates thereof. 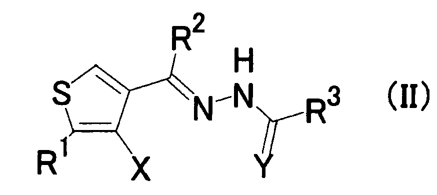
(2) Compounds represented by the formula (III), which are compounds represented by the formula (I) wherein A is CH, B is a sulfur atom, L¹ is a bond, L² is a bond, L³ is NH, and L⁴ is NH, tautomers or pharmaceutically acceptable salts of the compounds or solvates thereof. 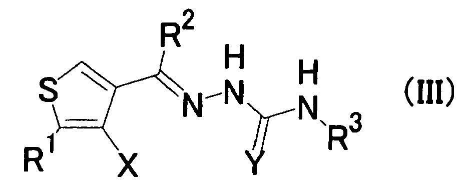
(3) Compounds represented by the formula (IV), which are compounds represented by the formula (I) wherein A is a nitrogen atom, B is NMe, L¹ is a bond, L² is a bond, L³ is NH, and L⁴ is a bond, tautomers or pharmaceutically acceptable salts of the compounds or solvates thereof. 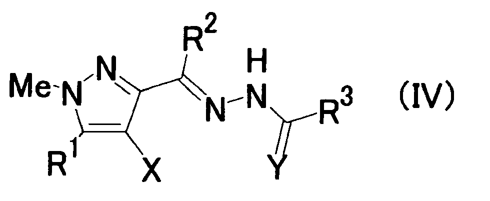
(4) Compounds represented by the formula (V), which are compounds represented by the formula (I) wherein A is a nitrogen atom, B is NMe, L¹ is a bond, L² is a bond, L³ is NH, and L⁴ is NH, tautomers or pharmaceutically acceptable salts of the compounds or solvates thereof. 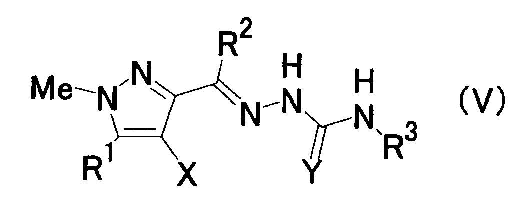
(5) The compounds according to (1), (2), (3) or (4), wherein R¹ is a phenyl group (the phenyl group is substituted with one or two substituents selected from the group consisting of: halogen atoms, C₁₋₆ alkyl groups, C₁₋₆ alkoxy groups, trifluoromethyl groups and trifluoromethoxy groups), tautomers or pharmaceutically acceptable salts of the compounds or solvates thereof.
(6) The compounds according to (1), (2), (3), (4) or (5), wherein R² is a hydrogen atom or a C₁₋₃ alkyl group (the C₁₋₃ alkyl group may be substituted with one or more halogen atoms), tautomers or pharmaceutically acceptable salts of the compounds or solvates thereof.
(7) The compounds according to (6), wherein X is OH, tautomers or pharmaceutically acceptable salts of the compounds or solvates thereof.
(8) The compounds according to (7), wherein Y is a sulfur atom, tautomers or pharmaceutically acceptable salts of the compounds or solvates thereof.
(9) The compounds according to (7), wherein Y is an oxygen atom, tautomers or pharmaceutically acceptable salts of the compounds or solvates thereof.
(10) The compounds according to (8) or (9), wherein R³ is any of the following substituted phenyl groups, tautomers or pharmaceutically acceptable salts of the compounds or solvates thereof. 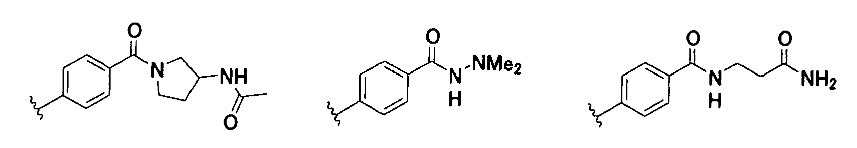 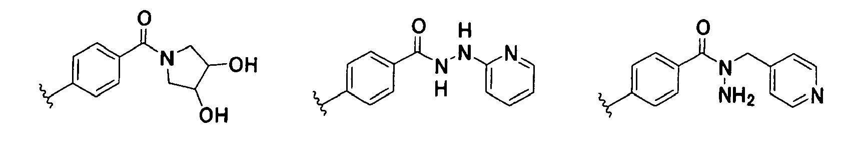 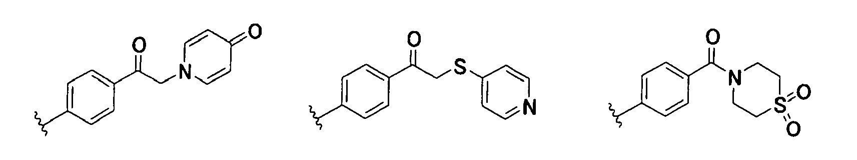 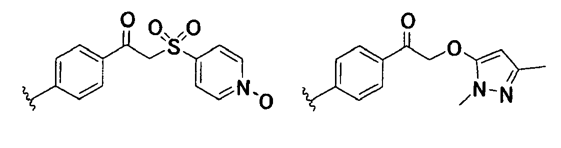
(11) The compounds according to (8) or (9), wherein R³ is any of the following substituted thienyl groups, tautomers or pharmaceutically acceptable salts of the compounds or solvates thereof.

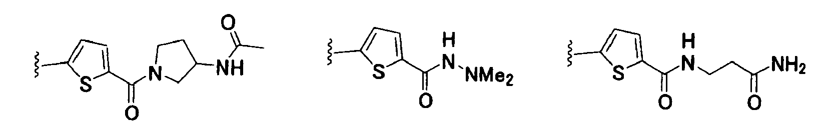

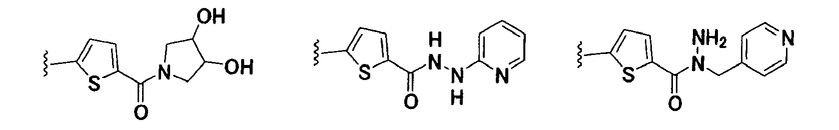

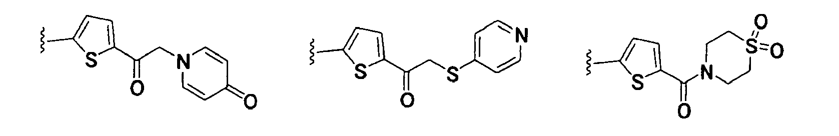

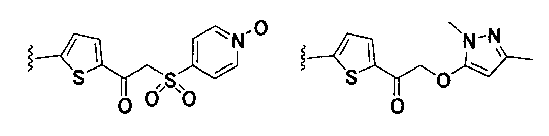

The compound of the present invention can be synthesized by reference to Patent Document WO2006/064957.

The compound of the present invention represented by the formula (I) or a pharmaceutically acceptable salt thereof may be in the form of arbitrary crystals or an arbitrary hydrate, depending on the production conditions. The present invention covers these crystals, hydrates and mixtures. They may be in the form of a solvate with an organic solvent such as acetone, ethanol and tetrahydrofuran, and the present invention covers any of these forms. The compound of the present invention represented by the formula (I) may be converted to a pharmaceutically acceptable salt or may be liberated from the resulting salt, if necessary. The pharmaceutically acceptable salt of the present invention may be, for example, a salt with an alkali metal (such as lithium, sodium and potassium), an alkaline earth metal (such as magnesium and calcium), ammonium, an organic base or an amino acid. It may be a salt with an inorganic acid (such as hydrochloric acid, hydrobromic acid, phosphoric acid and sulfuric acid) or an organic acid (such as acetic acid, citric acid, maleic acid, fumaric acid, benzenesulfonic acid and p-toluenesulfonic acid).

### EXAMPLES

Now, the present invention will be described in further detail with reference to Examples. However, it should be understood that the present invention is by no means restricted by these specific Examples.

### (EXAMPLE: Expansion of CD34⁺ cells from human cord blood-derived CD34⁺ cells)

Commercially available human cord blood-derived CD34⁺ cells (Cambrex Bio Science Walkersville) were plated on a 12-well plate (Corning) (from 5000 to 10000 cells/1 mL/ well). As the culture medium, StemSpan SFEM (Stemcell Technologies) containing 100 ng/mL SCF (R&D Systems) was used, and Compounds Nos. 1 to 11 dissolved in dimethyl sulfoxide were added in an amount of 0.1 %(v/v) to a final concentration of 0.3 or 1 µg/mL.

After the cells were incubated in liquid culture at 37°C for 7 days in a CO₂ incubator (in an atmosphere containing 5 vol% CO₂) the total number of cells was counted by trypan blue assay (Invitrogen) or Flow-Count^{™} fluorosphere assay (Beckman Coulter). The number of CD34⁺ cells was calculated as follows. After the incubation, the cells in the liquid culture was stained with a CD34 antibody (PE, Becton, Dickinson and Company). The stained cells were analyzed with a flow cytometer (Beckman Coulter) to determined the proportion of CD34⁺ cells, which was multiplied by the total number of cells to calculate the number of CD34⁺ cells.

The results demonstrate that the compounds of the present invention showed excellent expansion activity on CD34⁺ cells and have expansion activity on hematopoietic stem cells and hematopoietic progenitor cells.

The expansion efficiencies in the presence of 0.3 µg/mL or 1 µg/mL of compounds based on the number of CD34⁺ cells in the absence of them are shown in Table 1 on a scale of A for expansion efficiencies of 3 or greater, B for expansion efficiencies of at least 2 and less than 3, and C for expansion efficiencies of at least 1.4 and less than 2.

**TABLE 1**

| Compound No. | Concentration of compound | Expansion efficiency |
|---|---|---|
| 1 | 1 | A |
| 2 | 1 | A |
| 3 | 1 | A |
| 4 | 1 | A |
| 5 | 0.3 | C |
| 6 | 0.3 | C |
| 7 | 0.3 | C |
| 8 | 0.3 | B |
| 9 | 0.3 | C |
| 10 | 1 | A |
| 11 | 1 | B |

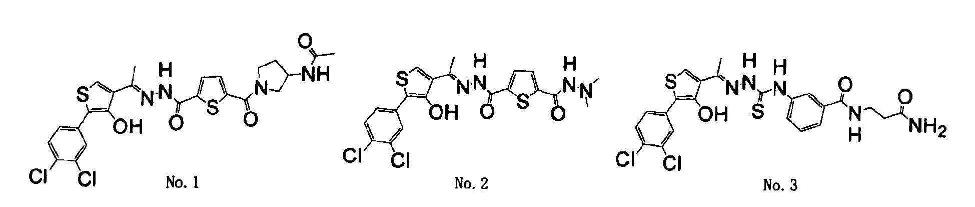

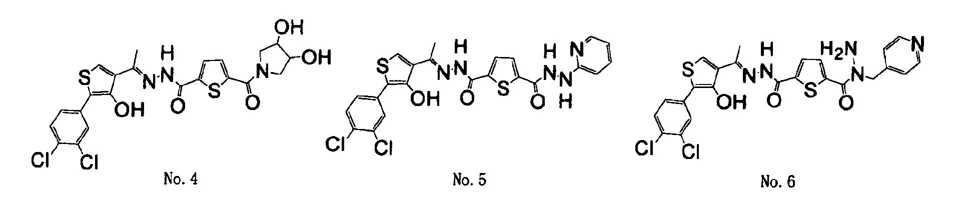

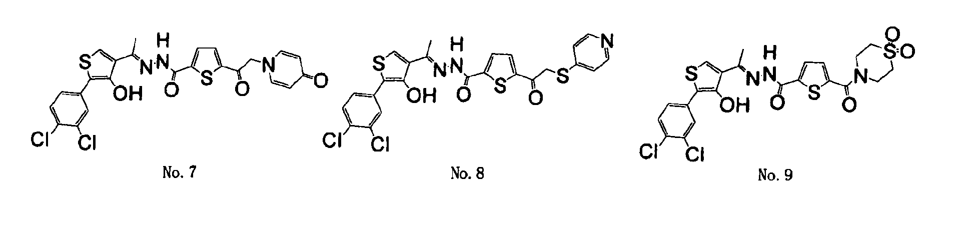

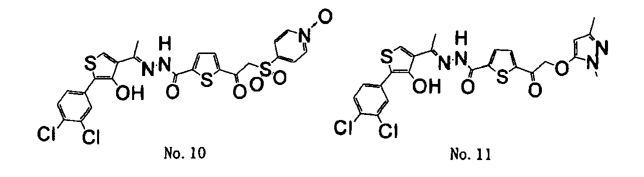

### INDUSTRIAL APPLICABILITY

The method of the present invention can expand human CD34⁺ cells by using a low molecular weight compound as an active ingredient. Cells expanded by the method of the present invention are useful as a hematopoietic cell and hematopoietic progenitor cell transplant for diseases accompanying hematopoietic dysfunction, ischemia or immune dysfunction and hence its application to cell therapy is expected.

The entire disclosure of Japanese Patent Application No. 2007-315165 filed on December 5, 2007 including specification, claims and summary is incorporated herein by reference in its entirety.

## Claims

1. A method for expanding CD34⁺ cells, which comprises culturing CD34⁺ cells ex vivo in the presence of a compound represented by the formula (I), a tautomer or pharmaceutically acceptable salt of the compound or a solvate thereof: 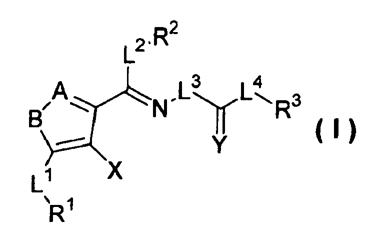 wherein A is a nitrogen atom or CR⁴ (wherein R⁴ is a hydrogen atom, a hydroxyl group (the hydroxyl group may be substituted with a C₂₋₆ alkenyl group or a C₂₋₆ alkynyl group), a thiol group (the thiol group may be substituted with a C₁₋₁₀ alkyl group, a C₂₋₆ alkenyl group, a C₂₋₆ alkynyl group or a C₁₋₁₀ alkylcarbonyl group), an amino group (the amino group may be substituted with one or two C₂₋₆ alkenyl groups or one or two C₂₋₆ alkynyl groups), a formyl group, a halogen atom, a nitro group, a cyano group, a C₁₋₁₀ alkyl group, a C₂₋₆ alkenyl group, a C₂₋₆ alkynyl group, a C₁₋₁₀ alkylcarbonylamino group, a mono- or di-C₁₋₁₀ alkylamino group, a C₁₋₁₀ alkoxy group (the C₁₋₁₀ alkyl group, the C₂₋₆ alkenyl group, the C₂₋₆ alkynyl group, the C₁₋₁₀ alkylcarbonylamino group, the mono- or di-C₁₋₁₀ alkylamino group and the C₁₋₁₀ alkoxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), a C₂₋₁₄ aryloxy group (the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), SO₂R⁵, SOR⁵ or COR⁵ (wherein R⁵ is a hydroxyl group, a C₁₋₁₀ alkyl group, a C₂₋₆ alkenyl group, a C₂₋₆ alkynyl group, a C₁₋₁₀ alkoxy group (the C₁₋₁₀ alkyl group, the C₂₋₆ alkenyl group, the C₂₋₆ alkynyl group and the C₁₋₁₀ alkoxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl group may be substituted with one or more halogen atoms) or one or more halogen atoms)), a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), a C₂₋₁₄ aryloxy group (the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) or NR⁶R⁷ (wherein each of R⁶ and R⁷ is independently a hydrogen atom, a hydroxyl group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), or R⁶ and R⁷ mean, together with each other, -(CH₂)ₘ₁-E-(CH₂)ₘ₂- (wherein E is an oxygen atom, a sulfur atom, CR²⁶R²⁷ (wherein each of R²⁶ and R²⁷ is independently a hydrogen atom, a C₁₋₁₀ alkyl group, a C₂₋₁₄ aryl group, a C₁₋₁₀ alkoxy group, a C₂₋₁₄ aryloxy group, a hydroxyl group or a protected hydroxyl group) or NR⁸ (wherein R⁸ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), and each of m1 and m2 is independently an integer of from 0 to 5 provided that m1+m2 is 3, 4 or 5)))),
B is an oxygen atom, a sulfur atom or NR⁹ (wherein R⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups) or a C₂₋₁₄ aryloxy group (the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)) (provided that when A is a nitrogen atom, B is not NH),
R¹ is a C₂₋₁₄ aryl group (the C₂₋₁₄ aryl group may be substituted with one or more substituents selected from the group consisting of: halogen atoms, carboxyl groups, nitro groups, formyl groups, cyano groups, hydroxyl groups, protected hydroxyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyl groups, C₁₋₁₀ alkylcarbonyloxy groups, C₁₋₁₀ alkoxycarbonyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyl groups, the C₁₋₁₀ alkylcarbonyloxy groups and the C₁₋₁₀ alkoxycarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups (the C₂₋₁₄ aryl groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), thiol groups and amino groups (the thiol groups and the amino groups may be optionally substituted with one or two substituents selected from the group consisting of: formyl groups, C₁₋₁₀ alkyl groups, C₂₋₆ alkenyl groups, C₂₋₆ alkynyl groups and C₁₋₁₀ alkylcarbonyl groups (the C₁₋₁₀ alkyl groups, the C₂₋₆ alkenyl groups, the C₂₋₆ alkynyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)))),
L¹ is a bond, CR¹⁰R¹¹ (wherein each of R¹⁰ and R¹¹ is independently a hydrogen atom or a C₁₋₆ alkyl group (the C₁₋₆ alkyl group may be substituted with one or more halogen atoms)), an oxygen atom, a sulfur atom or NR¹² (wherein R¹² is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
X is OR¹³, SR¹³ or NR¹⁴R¹⁵ (wherein R¹³ is a hydrogen atom, a C₁₋₁₀ alkyl group, a C₂₋₆ alkenyl group, a C₂₋₆ alkynyl group or a C₁₋₁₀ alkylcarbonyl group (the C₁₋₁₀ alkyl group, the C₂₋₆ alkenyl group, the C₂₋₆ alkynyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), and each of R¹⁴ and R¹⁵ is independently a hydrogen atom, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₁₋₁₀ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
R² is a hydrogen atom, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups),
L² is a bond, CR³⁴R³⁵ (wherein each of R³⁴ and R³⁵ is independently a hydrogen atom or a C₁₋₆ alkyl group (the C₁₋₆ alkyl group may be substituted with one or more halogen atoms)), an oxygen atom, a sulfur atom or NR¹⁶ (wherein R¹⁶ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
L³ is a bond, CR¹⁷R¹⁸ (wherein each of R¹⁷ and R¹⁸ is independently a hydrogen atom, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), an oxygen atom, a sulfur atom or NR¹⁹ (wherein R¹⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
L⁴ is a bond, CR²⁰R²¹ (wherein each of R²⁰ and R²¹ is independently a hydrogen atom, a C₁₋₁₀ alkyl group, a C₂₋₆ alkenyl group, a C₂₋₆ alkynyl group, a C₁₋₁₀ alkoxy group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), an oxygen atom, a sulfur atom or NR²² (wherein R²² is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂-₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)),
Y is an oxygen atom, a sulfur atom or NR²³ (wherein R²³ is a hydrogen atom, a hydroxyl group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)) or a C₂₋₁₄ aryl group (the C₂₋₁₄ aryl group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), and
R³ is a C₂₋₁₄ aryl group (the C₂₋₁₄ aryl group is optionally substituted with one or more substituents independently represented by -V¹ (wherein V¹ is a hydrogen atom, a hydroxyl group, a protected hydroxyl group, an amino group, a thiol group, a nitro group, a cyano group, a halogen atom, a carboxyl group, a sulfo group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono- or di-C₁₋₁₀ alkylamino group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀
alkoxy group, the C₁₋₁₀ alkylcarbonyl group, the C₁₋₁₀ alkylcarbonylamino group, the mono- or di-C₁₋₁₀ alkylamino group, the C₁₋₁₀ alkylaminosulfonyl group, the C₁₋₁₀ alkylaminocarbonyl group, the C₁₋₁₀ alkylsulfonylamino group and the C₁₋₁₀ thioalkyl group may be optionally substituted with one or more substituents selected from the group consisting of: carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group or a C₂₋₁₄ aryloxy group (the C₂₋₁₄ aryl group and the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)) and with one or more substituents independently represented by-W¹(CW²W³)ₘW⁴ (wherein W¹ is (CR²⁴R²⁵)ₙ (wherein each of R²⁴ and R²⁵ is independently a hydrogen atom, a hydroxyl group, an amino group, a C₁₋₆ alkyl group or a C₁₋₆ alkoxy group (the C₁₋₆ alkyl group and the C₁₋₆ alkoxy group may be substituted with one or more halogen atoms), and n is 0, 1, 2 or 3), an oxygen atom, a sulfur atom or NR³⁶ (wherein R³⁶ is a hydrogen atom, a C₁₋₆ alkyl group, a formyl group or a C₁₋₆ alkylcarbonyl group), each of W² and W³ is independently a hydrogen atom or a C₁₋₃ alkyl group (the C₁₋₃ alkyl group may be substituted with one or more halogen atoms), m is 0, 1, 2 or 3, and W⁴ is SO₂R²⁸, SOR²⁸ or COR²⁸ (wherein R²⁸ is NR²⁹R³⁰ (wherein R²⁹ is a hydrogen atom, a hydroxyl group, a formyl group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a C₂₋₁₄ aryl group or a C₂₋₁₄ aryloxy group (the C₂₋₁₄ aryl group and the C₂₋₁₄ aryloxy group may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups), and R³⁰ is an amino group, a mono- or di-C₁₋₁₀ alkylamino group, a C₁₋₁₀ alkylcarbonylamino group, a C₁₋₁₀ alkylaminocarbonyl group, a C₂₋₉ heterocyclyl group (the amino group, the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups)), a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkylcarbonyl group (the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group and the C₁₋₁₀ alkylcarbonyl group are optionally substituted with one or more substituents selected from the group consisting of: hydrogen atoms, carboxyl groups, carbamoyl groups, sulfamoyl groups, sulfo groups, nitro groups, cyano groups, halogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂-₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- ordi-C₁₋₁₀ alkylamino groups, hydroxyl groups, protected hydroxyl groups, C₂₋₁₄ aryl groups and C₂₋₁₄ aryloxy groups) and with one or more substituents selected from the group consisting of: carboxyl groups, carbamoyl groups, sulfo groups, sulfamoyl groups and C₂₋₉ heterocyclyl groups (the C₂₋₉ heterocyclyl groups may be optionally substituted with one or more substituents selected from the group consisting of: hydrogen atoms, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups, C₂₋₉ heterocyclyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups, the C₁₋₁₀ alkylcarbonyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))), or R²⁹ and R³⁰ mean, together with each other, -(CH₂)ₘ₃-G-(CH₂)ₘ₄- (wherein G is CR³¹R³² (wherein R³¹ is a hydrogen atom, a C₁₋₁₀ alkyl group, a C₂₋₁₄ aryl group, a C₁₋₁₀ alkoxy group, a C₂₋₁₄ aryloxy group (the C₁₋₁₀ alkyl group, the C₂₋₁₄ aryl group, the C₁₋₁₀ alkoxy group and the C₂₋₁₄ aryloxy group may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), a hydroxyl group or a protected hydroxyl group, and R³² is an amino group, a mono- or di-C₁₋₁₀ alkylamino group, a C₁₋₁₀ alkylcarbonylamino group, a C₁₋₁₀ alkylaminocarbonyl group or a C₂₋₉ heterocyclyl group (the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, amino groups, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))) or NR³³ (wherein R³³ is an amino group, a mono- or di-C₁₋₁₀ alkylamino group, a C₁₋₁₀ alkylcarbonylamino group, a C₁₋₁₀ alkylaminocarbonyl group or a C₂₋₉ heterocyclyl group (the mono- or di-C₁₋₁₀ alkylamino group, the C₁₋₁₀ alkylcarbonylamino group, the C₁₋₁₀ alkylaminocarbonyl group and the C₂₋₉ heterocyclyl group may be substituted with one or more substituents selected from the group consisting of: hydrogen atoms, carboxyl groups, carbamoyl groups, sulfamoyl groups, sulfo groups, nitro groups, cyano groups, halogen atoms, amino groups, hydroxyl groups, formyl groups, C₁₋₁₀ alkyl groups, C₂₋₆ alkenyl groups, C₂₋₆ alkynyl groups, C₁₋₁₀ alkylcarbonyloxy groups, C₁₋₁₀ alkoxycarbonyl groups, C₁₋₁₀ alkylcarbonyl groups (the C₁₋₁₀ alkyl groups, the C₂₋₆ alkenyl groups, the C₂₋₆ alkynyl groups, the C₁₋₁₀ alkylcarbonyloxy groups, the C₁₋₁₀ alkoxycarbonyl groups and the C₁₋₁₀ alkylcarbonyl groups may be optionally substituted with one or more substituents selected from the group consisting of: carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, Cho alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))), and each of m3 and m4 is independently an integer of from 0 to 5, provided that m3+m4 is 3, 4 or 5), or NR²⁹R³⁰ as a whole, means a nitrogen-containing C₂₋₉ cyclyl group (the nitrogen-containing C₂₋₉ cyclyl group may be optionally substituted with one or more hydrogen atoms and is substituted with two or three substituents independently selected from the group consisting of: hydroxyl groups, amino groups, thiol groups, nitro groups, cyano groups, halogen atoms, carboxyl groups, carbamoyl groups, sulfamoyl groups, formyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyloxy groups, C₁₋₁₀ alkoxycarbonyl groups, C₁₋₁₀ alkylcarbonyl groups, C₁₋₁₀ alkylcarbonylamino groups, mono- or di-C₁₋₁₀ alkylamino groups, C₁₋₁₀ alkylsulfonylamino groups, C₁₋₁₀ thioalkyl groups, C₂₋₉ heterocyclyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyloxy groups, the C₁₋₁₀ alkoxycarbonyl groups, the C₁₋₁₀ alkylcarbonyl groups, the C₁₋₁₀ alkylcarbonylamino groups, the mono- or di-C₁₋₁₀ alkylamino groups, the C₁₋₁₀ alkylsulfonylamino groups, the C₁₋₁₀ thioalkyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))) or a substituted C₂₋₉ heterocyclyl group (the substituted C₂₋₉ heterocyclyl group may be optionally substituted with one or more substituents selected from the group consisting of: hydrogen atoms, hydroxyl groups, amino groups, thiol groups, nitro groups, cyano groups, halogen atoms, carboxyl groups, carbamoyl groups, sulfamoyl groups, formyl groups, C₁₋₁₀ alkyl groups, C₂₋₆ alkenyl groups, C₂₋₆ alkynyl groups, C₁₋₁₀ alkoxy groups, C₁₋₁₀ alkylcarbonyloxy groups, C₁₋₁₀ alkoxycarbonyl groups, C₁₋₁₀ alkylcarbonyl groups, C₁₋₁₀ alkylcarbonylamino groups, mono- or di-C₁₋₁₀ alkylamino groups, C₁₋₁₀ alkylsulfonylamino groups, C₁₋₁₀ thioalkyl groups, C₂₋₉ heterocyclyl groups (the C₁₋₁₀ alkyl groups, the C₂₋₆ alkenyl groups, the C₂₋₆ alkynyl groups, the C₁₋₁₀ alkoxy groups, the C₁₋₁₀ alkylcarbonyloxy groups, the C₁₋₁₀ alkoxycarbonyl groups, the C₁₋₁₀ alkylcarbonyl groups, the C₁₋₁₀ alkylcarbonylamino groups, the mono- or di-C₁₋₁₀ alkylamino groups, the C₁₋₁₀ alkylsulfonylamino groups, the C₁₋₁₀ thioalkyl groups and the C₂₋₉ heterocyclyl groups may be optionally substituted with one or more substituents selected from the group consisting of: hydrogen atoms, carboxyl groups, carbamoyl groups, sulfamoyl groups, sulfo groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms)), C₂₋₁₄ aryl groups and C₂₋₁₄ aryloxy groups (the C₂₋₁₄ aryl groups and the C₂₋₁₄ aryloxy groups may be optionally substituted with one or more substituents selected from the group consisting of: C₁₋₁₀ alkyl groups (the C₁₋₁₀ alkyl groups may be substituted with one or more halogen atoms), C₂₋₆ alkenyl groups, C₂₋₆ alkynyl groups, carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups))))), a C₂₋₁₄ aryl group (the C₂₋₁₄ aryl group is optionally substituted with one or more substituents independently represented by -V¹ (wherein V¹ is the same as defined above) and with one or more substituents selected from the group consisting of: C₁₋₆ alkyl groups, C₂₋₆ alkenyl groups and C₂₋₆ alkynyl groups (the C₁₋₆ alkyl groups, the C₂₋₆ alkenyl groups and the C₂₋₆ alkynyl groups are optionally substituted with one or more substituents selected from the group consisting of: hydroxyl groups, amino groups, mono- or di-C₁₋₁₀ alkylamino groups and C₁₋₆ alkoxy groups (the mono- or di-C₁₋₁₀ alkylamino groups and the C₁₋₆ alkoxy groups may be substituted with one or more halogen atoms), with one or more substituents independently represented by -V² (wherein V² is the same as V¹, and V¹ is the same as defined above) and with -W⁵ (wherein W⁵ is SO₂R⁴², SOR⁴², COR⁴² (wherein R⁴² is a hydrogen atom, a hydroxyl group or NR⁴³R⁴⁴, R⁴³ is the same as R⁶, R⁴⁴ is the same as R⁷, and R⁶ and R⁷ are the same as defined above) or W⁴, and W⁴ is the same as defined above))), a C₂₋₁₄ aryl group (the C₂₋₁₄ aryl group is optionally substituted with one or more substituents independently represented by -V¹ (wherein V¹ is the same as defined above) and with one or more substituents selected from the group consisting of: C₁₋₆ alkyl groups, C₂₋₆ alkenyl groups, C₂₋₆ alkynyl groups, C₁₋₁₀ alkylcarbonyl groups, C₁₋₁₀ alkylthiocarbonyl groups and C₁₋₁₀ alkylsulfonyl groups (the C₁-₆ alkyl groups, the C₂₋₆ alkenyl groups, the C₂₋₆ alkynyl groups, the C₁₋₁₀ alkylcarbonyl groups, the C₁₋₁₀ alkylthiocarbonyl groups and the C₁₋₁₀ alkylsulfonyl groups are optionally substituted with one or more substituents independently represented by -V² (wherein V² is the same as V¹, and V¹ is the same as defined above) and with one or more substituents selected from C₂₋₉ heterocyclyl groups (the C₂₋₉ heterocyclyl groups are optionally substituted with one or more substituents independently represented by -V³ (wherein V³ is the same as V¹, and V¹ is the same as defined above)))), a C₂₋₁₄ aryl group (the C₂₋₁₄ aryl group is optionally substituted with one or more substituents independently represented by -V¹ (wherein V¹ is the same as defined above) and with one or more substituents selected from the group consisting of: C₁₋₆ alkyl groups, C₂₋₆ alkenyl groups, C₂₋₆ alkynyl groups, C₁₋₁₀ alkylcarbonyl groups, C₁₋₁₀ alkylthiocarbonyl groups and C₁₋₁₀ alkylsulfonyl groups (the C₁₋₆ alkyl groups, the C₂₋₆ alkenyl groups, the C₂₋₆ alkynyl groups, the C₁₋₁₀ alkylcarbonyl groups, the C₁₋₁₀ alkylthiocarbonyl groups and the C₁₋₁₀ alkylsulfonyl groups are optionally substituted with one or more substituents independently represented by -V² (wherein V² is the same as V¹, and V¹ is the same as defined above) and with one or more substituents selected from the group consisting of: amino groups (the amino groups are substituted with a C₁₋₁₀ alkyl group (the C₁₋₁₀ alkyl group is optionally substituted with one or more substituents independently represented by -V³ (wherein V³ is the same as V¹, and V¹ is the same as defined above)) and with a C₂₋₉ heterocyclyl group or a C₂₋₁₄ aryl group (the C₂₋₉ heterocyclyl group and the C₂₋₁₄ aryl group are optionally substituted with one or more substituents independently represented by -V⁴ (wherein V⁴ is the same as V¹, and V¹ is the same as defined above))), amino groups, mono- or di-C₁₋₁₀ alkylamino groups, C₁₋₁₀ alkoxy groups, C₁₋₁₀ thioalkyl groups, C₁₋₁₀ alkylsulfonyl groups, sulfonyl groups, sulfinyl groups and thiol groups (the amino groups, the mono- or di-C₁₋₁₀ alkylamino groups, the C₁₋₁₀ alkoxy groups, the C₁₋₁₀ thioalkyl groups, the C₁₋₁₀ alkylsulfonyl groups, the sulfonyl groups, the sulfinyl groups and the thiol groups are optionally substituted with one or more C₂₋₉ heterocyclyl groups, one or more C₂₋₁₄ aryl groups or one or more C₂₋₁₄ aryloxide groups (the C₂₋₉ heterocyclyl groups, the C₂₋₁₄ aryl groups and the C₂₋₁₄ aryloxide groups are optionally substituted with one or more substituents independently represented by -V⁵ (wherein V⁵ is the same as V¹, and V¹ is the same as defined above))))) or a C₂₋₁₄ substituted aryl group (the C₂₋₁₄ substituted aryl group may be optionally substituted with one or more substituents independently represented by -V¹ (wherein V¹ is the same as defined above)).

2. The method for expanding CD34⁺ cells according to Claim 1, wherein A is CR⁴ (wherein R⁴ is the same as defined in Claim 1), B is a sulfur atom, L¹ is a bond, L² is a bond, L³ is NH, L⁴ is a bond or NH, X is a hydroxyl group, and Y is an oxygen atom or a sulfur atom.

3. The method for expanding CD34⁺ cells according to Claim 1, wherein A is a nitrogen atom, B is NR³⁷ (wherein R³⁷ is a C₁₋₁₀ alkyl group (the C₁₋₁₀ alkyl group may be optionally substituted with one or more substituents selected from the group consisting of carboxyl groups, nitro groups, cyano groups, halogen atoms, C₁₋₁₀ alkoxy groups, C₁₋₁₀ alkylcarbonyl groups, C₁₋₁₀ alkylcarbonyloxy groups, C₁₋₁₀ alkoxycarbonyl groups, C₁₋₁₀ alkylcarbonylamino groups, amino groups, mono- or di-C₁₋₁₀ alkylamino groups, hydroxyl groups, protected hydroxyl groups, C₂₋₁₄ aryl groups and C₂₋₁₄ aryloxy groups (the C₂₋₁₄ aryl groups and the C₂₋₁₄ aryloxy groups may be substituted with one or more C₁₋₆ alkyl groups (the C₁₋₆ alkyl groups may be substituted with one or more halogen atoms) or one or more halogen atoms))), L¹ is a bond, L² is a bond, L³ is NH, L⁴ is a bond or NH, X is a hydroxyl group, and Y is an oxygen atom or a sulfur atom.

4. The method for expanding CD34⁺ cells according to Claim 2, wherein A is CH.

5. The method for expanding CD34⁺ cells according to Claim 3, wherein B is NR³⁸ (wherein R³⁸ is a C₁₋₁₀ alkyl group).

6. The method for expanding CD34⁺ cells according to any one of Claims 1 to 5, wherein R¹ is a C₂₋₁₄ aryl group (the C₂₋₁₄ aryl group may be optionally substituted with one or more halogen atoms, one or more C₁₋₁₀ alkyl groups or one or more C₁₋₁₀ alkoxy groups (the C₁₋₁₀ alkyl groups and the C₁₋₁₀ alkoxy groups may be optionally substituted with one or more halogen atoms)), and R² is a C₁₋₁₀ alkyl group (the C₁₋₁₀ alkyl group may be optionally substituted with one or more halogen atoms).

7. The method for expanding CD34⁺ cells according to any one of Claims 1 to 6, which involves addition of at least one blood cell stimulating factor.

8. The method for expanding CD34⁺ cells according to Claim 7, wherein the blood cell stimulating factor is selected from the group consisting of stem cell factor (SCF), interleukin 3 (IL-3), interleukin 6 (IL-6), interleukin 11 (IL-11), flk2/flt3 ligand (FL), granulocyte colony stimulating factor (G-CSF), granulocyte-macrophage colony stimulating factor (GM-CSF), thrombopoietin (TPO) and erythropoietin (EPO).

9. The method for expanding CD34⁺ cells according to Claim 8, wherein the blood cell stimulating factor is stem cell factor (SCF).

10. The method for expanding CD34⁺ cells according to any one of Claims 1 to 9, wherein the CD34⁺ cells are obtained from the bone marrow, the liver, the spleen or peripheral or cord blood.

11. The method for expanding CD34⁺ cells according to Claim 10, wherein the CD34⁺ cells are obtained from cord blood.

12. The method for expanding CD34⁺ cells according to Claim 11, wherein CD34⁺ cells obtained from cord blood are cultured in the presence of stem cell factor (SCF).

13. CD34⁺ cells expanded by the method as defined in any one of Claims 1 to 12.

14. A material for cell therapy by transplanting CD34⁺ cells expanded by the method as defined in any one of Claims 1 to 12 into a human for treatment of a disease.

15. The material for cell therapy according to Claim 14, wherein the disease to be treated is leukemia, aplastic anemia, myelodysplastic syndrome, malignant lymphoma, multiple myeloma, myeloproliferative disease, a genetic blood disease, a solid tumor, an autoimmune disease, immunodeficiency, cerebral infarction, myocardial infarction or obstructive arteriosclerosis.
